# EUROPEAN PATENT APPLICATION

(11) **EP 4 375 274 A1**
(43) Date of publication of application: **29.05.2024**
(21) Application number: 22845935.0
(22) Date of filing: 20.07.2022
(51) Int. Cl.: C07D 237/32, A61K 31/502, A61K 31/5025, A61K 31/504, A61P 25/00, A61P 25/28, A61P 29/00, A61P 37/02, C07D 237/26, C07D 237/34, C07D 401/12, C07D 403/12, C07D 405/12, C07D 405/14, C07D 409/12, C07D 471/04, C07D 487/04, C07D 491/044, C07D 491/052, C07D 491/107, C07D 495/04

(54) **ANNULATED PYRIDAZINE COMPOUND**

(30) Priority: 21.07.2021 JP 2021120560
(71) Applicant: Nico Therapeutics, Inc., Chicago, Illinois 60631 (US)
(72) Inventor: INAGAKI, Yusuke, Tokyo 103-8411 (JP); WASHIO, Takuya, Tokyo 103-8411 (JP); KOIZUMI, Yuka, Tokyo 103-8411 (JP); TOYA, Hiroki, Tokyo 103-8411 (JP); YAMASHITA, Yumi, Tokyo 103-8411 (JP); KURIWAKI, Ikumi, Tokyo 103-8411 (JP); MAEDA, Junko, Tokyo 103-8411 (JP); KOIKE, Takanori, Tokyo 103-8411 (JP); KAMIKUBO, Takashi, Tokyo 103-8411 (JP); YAMAKI, Susumu, Tokyo 103-8411 (JP); KURAMOTO, Kazuyuki, Tokyo 103-8411 (JP); SABA, Kengo, Tokyo 103-8411 (JP); TOMIYAMA, Hiroshi, Hanishina-gun, Nagano 389-0606 (JP); IWAI, Yoshinori, Hanishina-gun, Nagano 389-0606 (JP); NAKAMURA, Akihiko, Hanishina-gun, Nagano 389-0606 (JP)
(74) Representative: Gille Hrabal Partnerschaftsgesellschaft mbB Patentanwälte
(86) International application number: PCT/JP2022/028138
(87) International publication number: WO 2023/003002

(57) **Abstract**

An object of the present invention is to provide a pharmaceutical composition, in particular, a compound suitable for preventing and/or treating inflammatory diseases and/or neurodegenerative disease. The present inventors have conducted intensive studies to find a compound having an inhibitory effect on NLRP3 inflammasome activation and found that an annulated pyridazine compound has an inhibitory effect on NLRP3 inflammasome activation, thus completing the present invention. The annulated pyridazine compound of the present invention is expected to serve as a prophylactic and/or therapeutic agent for inflammatory diseases and/or neurodegenerative diseases.

## Description

### TECHNICAL FIELD

The present invention relates to an annulated pyridazine compound having an inhibitory effect on NLRP3 inflammasome activation, which is expected to be useful as an active ingredient of a pharmaceutical composition, for example, a pharmaceutical composition for preventing and/or treating inflammatory diseases and/or neurodegenerative diseases, or a salt thereof.

### BACKGROUND ART

An inflammasome is an assembly of intracellular proteins induced by endogenous and exogenous alarm molecules and a mechanism responsible for amplification of inflammatory responses through activation caused by cleavage of inflammatory cytokines IL-1 β and IL-18 via activation of caspase-1, as well as induction of cell death. A plurality of types of molecules recognizing alarm molecules are known, which include NLRP1, NLRP3, NLRC4, and AIM2, and NLRP3 is activated by recognizing cellular stress caused by, for example, an extracellular ATP molecule, a toxin of a pathogen, a crystal of uric acid or cholesterol, and an abnormal aggregate of proteins.

As a disease cause by NLRP3 gain-of-function mutations, cryopyrin-associated periodic syndrome (CAPS) is known (Nature Genetics, Vol. 29, No. 3, pp. 301-305, 2001). Moreover, it is reported that NLRP3 inflammasomes are activated or highly expressed in a wide variety of diseases such as gout (Arthritis Research and Therapy, Vol. 12, No. 2, pp. 206, 2010), nonalcoholic steatohepatitis (Journal of Molecular Medicine, Vol. 92, No. 10, pp. 1069-1082, 2014), inflammatory bowel disease (Gut, Vol. 59, No. 9, pp. 1192-1100, 2010), Alzheimer's disease (Nature, Vol. 493, No. 7434, pp. 674-678, 2013), Parkinson's disease (PLoS ONE, Vol. 8, No. 1, Article No. e55375, 2013), amyotrophic lateral sclerosis (Inflammation, Vol. 41, No. 1, pp. 93-103, 2018), and multiple system atrophy (Journal of Neuropathology and Exprimental Neurology, Vol. 77, No. 11, pp. 1055-1065, 2018).

It is also known that α- synuclein fibrils activate NLRP3 to promote IL-1β production from microglia; and that administration of an NLRP3 inhibitor improves functions in a mouse model with α-synucleinopathy induced by α-synuclein fibrils (Science Translational Medicine, Vol. 10, Article No. eaah4066, 2018).

PTL 1 describes that a compound represented by the following formula has an inhibitory effect on the NLRP3 inflammasome pathway (R³ and R⁴ are H, cyano, C₁₋₄ alkyl, or halogeno C₁₋₄ alkyl. See the publication for other symbols in the formula).

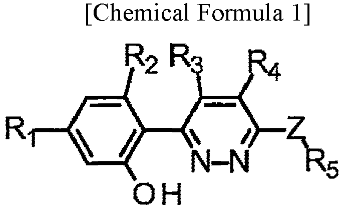

PTL 2, published after the priority date of the present application, describes that a compound represented by the following formula has an inhibitory effect on NLRP3 inflammasome activation (see the publication for symbols in the formula).

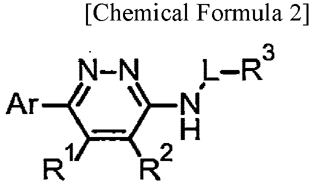

PTL 3, published after the priority date of the present application, describes that a compound represented by the following formula has an NLRP3 inhibitory effect (see the publication for symbols in the formula).

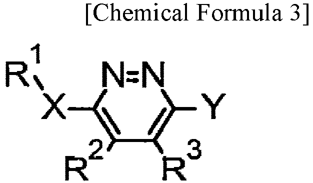

PTL 4 describes that a compound represented by the following formula has an NLRP3 inhibitory effect (see the publication for symbols in the formula).

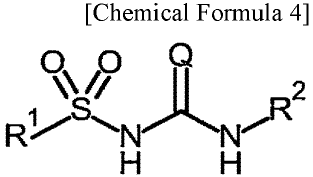

PTL 5 describes that a compound represented by the following formula has an NLRP1/3 modulatory effect (see the publication for symbols in the formula).

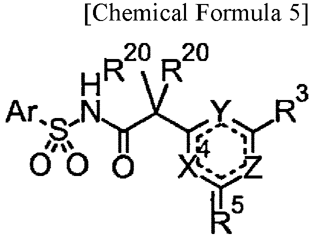

PTL 6 describes that a compound represented by the following formula has an inhibitory effect on NLRP3 inflammasome (see the publication for symbols in the formula).

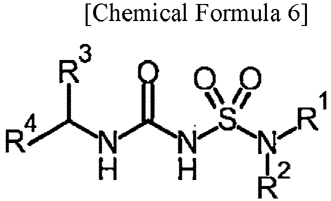

PTL 7 describes that a compound represented by the following formula has an inhibitory effect on C5a receptors (see the publication for symbols in the formula).

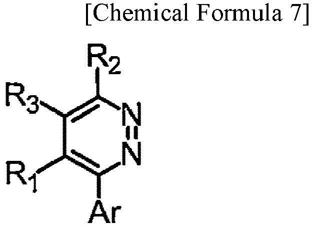

PTL 8 describes that a compound represented by the following formula has an inhibitory effect on the Hedgehog signaling pathway (see the publication for symbols in the formula).

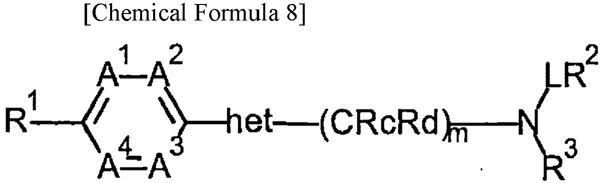

PTL 9 describes that a compound represented by the following formula has an inhibitory effect on the Hedgehog signaling pathway (see the publication for symbols in the formula).

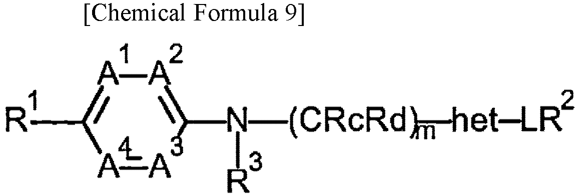

PTL 10 describes that a compound represented by the following formula has an antagonistic effect on muscarinic M4 receptors (see the publication for symbols in the formula).

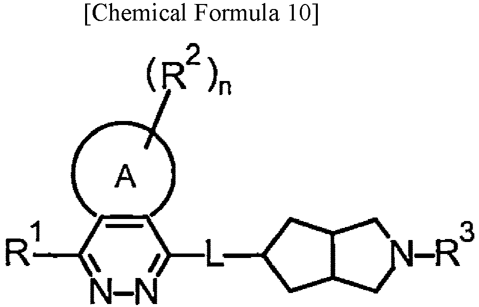

### CITATION LIST

### PATENT LITERATURE

PTL 1: International Publication No. WO 2020/234715
PTL 2: International Publication No. WO 2021/193897
PTL 3: U.S. Patent No. 11319319
PTL 4: International Publication No. WO 2019/008025
PTL 5: International Publication No. WO 2017/184604
PTL 6: International Publication No. WO 2018/015445
PTL 7: International Publication No. WO 2006/004589
PTL 8: International Publication No. WO 2014/191737
PTL 9: International Publication No. WO 2015/001348
PTL 10: International Publication No. WO 2021/067696

### SUMMARY OF INVENTION

### TECHNICAL PROBLEM

Provided is a compound having an inhibitory effect on NLRP3 inflammasome activation, which is expected to be useful as an active ingredient of a pharmaceutical composition, in particular, a pharmaceutical composition for preventing and/or treating, for example inflammatory diseases and/or neurodegenerative diseases.

### SOLUTION TO PROBLEM

As a result of intensive studies on a compound having an inhibitory effect on NLRP3 inflammasome activation, the present inventors have found that an annulated pyridazine compound has an inhibitory effect on NLRP3 inflammasome activation, which is expected to be useful as an active ingredient of a pharmaceutical composition for preventing and/or treating, for example, inflammatory diseases and/or neurodegenerative diseases, thus completing the present invention.

In other words, the present invention relates to a compound of formula (I) or a salt thereof and a pharmaceutical composition containing the compound of formula (I) or a salt thereof and one or more excipients. 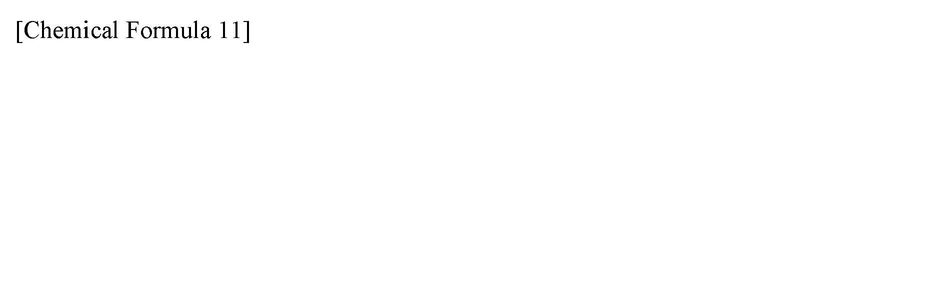 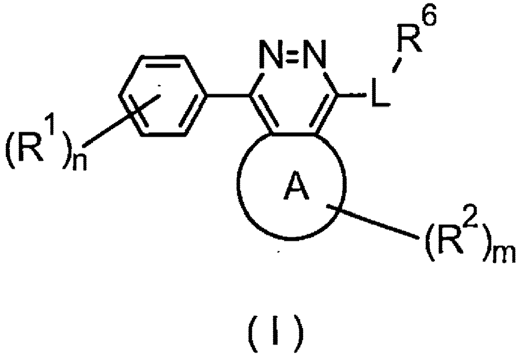 wherein
ring A is C₅₋₈ cycloalkenyl, 5- to 11-membered partially unsaturated heterocyclyl, aryl, or heteroaryl;
R¹, which are identical or different from each other, are OH, C₁₋₆ alkyl, halogeno C₁₋₆ alkyl, C₃₋₈ cycloalkyl, halogen, cyano, -O-C₁₋₆ alkyl, -O-halogeno C₁₋₆ alkyl, or -O-C₃₋₈ cycloalkyl; R², which are identical or different from each other, are C₁₋₆ alkyl, -C₁₋₆ alkylene-aryl, C₃₋₈ cycloalkyl, halogen, -O-C₁₋₆ alkyl, -O-halogeno C₁₋₆ alkyl, oxo, -C(O)-C₁₋₆ alkyl, or -S(O)₂-C₁₋₆ alkyl;
L is -NR³-, -O-, or -CR⁴R⁵-;
R³ is H or C₁₋₆ alkyl;
R⁴ and R⁵, which are identical or different from each other, are H or C₁₋₆ alkyl;
R⁶ is C₁₋₆ alkyl substituted with one to four identical or different R⁷,
-C₁₋₆ alkylene-(C₃₋₈ cycloalkyl optionally substituted with one to four identical or different R⁸), -C₁₋₆ alkylene-(4- to 7-membered saturated heterocyclyl optionally substituted with one to four identical or different R⁹), C₃₋₈ cycloalkyl optionally substituted with one to four identical or different R¹⁰, or 4- to 7-membered saturated heterocyclyl optionally substituted with one to four identical or different R¹¹;
R⁷ is -OR¹², -NR¹³R¹⁴, halogen, or cyano;
R⁸ and R¹⁰ are C₁₋₆ alkyl, halogeno C₁₋₆ alkyl, C₃₋₈ cycloalkyl, -OR¹², -NR¹³R¹⁴,
-C₁₋₆ alkylene-OR¹², -C₁₋₆ alkylene-NR¹³R¹⁴, halogen, or cyano;
R⁹ and R¹¹ are C₁₋₆ alkyl, halogeno C₁₋₆ alkyl, C₃₋₈ cycloalkyl, 4- to 7-membered saturated heterocyclyl, -OR¹², -NR¹³R¹⁴, -C₁₋₆ alkylene-OR¹², -C₁₋₆ alkylene-NR¹³R¹⁴, halogen, cyano, oxo, -C(O)-C₁₋₆ alkyl, or -S(O)₂-C₁₋₆ alkyl;
R¹² is H or C₁₋₆ alkyl;
R¹³ and R¹⁴, which are identical or different from each other, are H, C₁₋₆ alkyl, or
-C(O)-C₁₋₆ alkyl;
n is an integer of 1 to 4 and represents the number of substituents R¹; and
m is an integer of 0 to 3 and represents the number of substituents R²,
provided that when ring A is aryl or heteroaryl, formula (I) is formula (Ia): 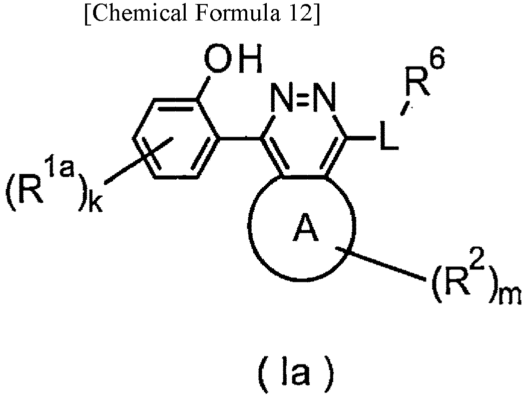
R^{1a}, which are identical or different from each other, are C₁₋₆ alkyl, halogeno C₁₋₆ alkyl, C₃₋₈ cycloalkyl, halogen, cyano, -O-C₁₋₆ alkyl, -O-halogeno C₁₋₆ alkyl, or -O-C₃₋₈ cycloalkyl; and
k is an integer of 0 to 3 and represents the number of substituents R^{1a}.

Note that when a symbol in a chemical formula in the present specification is used in another chemical formula, the same symbol has the same meaning, unless otherwise specified.

The present invention also relates to a pharmaceutical composition containing the compound of formula (I) or a salt thereof and one or more pharmaceutically acceptable excipients, in particular, a pharmaceutical composition for preventing and/or treating inflammatory diseases and/or neurodegenerative diseases. Note that the pharmaceutical composition include a prophylactic and/or therapeutic agent for inflammatory diseases and/or neurodegenerative diseases that contains the compound of formula (I) or a salt thereof.

In addition, the present invention relates to the compound of formula (I) or a salt thereof, which is an inhibitor of NLRP3 inflammasome activation; the compound of formula (I) or a salt thereof for use as an inhibitor of NLRP3 inflammasome activation; an inhibitor of NLRP3 inflammasome activation containing the compound of formula (I) or a salt thereof; a pharmaceutical composition containing the compound of formula (I) or a salt thereof, which is an inhibitor of NLRP3 inflammasome activation, and one or more pharmaceutically acceptable excipients; use of the compound of formula (I) or a salt thereof for production of a medicament or a pharmaceutical composition for preventing and/or treating inflammatory diseases and/or neurodegenerative diseases; use of the compound of formula (I) or a salt thereof for prevention and/or treatment of inflammatory diseases and/or neurodegenerative diseases; the compound of formula (I) or a salt thereof for use in prevention and/or treatment of inflammatory diseases and/or neurodegenerative diseases; and a method for preventing and/or treating inflammatory diseases and/or neurodegenerative diseases, including administering an effective amount of the compound of formula (I) or a salt thereof to a subject. Note that the "subject" is a human or a non-human animal in need of the prevention and/or treatment, and in an aspect, is a human in need of the prevention and/or treatment.

### ADVANTAGEOUS EFFECTS OF INVENTION

The compound of (I) or a salt thereof has an inhibitory effect on NLRP3 inflammasome activation, which can be used as a prophylactic and/or therapeutic agent, for example, for inflammatory diseases and/or neurodegenerative diseases.

### DESCRIPTION OF EMBODIMENTS

The present invention will be described in detail below.

In the present specification, the following terms, unless otherwise specified, have the following meanings. The following definitions are intended to clarify, but not limit, the terms defined. If a term used herein is not specifically defined, such a term is used in a sense generally accepted by those skilled in the art.

As used herein, the term "C₁₋₆ alkyl" refers to a linear or branched saturated hydrocarbon group having 1 to 6 carbon atoms (hereinafter abbreviated as C₁₋₆), such as methyl, ethyl, n-propyl, isopropyl, n-butyl, isobutyl, sec-butyl, tert-butyl, n-pentyl, 1-methylbutyl, 2-methylbutyl, 3-methylbutyl, 1,1-dimethylpropyl, 2,2-dimethylpropyl, 1,2-dimethylpropyl, 1-ethylpropyl, n-hexyl, 1,3-dimethylbutyl, and 1-ethyl-2-methylpropyl. The C₁₋₆ alkyl is a linear or branched C₁₋₄ alkyl group in an aspect; methyl, ethyl, n-propyl, isopropyl, n-butyl, isobutyl, or tert-butyl in another aspect; methyl, ethyl, n-propyl, n-butyl, or isobutyl in still another aspect; and n-propyl, n-butyl, or isobutyl in a further aspect. The C₁₋₆ alkyl is methyl, ethyl, n-propyl, or isopropyl in yet another aspect; methyl or ethyl in an aspect; ethyl in another aspect; and methyl in a further aspect.

The term "C₁₋₆ alkylene" refers to a linear or branched C₁₋₆ divalent saturated hydrocarbon group, such as methylene, ethylene, trimethylene, tetramethylene, pentamethylene, hexamethylene, methylmethylene, 1-methylethylene, 2-methylethylene, 1,1-dimethylethylene, 2,2-dimethylethylene, 2-methyltrimethylene, ethylethylene, 1,2-dimethylethylene, and 1,1,2,2-tetramethylethylene. The C₁₋₆ alkylene is C₁₋₄ alkylene in an aspect; methylene or ethylene in another aspect; ethylene in a further aspect; and methylene in yet another aspect.

The term "C₃₋₈ cycloalkyl" refers to a C₃₋₈ saturated hydrocarbon ring group, which may have a cross-link or form a spiro ring, such as cyclopropyl, cyclobutyl, cyclopentyl, cyclohexyl, cycloheptyl, cyclooctyl, bicyclo[1.1.1]pentyl, bicyclo[3.1.0]hexyl, bicyclo[2.2.1]heptyl, bicyclo[3.1.1]heptyl, bicyclo[3.3.0]octyl, bicyclo[2.2.2]octyl, spiro[2.2]pentyl, spiro[3.3]heptyl, and spiro[2.5]octyl. The C₃₋₈ cycloalkyl is a C₃₋₆ saturated hydrocarbon ring group in an aspect; cyclopropyl, cyclobutyl, cyclopentyl, cyclohexyl, or bicyclo[1.1.1]pentyl in an aspect; cyclopropyl in a further aspect; cyclobutyl, cyclopentyl, cyclohexyl, or bicyclo[1.1.1]pentyl in another aspect; cyclobutyl, cyclopentyl, or cyclohexyl in an aspect; cyclobutyl or cyclohexyl in a further aspect; cyclobutyl in a further aspect; cyclopentyl in another aspect; cyclohexyl in still another aspect.

The term "C₅₋₈ cycloalkenyl" refers to a C₅₋₈ hydrocarbon ring group having one double bond in the molecule, which may have a cross-link, such as cyclopentenyl, cyclohexenyl, cycloheptenyl, cyclooctenyl, bicyclo[2.2.1]heptenyl, and bicyclo[2.2.2]octenyl. The C₅₋₈ cycloalkenyl is cyclopentenyl, cyclohexenyl, cycloheptenyl, or bicyclo[2.2.2]octenyl in an aspect; cyclopentenyl, cyclohexenyl, or cycloheptenyl in a further aspect; cyclopentenyl in a further aspect; cyclohexenyl in another aspect; and cycloheptenyl in still another aspect.

If ring A is C₅₋₈ cycloalkenyl, the following formula represents a substructure of formula (I) and formula (Ia): 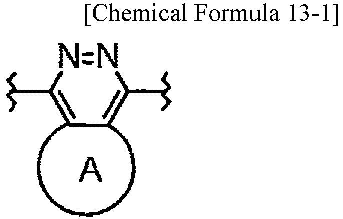
(Each wavy line represents a bonding moiety to a phenyl group and L in the formula (I). The same shall apply hereinafter.)
in which the C₅₋₈ cycloalkenyl is fused with the pyridazine ring at a double-bond site thereof to form, for example, but not limited to, the following substructures.

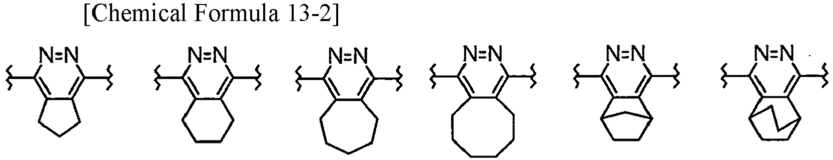

The term "4- to 7-membered saturated heterocyclyl" is a 4- to 7-membered saturated hydrocarbon ring group containing one or more hetero atoms, in particular, oxygen atoms, nitrogen atoms, or sulfur atoms as ring-constituting atoms, and examples thereof include, but are not limited to, oxetanyl, azetidinyl, thietanyl, tetrahydrofuranyl, pyrrolidinyl, tetrahydrothiophenyl, tetrahydropyranyl, piperidinyl, tetrahydrothiopyranyl, oxepanyl, azepanyl, thiepanyl, dioxolanyl, imidazolidinyl, pyrazolidinyl, dithiolanyl, oxazolidinyl, isoxazolidinyl, thiazolidinyl, isothiazolidinyl, dioxanyl, piperazinyl, dithianyl, morpholinyl, thiomorpholinyl, oxathialanyl, dioxepanyl, diazepanyl, dithiepanyl, oxazepanyl, thieazepanyl, and oxathiepanyl. The 4- to 7-membered saturated heterocyclyl is oxetanyl, tetrahydrofuranyl, pyrrolidinyl, tetrahydropyranyl, piperidinyl, or tetrahydrothiopyranyl in an aspect; tetrahydrofuranyl, pyrrolidinyl, tetrahydropyranyl, or piperidinyl in an aspect; pyrrolidinyl, tetrahydropyranyl, or piperidinyl in a further aspect; tetrahydropyranyl or piperidinyl in a further aspect; tetrahydrofuranyl or tetrahydropyranyl in a further aspect; tetrahydrofuranyl in an aspect; or tetrahydropyranyl in a further aspect. The 4- to 7-membered saturated heterocyclyl is pyrrolidinyl, or piperidinyl in another aspect; pyrrolidinyl in still another aspect; or piperidinyl in yet another aspect. The 4- to 7-membered saturated heterocyclyl is oxetanyl in a further aspect.

The term "5- to 11-membered partially unsaturated heterocyclyl" refers to a 5- to 11-membered hydrocarbon ring group containing one or more hetero atoms, in particular, oxygen atoms, nitrogen atoms, or sulfur atoms as ring-constituting atoms, with one double bond in the molecule, which may have a cross-link or form a spiro ring. Examples thereof include, but are not limited to, dihydrofuranyl, dihydropyrrolyl, dihydrothiophenyl, dihydropyranyl, tetrahydropyridyl, dihydrothiopyranyl, tetrahydrooxepinyl, tetrahydroazepinyl, tetrahydrothiepinyl, tetrahydrooxocinyl, hexahydroazocinyl, tetrahydrothiocinyl, dioxolyl, dihydroimidazolyl, dihydropyrazolyl, dithiolyl, dihydrooxazolyl, dihydroisoxazolyl, dihydrothiazolyl, dihydroisothiazolyl, dihydrodioxinyl, tetrahydropyrazinyl, dihydrodithiinyl, dihydrooxazinyl, dihydrothiazinyl, dihydrooxathiinyl, dihydrodioxepinyl, tetrahydrodiazepinyl, dihydrodithiepinyl, tetrahydrooxazepinyl, tetrahydrothiazepinyl, dihydrooxathiepinyl, 2-oxaspiro[3.5]nonenyl, 2-oxaspiro[4.5]decenyl, and 3-oxaspiro[5.5]undecenyl. The 5- to 11-membered partially unsaturated heterocyclyl is, in an aspect, dihydrofuranyl, dihydropyrrolyl, dihydrothiophenyl, dihydropyranyl, tetrahydropyridyl, dihydrothiopyranyl, tetrahydrooxepinyl, or 2-oxaspiro[3.5]nonenyl. The 5- to 11-membered partially unsaturated heterocyclyl is 5- to 8-membered partially unsaturated heterocyclyl in an aspect; dihydrofuranyl, dihydropyrrolyl, dihydrothiophenyl, dihydropyranyl, tetrahydropyridyl, dihydrothiopyranyl, tetrahydrooxepinyl, tetrahydroazepinyl, tetrahydrothiepinyl, tetrahydrooxocinyl, hexahydroazocinyl, tetrahydrothiocinyl, dioxolyl, dihydroimidazolyl, dihydropyrazolyl, dithiolyl, dihydrooxazolyl, dihydroisoxazolyl, dihydrothiazolyl, dihydroisothiazolyl, dihydrodioxinyl, tetrahydropyrazinyl, dihydrodithiinyl, dihydrooxazinyl, dihydrothiazinyl, dihydrooxathiinyl, dihydrodioxepinyl, tetrahydrodiazepinyl, dihydrodithiepinyl, tetrahydrooxazepinyl, tetrahydrothiazepinyl, or dihydrooxathiepinyl in an aspect; dihydropyrrolyl, dihydropyranyl, tetrahydropyridyl, or tetrahydrooxepinyl in an aspect; and dihydropyranyl, tetrahydropyridyl, or tetrahydrooxepinyl in a further aspect. The 5- to 11-membered partially unsaturated heterocyclyl is dihydrofuranyl, dihydrothiophenyl, dihydropyranyl, tetrahydropyridyl, or tetrahydrooxepinyl in another aspect; dihydrofuranyl, dihydropyranyl, tetrahydrooxepinyl, or 2-oxaspiro[3.5]nonenyl in another aspect; dihydropyranyl in another aspect; tetrahydropyridyl in yet another aspect; and tetrahydrooxepinyl in still another aspect.

If ring A is 5- to 11-membered partially unsaturated heterocyclyl, the following formula represents a substructure of formula (I) and formula (Ia): 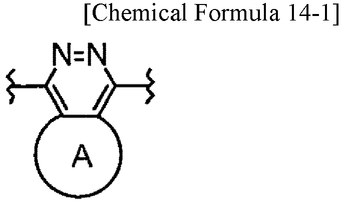 in which the 5- to 11-membered partially unsaturated heterocyclyl is fused with the pyridazine ring at a double-bond site thereof to form, for example, but not limited to, the following substructures.

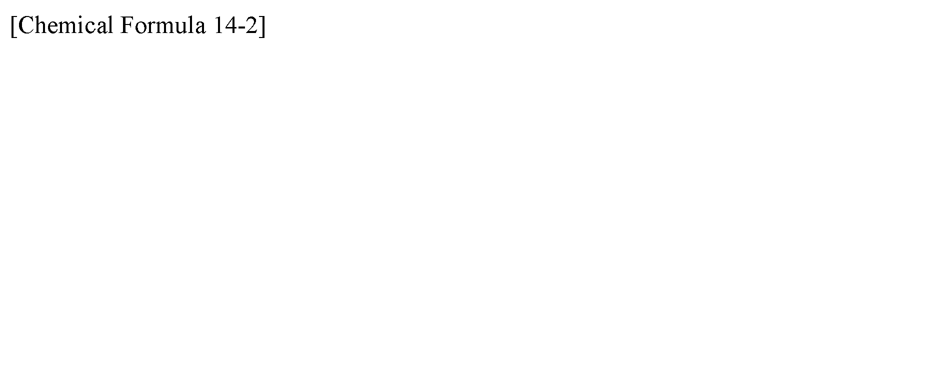

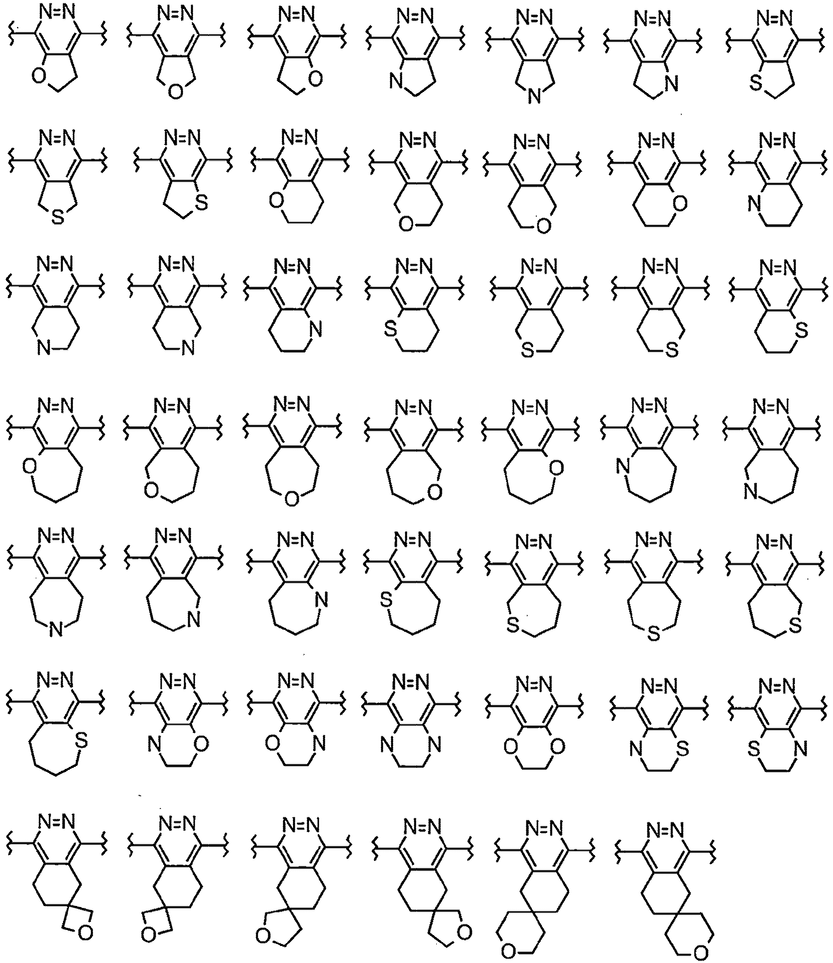

The term "aryl" refers to a C₆₋₁₄ mono- to tricyclic aromatic hydrocarbon group, which includes a bi- to tricyclic aromatic hydrocarbon group fused with C₅₋₈ cycloalkene at a double-bond site thereof, such as phenyl, naphthyl, tetrahydronaphthyl, indenyl, and fluorenyl. In an aspect, the aryl is phenyl.

If ring A is aryl, the following formula represents a substructure of formula (I) and formula (Ia): 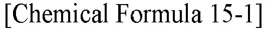 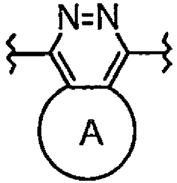 in which the aryl has a benzene ring fused with a pyridazine ring to form, for example, but not limited to, the following substructures.

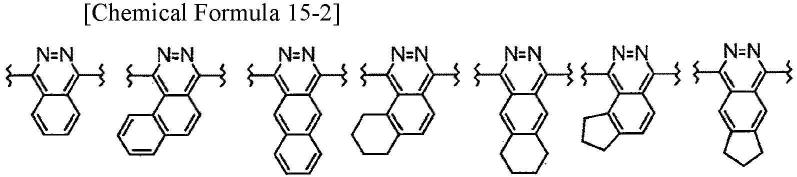

The term "heteroaryl" refers to a 5 or 6-membered aromatic hydrocarbon group containing 1 to 4 hetero atoms selected from oxygen, sulfur, and nitrogen, such as pyrrolyl, furyl, thienyl, pyrazolyl, imidazolyl, isoxazolyl, oxazolyl, isothiazolyl, thiazolyl, triazolyl, oxadiazolyl, thiadiazolyl, pyridyl, pyrazinyl, pyrimidinyl, pyridazinyl, triazinyl, and tetrazinyl. The heteroaryl is thienyl or pyridyl in an aspect; pyridyl in a further aspect; or thienyl in another aspect.

If ring A is heteroaryl, the following formula represents a substructure of formula (I) and formula (Ia): 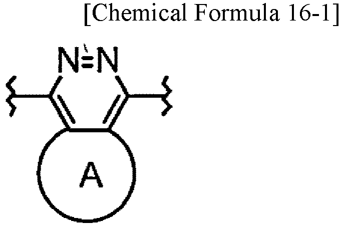 in which the heteroaryl is fused with pyridazine ring to form, for example, but not limited to, the following substructures.

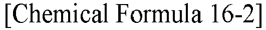

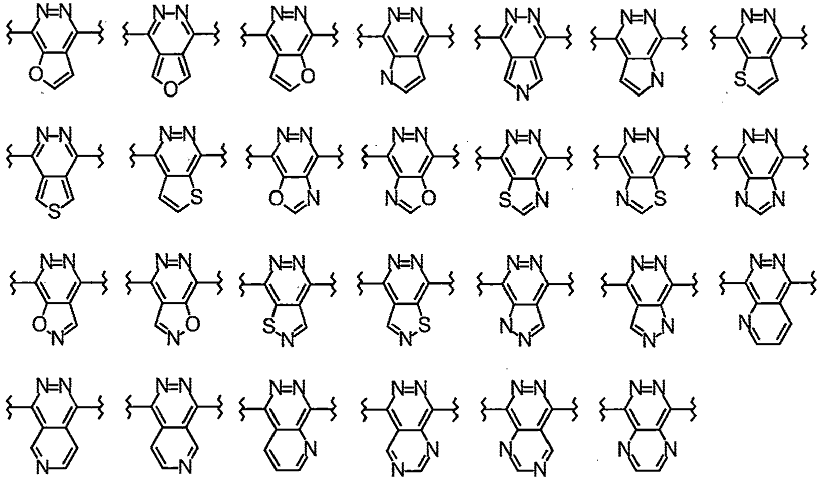

The term "halogen" means F, Cl, Br, or I. An aspect thereof is F or Cl; another aspect is F; and still another aspect is Cl.

The term "halogeno C₁₋₆ alkyl" refers to a linear or branched C₁₋₆ alkyl group substituted with one or more halogens. Examples thereof include trifluoromethyl, trifluoroethyl, trifluoropropyl, 2-fluoro-2-methylpropyl, difluoromethyl, difluoroethyl, fluoromethyl, and chloromethyl. The halogeno C₁₋₆ alkyl is difluoroethyl, trifluoromethyl, or difluoromethyl in one aspect; trifluoromethyl or difluoromethyl in another aspect; and trifluoromethyl in still another aspect; and difluoromethyl in a further aspect.

As used herein, the term "optionally substituted" means being unsubstituted or "substituted with one or more substituents". The substitution may occur at any position where hydrogen is normally present in the group of interest.

Even if a combination is not specifically described, one or more aspects may be combined with another aspect.

As used herein, the term "inflammatory disease and/or neurodegenerative disease" refers to an inflammatory disease and a neurodegenerative disease in an aspect; an inflammatory disease in a further aspect; and a neurodegenerative disease in an aspect.

As used herein, the term "inflammatory disease" refers to, but is not limited to, an autoinflammatory disease including cryopyrin-associated periodic syndrome (CAPS), gout, and pseudogout; and a disease including nonalcoholic steatohepatitis (NASH). The inflammatory disease is an autoinflammatory disease in an aspect; CAPS in another aspect; and gout in yet another aspect. Note that the term "cryopyrin-associated periodic syndrome (CAPS)" refers to a disease selected from the group consisting of the familial cold urticaria (FCAS), Muckle-Wells syndrome (MWS), and neonatal onset multisystem inflammatory diseases/chronic infantile neurologic cutaneous, and articular syndrome (NOMID/CINCA syndrome).

As used herein, the term "neurodegenerative disease" refers to a group of diseases including, but not limited to, α-synucleinopathies including Parkinson's disease, multiple system atrophy, and Lewy body dementia; Alzheimer's disease; amyotrophic lateral sclerosis; and multiple sclerosis. The neurodegenerative disease is Alzheimer's disease, multiple sclerosis, and amyotrophic lateral sclerosis in an aspect; or multiple sclerosis in a further aspect. The neurodegenerative disease is α-synucleinopathy in another aspect; Parkinson's disease in yet another aspect; multiple system atrophy in still another aspect; and Lewy body dementia in still another aspect.

As used herein, the term "treatment" includes both "treatment for therapeutic purposes" and "treatment for prophylactic purposes". The term "treatment for therapeutic purposes" means, for example, alleviating symptoms, altering the course of a disease, and prolonging life, and the term "treatment for prophylactic purposes" means reducing the likelihood of developing a disease in a subject at risk of developing the disease. The "subject at risk of developing the disease" means an individual with known risk factors who is more likely to develop the disease than the general population.

Aspects of the compound of formula (I) or a salt thereof in the present invention are shown below.
(1-1) A compound of formula (I) or a salt thereof (n and m represent the numbers of substituents R¹ and R², respectively. The same shall apply hereinafter.). 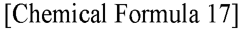 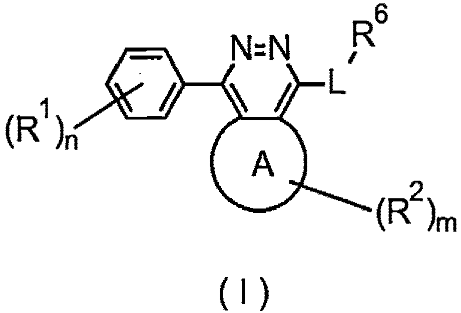 When ring A is aryl or heteroaryl, however, formula (I) is formula (Ia) (k represents the number of substituents R^{1a}. The same shall apply hereinafter.). 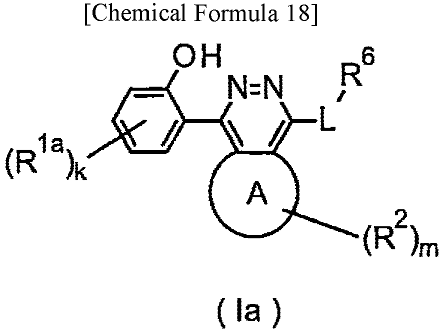
(1-2) A compound of formula (Ia) or a salt thereof.
(1-3) A compound of formula (Ib), (Ic), (Id), (Ie), (If), (Ig), (Ih), (Ii), (Ij), (Ik), (Im), (In), (Io), (Ip), (Iq), (Ir), (Is), (It), (Iu), (Iv), (Iw), (Ix), or (Iy) or a salt thereof. 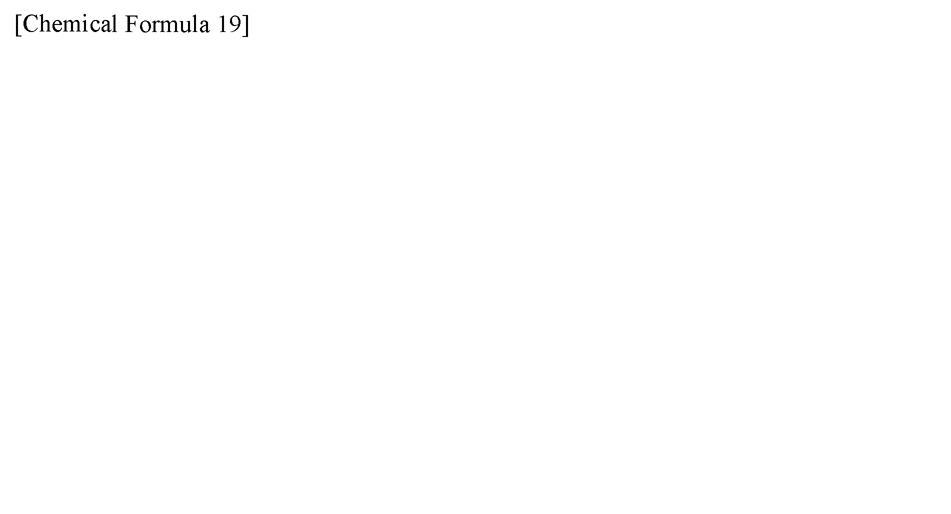 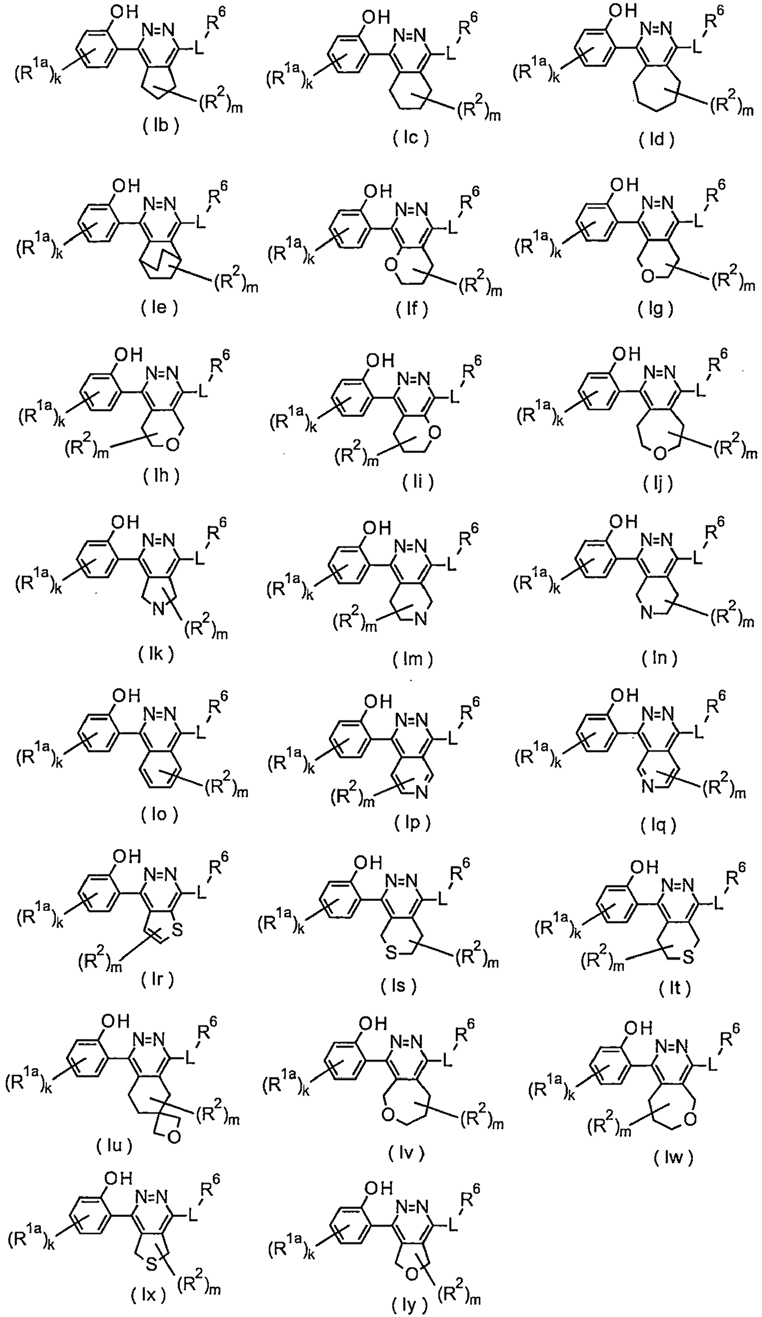
(1-4) A compound of formula (Ib), (Ic), (Id), (Ie), (If), (Ig), (Ih), (Ii), (Ij), (Ik), (Im), (In), (Is), (It), (Iu), (Iv), or (Iw), (Ix), or (Iy) or a salt thereof.
(1-5) A compound of formula (Ib), (Ic), (Id), (If), (Ig), (Ih), (Ii), (Ij), (Ik), (Im), (In), (Is), (It), (Iv), (Iw), (Ix), or (Iy) or a salt thereof.
(1-6) A compound of formula (Ic), (Ih), (Ij), (Im), (Ix), or (Iy) or a salt thereof.
(1-7) A compound of formula (Ib), (Ic), (Id), (Ie), (If), (Ig), (Ih), (Ii), (Ij), (Ik), (Im), (In), (Io), (Ip), (Iq), or (Ir) or a salt thereof.
(1-8) A compound of formula (Ib), (Ic), (Id), (Ie), (If), (Ig), (Ih), (Ii), (Ij), (Ik), (Im), or (In) or a salt thereof.
(1-9) A compound of formula (Ib), (Ic), (Id), or (Ie) or a salt thereof.
(1-10) A compound of formula (Ib) or a salt thereof.
(1-11) A compound of formula (Ic) or a salt thereof.
(1-12) A compound of formula (Id) or a salt thereof.
(1-13) A compound of formula (If), (Ig), (Ih), (Ii), (Ij), (Ik), (Im), or (In) or a salt thereof.
(1-14) A compound of formula (If), (Ig), (Ih), (Ii), or (Ij) or a salt thereof.
(1-15) A compound of formula (Ig), (Ih), or (Ij) or a salt thereof.
(1-16) A compound of formula (Ig) or a salt thereof.
(1-17) A compound of formula (Ih) or a salt thereof.
(1-18) A compound of formula (Ij) or a salt thereof.
(1-19) A compound of formula (Ik), (Im), or (In) or a salt thereof.
(1-20) A compound of formula (Ik) or a salt thereof.
(1-21) A compound of formula (Im) or a salt thereof.
(1-22) A compound of formula (In) or a salt thereof.
(1-23) A compound of formula (Io), (Ip), (Iq), or (Ir) or a salt thereof.
(1-24) A compound of formula (Io) or a salt thereof.
(1-25) A compound of formula (Ip) or a salt thereof.
(1-26) A compound of formula (Iq) or a salt thereof.
(1-27) A compound of formula (Ir) or a salt thereof.
(1-28) A compound of formula (Ic), (Ig), (Ih), (Ij), or (Im) or a salt thereof.
(1-29) A compound of formula (Ic), (Ih), (Ij), or (Im) or a salt thereof.
(1-30) A compound of formula (Ic) or formula (Ih) or a salt thereof.
(1-31) A compound of formula (Ix) or a salt thereof.
(1-32) A compound of formula (Iy) or a salt thereof.
(1-33) A compound of formula (If), (Ig), (Ih), (Ii), (Ij), (Ik), (Im), (In), (Is), (It), (Iu), (Iv), or (Iw), (Ix), or (Iy) or a salt thereof.
(1-34) A compound of formula (If), (Ig), (Ih), (Ii), (Ij), (Iu), (Iv), (Iw), or (Iy) or a salt thereof.
(1-35) A compound of formula (Ih), (Ij), (Im), (Ix), or (Iy) or a salt thereof.
(1-36) A compound of formula (If), (Ig), (Ih), (Ii), (Ij), (Iv), (Iw), or (Iy) or a salt thereof.
(2-1) The compound or a salt thereof, wherein ring A is C₅₋₈ cycloalkenyl, 5- to 11-membered partially unsaturated heterocyclyl, aryl, or heteroaryl, provided that when ring A is aryl or heteroaryl, formula (I) is formula (Ia), wherein R^{1a}, which are identical or different from each other, are C₁₋₆ alkyl, halogeno C₁₋₆ alkyl, C₃₋₈ cycloalkyl, halogen, cyano, -O-C₁₋₆ alkyl, -O-halogeno C₁₋₆ alkyl, or -O-C₃₋₈ cycloalkyl; and k is an integer of 0 to 3.
(2-2) The compound or a salt thereof, wherein ring A is C₅₋₈ cycloalkenyl, 5- to 8-membered partially unsaturated heterocyclyl, aryl, or heteroaryl, provided that when ring A is aryl or heteroaryl, formula (I) is formula (Ia), wherein R^{1a}, which are identical or different from each other, are C₁₋₆ alkyl, halogeno C₁₋₆ alkyl, C₃₋₈ cycloalkyl, halogen, cyano, -O-C₁₋₆ alkyl, -O-halogeno C₁₋₆ alkyl, or -O-C₃₋₈ cycloalkyl; and k is an integer of 0 to 3.
(2-3) The compound or a salt thereof, wherein ring A is C₅₋₈ cycloalkenyl or 5- to 8-membered partially unsaturated heterocyclyl.
(2-4) The compound or a salt thereof, wherein ring A is C₅₋₈ cycloalkenyl.
(2-5) The compound or a salt thereof, wherein ring A is 5- to 8-membered partially unsaturated heterocyclyl.
(2-6) The compound or a salt thereof, wherein ring A is C₅₋₈ cycloalkenyl, 5- to 11-membered partially unsaturated heterocyclyl, aryl, or heteroaryl, provided that when ring A is aryl or heteroaryl, formula (I) is formula (Ia), wherein R^{1a}, which are identical or different from each other, are C₁₋₆ alkyl, halogeno C₁₋₆ alkyl, C₃₋₈ cycloalkyl, halogen, -O-C₁₋₆ alkyl, or -O-halogeno C₁₋₆ alkyl; and k is an integer of 0 to 2.
(2-7) The compound or a salt thereof, in which ring A is C₅₋₈ cycloalkenyl or 5- to 11-membered partially unsaturated heterocyclyl.
(2-8) The compound or a salt thereof, wherein ring A is aryl or heteroaryl, provided that formula (I) is formula (Ia), wherein R^{1a}, which are identical or different from each other, are C₁₋₆ alkyl, halogeno C₁₋₆ alkyl, C₃₋₈ cycloalkyl, halogen, cyano, -O-C₁₋₆ alkyl, -O-halogeno C₁₋₆ alkyl, or -O-C₃₋₈ cycloalkyl; and k is an integer of 0 to 3.
(2-9) The compound or a salt thereof, wherein ring A is aryl, provided that formula (I) is formula (Ia), wherein R^{1a}, which are identical or different from each other, are C₁₋₆ alkyl, halogeno C₁₋₆ alkyl, C₃₋₈ cycloalkyl, halogen, cyano, -O-C₁₋₆ alkyl, -O-halogeno C₁₋₆ alkyl, or -O-C₃₋₈ cycloalkyl; and k is an integer of 0 to 3.
(2-10) The compound or a salt thereof, wherein ring A is heteroaryl, provided that formula (I) is formula (Ia), wherein R^{1a}, which are identical or different from each other, are C₁₋₆ alkyl, halogeno C₁₋₆ alkyl, C₃₋₈ cycloalkyl, halogen, cyano, -O-C₁₋₆ alkyl, -O-halogeno C₁₋₆ alkyl, or -O-C₃₋₈ cycloalkyl; and k is an integer of 0 to 3.
(2-11) The compound or a salt thereof, wherein ring A is 5- to 11-membered partially unsaturated heterocyclyl.
(3-1) The compound or a salt thereof, wherein R¹, which are identical or different from each other, are OH, C₁₋₆ alkyl, halogeno C₁₋₆ alkyl, C₃₋₈ cycloalkyl, halogen, cyano, -O-C₁₋₆ alkyl, -O-halogeno C₁₋₆ alkyl, or -O-C₃₋₈ cycloalkyl.
(3-2) The compound or a salt thereof, wherein R¹, which are identical or different from each other, are OH, C₁₋₆ alkyl, halogeno C₁₋₆ alkyl, C₃₋₈ cycloalkyl, halogen, -O-C₁₋₆ alkyl, or -O-halogeno C₁₋₆ alkyl.
(4-1) The compound or a salt thereof, wherein R^{1a}, which are identical or different from each other, are C₁₋₆ alkyl, halogeno C₁₋₆ alkyl, C₃₋₈ cycloalkyl, halogen, cyano, -O-C₁₋₆ alkyl, -O-halogeno C₁₋₆ alkyl, or -O-C₃₋₈ cycloalkyl.
(4-2) The compound or a salt thereof, wherein R^{1a}, which are identical or different from each other, are C₁₋₆ alkyl, halogeno C₁₋₆ alkyl, C₃₋₈ cycloalkyl, halogen, -O-C₁₋₆ alkyl, or -O-halogeno C₁₋₆ alkyl.
(4-3) The compound or a salt thereof, wherein R^{1a}, which are identical or different from each other, are halogeno C₁₋₆ alkyl, halogen, or -O-halogeno C₁₋₆ alkyl.
(4-4) The compound or a salt thereof, wherein R^{1a} is halogeno C₁₋₆ alkyl.
(4-5) The compound or a salt thereof, wherein R^{1a} is halogen.
(4-6) A compound or a salt thereof, wherein R^{1a} is -O-halogeno C₁₋₆ alkyl.
(4-7) The compound or a salt thereof, wherein R^{1a} is C₁₋₆ alkyl.
(4-8) The compound or a salt thereof, wherein R^{1a} is C₃₋₈ cycloalkyl.
(4-9) The compound or a salt thereof, wherein R^{1a} is -O-C₁₋₆ alkyl.
(5-1) The compound or a salt thereof, wherein R², which are identical or different from each other, are C₁₋₆ alkyl, -C₁₋₆ alkylene-aryl, C₃₋₈ cycloalkyl, halogen, -O-C₁₋₆ alkyl, -O-halogeno C₁₋₆ alkyl, oxo, -C(O)-C₁₋₆ alkyl, or -S(O)₂-C₁₋₆ alkyl.
(5-2) The compound or a salt thereof, wherein R², which are identical or different from each other, are C₁₋₆ alkyl, -C₁₋₆ alkylene-aryl, C₃₋₈ cycloalkyl, halogen, -O-C₁₋₆ alkyl, -O-halogeno C₁₋₆ alkyl, -C(O)-C₁₋₆ alkyl, or -S(O)₂-C₁₋₆ alkyl.
(5-3) The compound or a salt thereof, wherein R², which are identical or different from each other, are C₁₋₆ alkyl, -C₁₋₆ alkylene-aryl, oxo, -C(O)-C₁₋₆ alkyl, or -S(O)₂-C₁₋₆ alkyl.
(5-4) The compound or a salt thereof, wherein R², which are identical or different from each other, are C₁₋₆ alkyl, -C₁₋₆ alkylene-aryl, -C(O)-C₁₋₆ alkyl, or -S(O)₂-C₁₋₆ alkyl.
(5-5) The compound or a salt thereof, wherein R², which are identical or different from each other, are C₁₋₆ alkyl, oxo, -C(O)-C₁₋₆ alkyl, or -S(O)₂-C₁₋₆ alkyl.
(5-6) The compound or a salt thereof, wherein R², which are identical or different from each other, are C₁₋₆ alkyl, -C(O)-C₁₋₆ alkyl, or -S(O)₂-C₁₋₆ alkyl.
(5-7) The compound or a salt thereof, wherein R² is C₁₋₆ alkyl.
(5-8) The compound or a salt thereof, wherein R² is -C(O)-C₁₋₆ alkyl.
(5-9) The compound or a salt thereof, wherein R² is -S(O)₂-C₁₋₆ alkyl.
(5-10) The compound or a salt thereof, wherein R² is oxo.
(6-1) The compound or a salt thereof, wherein L is -NR³-, -O-, or -CR⁴R⁵-.
(6-2) The compound or a salt thereof, wherein L is -NR³- or -O-.
(6-3) The compound or a salt thereof, wherein L is -NR³-.
(6-4) The compound or a salt thereof, wherein L is -O-.
(6-5) The compound or a salt thereof, wherein L is -CR⁴R⁵-.
(7-1) The compound or a salt thereof, wherein R³ is H or C₁₋₆ alkyl.
(7-2) The compound or a salt thereof, wherein R³ is H.
(7-3) The compound or a salt thereof, wherein R³ is C₁₋₆ alkyl.
(8-1) The compound or a salt thereof, wherein R⁴ and R⁵, which are identical or different from each other, are H or C₁₋₆ alkyl.
(8-2) The compound or a salt thereof, wherein R⁴ and R⁵ are each H.
(8-3) The compound or a salt thereof, wherein R⁴ and R⁵ are each C₁₋₆ alkyl.
(9-1) The compound or a salt thereof, wherein R⁶ is C₁₋₆ alkyl substituted with one to four identical or different R⁷, -C₁₋₆ alkylene-(C₃₋₈ cycloalkyl optionally substituted with one to four identical or different R⁸), -C₁₋₆ alkylene-(4- to 7-membered saturated heterocyclyl optionally substituted with one to four identical or different R⁹), C₃₋₈ cycloalkyl optionally substituted with one to four identical or different R¹⁰, or 4- to 7-membered saturated heterocyclyl optionally substituted with one to four identical or different R¹¹.
(9-2) The compound or a salt thereof, wherein R⁶ is -C₁₋₆ alkylene-(C₃₋₈ cycloalkyl optionally substituted with one to four identical or different R⁸), -C₁₋₆ alkylene-(4- to 7-membered saturated heterocyclyl optionally substituted with one to four identical or different R⁹), C₃₋₈ cycloalkyl optionally substituted with one to four identical or different R¹⁰, or 4- to 7-membered saturated heterocyclyl optionally substituted with one to four identical or different R¹¹.
(9-3) The compound or a salt thereof, wherein R⁶ is C₁₋₆ alkyl substituted with one R⁷, -C₁₋₆ alkylene-(C₃₋₈ cycloalkyl optionally substituted with one R⁸), -C₁₋₆ alkylene-(4- to 7-membered saturated heterocyclyl), C₃₋₈ cycloalkyl optionally substituted with one or two identical or different R¹⁰, or 4- to 7-membered saturated heterocyclyl optionally substituted with one or two identical or different R¹¹.
(9-4) The compound or a salt thereof, wherein R⁶ is C₃₋₈ cycloalkyl optionally substituted with one to four identical or different R¹⁰ or 4- to 7-membered saturated heterocyclyl optionally substituted with one to four identical or different R¹¹.
(9-5) The compound or a salt thereof, wherein R⁶ is C₁₋₆ alkyl substituted one to four identical or different R⁷.
(9-6) The compound or a salt thereof, wherein R⁶ is C₁₋₆ alkyl substituted with 1 to 3 identical or different R⁷.
(9-7) The compound or a salt thereof, wherein R⁶ is C₁₋₆ alkyl substituted with one or two identical or different R⁷.
(9-8) The compound or a salt thereof, wherein R⁶ is C₁₋₆ alkyl substituted with one R⁷.
(9-9) The compound or a salt thereof, wherein R⁶ is -C₁₋₆ alkylene-(C₃₋₈ cycloalkyl optionally substituted with one to four identical or different R⁸).
(9-10) The compound or a salt thereof, wherein R⁶ is -C₁₋₆ alkylene-(C₃₋₈ cycloalkyl optionally substituted with one to three identical or different R⁸).
(9-11) The compound or a salt thereof, wherein R⁶ is -C₁₋₆ alkylene-(C₃₋₈ cycloalkyl optionally substituted with one or two identical or different R⁸).
(9-12) The compound or a salt thereof, wherein R⁶ is -C₁₋₆ alkylene-(C₃₋₈ cycloalkyl optionally substituted with one R⁸).
(9-13) The compound or a salt thereof, wherein R⁶ is -C₁₋₆ alkylene-C₃₋₈ cycloalkyl.
(9-14) The compound or a salt thereof, wherein R⁶ is -C₁₋₆ alkylene-(4- to 7-membered saturated heterocyclyl optionally substituted with one to four identical or different R⁹).
(9-15) The compound or a salt thereof, wherein R⁶ is -C₁₋₆ alkylene-(4- to 7-membered saturated heterocyclyl optionally substituted with one to three identical or different R⁹).
(9-16) The compound or a salt thereof, wherein R⁶ is -C₁₋₆ alkylene-(4- to 7-membered saturated heterocyclyl optionally substituted with one or two identical or different R⁹).
(9-17) The compound or a salt thereof, wherein R⁶ is -C₁₋₆ alkylene-(4- to 7-membered saturated heterocyclyl optionally substituted with one R⁹).
(9-18) The compound or a salt thereof, wherein R⁶ is -C₁₋₆ alkylene-(4- to 7-membered saturated heterocyclyl).
(9-19) The compound or a salt thereof, wherein R⁶ is C₃₋₈ cycloalkyl optionally substituted with one to four identical or different R¹⁰.
(9-20) The compound or a salt thereof, wherein R⁶ is C₃₋₈ cycloalkyl optionally substituted with one to three identical or different R¹⁰.
(9-21) The compound or a salt thereof, wherein R⁶ is C₃₋₈ cycloalkyl optionally substituted with one or two identical or different R¹⁰.
(9-22) The compound or a salt thereof, wherein R⁶ is C₃₋₈ cycloalkyl optionally substituted with one R¹⁰.
(9-23) The compound or a salt thereof, wherein R⁶ is C₃₋₈ cycloalkyl.
(9-24) The compound or a salt thereof, wherein R⁶ is 4- to 7-membered saturated heterocyclyl optionally substituted with one to four identical or different R¹¹.
(9-25) The compound or a salt thereof, wherein R6 is 4- to 7-membered saturated heterocyclyl optionally substituted with one to three identical or different R¹¹.
(9-26) The compound or a salt thereof, wherein R⁶ is 4- to 7-membered saturated heterocyclyl optionally substituted with one or two identical or different R¹¹.
(9-27) The compound or a salt thereof, wherein R⁶ is 4- to 7-membered saturated heterocyclyl optionally substituted with one R¹¹.
(9-28) The compound or a salt thereof, wherein R⁶ is 4- to 7-membered saturated heterocyclyl.
(9-29) The compound or a salt thereof, wherein R⁶ is C₁₋₆ alkyl substituted with one R⁷, C₃₋₈ cycloalkyl optionally substituted with one or two identical or different R¹⁰, or 4- to 7-membered saturated heterocyclyl optionally substituted with one or two identical or different R¹¹.
(9-30) The compound or a salt thereof, wherein R⁶ is C₃₋₈ cycloalkyl optionally substituted with one or two identical or different R¹⁰ or 4- to 7-membered saturated heterocyclyl optionally substituted with one or two identical or different R¹¹.
(9-31) The compound or a salt thereof, wherein R⁶ is C₃₋₈ cycloalkyl optionally substituted with one R¹⁰ or 4- to 7-membered saturated heterocyclyl optionally substituted with one R¹¹.
(10-1) The compound or a salt thereof, wherein R⁷is -OR¹², -NR¹³R¹⁴, halogen, or cyano.
(10-2) The compound or a salt thereof, wherein R⁷ is -OR¹² or -NR¹³R¹⁴.
(10-3) The compound or a salt thereof, wherein R⁷is -OR¹².
(10-4) The compound or a salt thereof, wherein R⁷is -NR¹³R¹⁴.
(10-5) The compound or a salt thereof, wherein R⁷ is halogen.
(10-6) The compound or a salt thereof, wherein R⁷is cyano.
(11-1) The compound or a salt thereof, wherein R⁸ is C₁₋₆ alkyl, halogeno C₁₋₆ alkyl, C₃₋₈ cycloalkyl, -OR¹², -NR¹³R¹⁴, -C₁₋₆ alkylene-OR¹², -C₁₋₆ alkylene-NR¹³R¹⁴, halogen, or cyano.
(11-2) The compound or a salt thereof, wherein R⁸ is -OR¹², -NR¹³R¹⁴, -C₁₋₆ alkylene-OR¹², or -C₁₋₆ alkylene-NR¹³R¹⁴.
(11-3) The compound or a salt thereof, wherein R⁸ is C₁₋₆ alkyl, -OR¹², -NR¹³R¹⁴, -C₁₋₆ alkylene-OR¹², halogen, or cyano.
(11-4) The compound or a salt thereof, wherein R⁸ is -OR¹² or -C₁₋₆ alkylene-OR¹².
(11-5) The compound or a salt thereof, wherein R⁸ is -OR¹².
(11-6) The compound or a salt thereof, wherein R⁸ is -C₁₋₆ alkylene-OR¹².
(12-1) The compound or a salt thereof, wherein R⁹ is C₁₋₆ alkyl, halogeno C₁₋₆ alkyl, C₃₋₈ cycloalkyl, 4- to 7-membered saturated heterocyclyl, -OR¹², -NR¹³R¹⁴, -C₁₋₆ alkylene-OR¹², -C₁₋₆ alkylene-NR¹³R¹⁴, halogen, cyano, oxo, -C(O)-C₁₋₆ alkyl, or -S(O)₂-C₁₋₆ alkyl.
(12-2) The compound or a salt thereof, wherein R⁹ is C₁₋₆ alkyl, halogeno C₁₋₆ alkyl, C₃₋₈ cycloalkyl, 4- to 7-membered saturated heterocyclyl, -OR¹², oxo, or -C(O)-C₁₋₆ alkyl.
(13-1) The compound or a salt thereof, wherein R¹⁰ is C₁₋₆ alkyl, halogeno C₁₋₆ alkyl, C₃₋₈ cycloalkyl, -OR¹², -NR¹³R¹⁴, -C₁₋₆ alkylene-OR¹², -C₁₋₆ alkylene-NR¹³R¹⁴, halogen, or cyano.
(13-2) The compound or a salt thereof, wherein R¹⁰ is C₁₋₆ alkyl, -OR¹², -NR¹³R¹⁴, -C₁₋₆ alkylene-OR¹², halogen, or cyano.
(13-3) The compound or a salt thereof, wherein R¹⁰ is C₁₋₆ alkyl or -OR¹².
(13-4) The compound or a salt thereof, wherein R¹⁰ is C₁₋₆ alkyl.
(13-5) The compound or a salt thereof, wherein R¹⁰ is -OR¹².
(13-6) The compound or a salt thereof, wherein R¹⁰ is -NR¹³R¹⁴.
(13-7) The compound or a salt thereof, wherein R¹⁰ is -C₁₋₆ alkylene-OR¹².
(13-8) The compound or a salt thereof, wherein R¹⁰ is halogen.
(13-9) The compound or a salt thereof, wherein R¹⁰ is cyano.
(13-10) The compound or a salt thereof, wherein R¹⁰ is C₁₋₆ alkyl, -OR¹², -NR¹³R¹⁴, or cyano.
(13-11) The compound or a salt thereof, wherein R¹⁰ is C₁₋₆ alkyl, -OR¹², or cyano.
(13-12) The compound or a salt thereof, wherein R¹⁰ is C₁₋₆ alkyl, -OR¹², -NR¹³R¹⁴, -C₁₋₆ alkylene-OR¹², or cyano.
(14-1) The compound or a salt thereof, wherein R¹¹ is C₁₋₆ alkyl, halogeno C₁₋₆ alkyl, C₃₋₈ cycloalkyl, 4- to 7-membered saturated heterocyclyl, -OR¹², -NR¹³R¹⁴, -C₁₋₆ alkylene-OR¹², -C₁₋₆ alkylene-NR¹³R¹⁴, halogen, cyano, oxo, -C(O)-C₁₋₆ alkyl, or -S(O)₂-C₁₋₆ alkyl.
(14-2) The compound or a salt thereof, wherein R¹¹ is C₁₋₆ alkyl, C₃₋₈ cycloalkyl, 4- to 7-membered saturated heterocyclyl, -OR¹², oxo, or -C(O)-C₁₋₆ alkyl.
(14-3) The compound or a salt thereof, wherein R¹¹ is C₁₋₆ alkyl.
(14-4) The compound or a salt thereof, wherein R¹¹ is C₃₋₈ cycloalkyl.
(14-5) The compound or a salt thereof, wherein R¹¹ is 4- to 7-membered saturated heterocyclyl.
(14-6) The compound or a salt thereof, wherein R¹¹ is -OR¹².
(14-7) The compound or a salt thereof, wherein R¹¹ is oxo.
(14-8) The compound or a salt thereof, wherein R¹¹ is -C(O)-C₁₋₆ alkyl.
(14-9) The compound or a salt thereof, wherein R¹¹ is C₁₋₆ alkyl, halogeno C₁₋₆ alkyl, C₃₋₈ cycloalkyl, 4- to 7-membered saturated heterocyclyl, -OR¹², oxo, or -C(O)-C₁₋₆ alkyl.
(14-10) The compound or a salt thereof, wherein R¹¹ is C₁₋₆ alkyl or -OR¹².
(14-11) The compound or a salt thereof, wherein R¹¹ is C₁₋₆ alkyl, halogeno C₁₋₆ alkyl, C₃₋₈ cycloalkyl, -OR¹², or -C(O)-C₁₋₆ alkyl.
(15-1) The compound or a salt thereof, wherein R¹² is H or C₁₋₆ alkyl.
(15-2) The compound or a salt thereof, wherein R¹² is H.
(15-3) The compound or a salt thereof, wherein R¹² is C₁₋₆ alkyl.
(16-1) The compound or a salt thereof, wherein R¹³ and R¹⁴, which are identical or different from each other, are H, C₁₋₆ alkyl, or -C(O)-C₁₋₆ alkyl.
(16-2) The compound or a salt thereof, wherein R¹³ and R¹⁴, which are identical or different from each other, are H or C₁₋₆ alkyl.
(16-3) The compound or a salt thereof, wherein R¹³ and R¹⁴ are each H.
(16-4) The compound or a salt thereof, wherein R¹³ and R¹⁴ are each H; or R¹³ is -C(O)-C₁₋₆ alkyl, and R¹⁴ is H.
(16-5) The compound or a salt thereof, wherein R¹³ is -C(O)-C₁₋₆ alkyl, and R¹⁴ is H.
(17-1) The compound or a salt thereof, wherein n is an integer of 1 to 4.
(17-2) The compound or a salt thereof, wherein n is an integer of 1 to 3.
(17-3) The compound or a salt thereof, wherein n is 1 or 2.
(17-4) The compound or a salt thereof, wherein n is 2.
(17-5) The compound or a salt thereof, wherein n is 1.
(18-1) The compound or a salt thereof, wherein m is an integer of 0 to 3.
(18-2) The compound or a salt thereof, wherein m is an integer of 0 to 2.
(18-3) The compound or a salt thereof, wherein m is 0 or 1.
(18-4) The compound or a salt thereof, wherein m is 1.
(18-5) The compound or a salt thereof, wherein m is 0.
(19-1) The compound or a salt thereof, wherein k is an integer of 0 to 3.
(19-2) The compound or a salt thereof, wherein k is an integer of 0 to 2.
(19-3) The compound or a salt thereof, wherein k is 0 or 1.
(19-4) The compound or a salt thereof, wherein k is 1.
(19-5) The compound or a salt thereof, wherein k is 0.
(19-6) The compound or a salt thereof, wherein k is 1 or 2.
(20) The compound or a salt thereof, which is a combination of two or more groups of the aspects described in (1-1) to (19-6) above that do not contradict each other. Examples thereof include, but are not limited to, the following combinations.
(20-1) The compound or a salt thereof, which is a combination of (1-1), (2-2), (3-1), (4-1), (5-2), (6-1), (7-1), (8-1), (9-1), (10-1), (11-1), (12-1), (13-1), (14-1), (15-1), (16-1), (17-1), (18-1), and (19-1) above.
(20-2) The compound or a salt thereof, which is a combination of (1-2), (2-2), (4-1), (5-2), (6-1), (7-1), (8-1), (9-1), (10-1), (11-1), (12-1), (13-1), (14-1), (15-1), (16-1), (18-1), and (19-1) above.
(20-3) The compound or a salt thereof, which is a combination of (1-2), (2-3), (4-1), (5-2), (6-1), (7-1), (8-1), (9-1), (10-1), (11-1), (12-1), (13-1), (14-1), (15-1), (16-1), (18-1), and (19-1).
(20-4) The compound or a salt thereof, which is a combination of (1-2), (2-6), (4-2), (5-5), (6-1), (7-2), (8-2), (9-3), (10-2), (11-3), (12-2), (13-2), (14-9), (15-2), (16-4), (18-2), and (19-2) above.
(20-5) The compound or a salt thereof, which is a combination of (1-2), (2-7), (4-2), (5-5), (6-3), (7-2), (9-29), (10-2), (13-2), (14-9), (15-2), (16-4), (18-2), and (19-2) above.
(20-6) The compound or a salt thereof, which is a combination of (1-2), (2-3), (4-3), (5-7), (6-3), (7-2), (9-30), (13-3), (14-10), (15-2), (18-3), and (19-6) above.
(20-7) The compound or a salt thereof, which is a combination of (1-2), (2-11), (4-2), (5-5), (6-3), (7-2), (9-29), (10-2), (13-2), (14-9), (15-2), (16-4), (18-2), and (19-2) above.
(20-8) The compound or a salt thereof, which is a combination of (1-34), (4-2), (5-5), (6-3), (7-2), (9-29), (10-2), (13-2), (14-10), (15-2), (16-4), (18-5), and (19-2) above.
(20-9) The compound or a salt thereof, which is a combination of (1-17), (4-2), (6-3), (7-2), (9-29), (10-2), (13-11), (14-10), (15-2), (16-5), (18-5), and (19-2) above.
(20-10) The compound or a salt thereof, which is a combination of (1-6), (4-3), (5-7), (6-3), (7-2), (9-31), (13-5), (14-3), (15-2), (18-3), and (19-4) above.
(20-11) The compound or a salt thereof, which is a combination of (1-35), (4-3), (5-7), (6-3), (7-2), (9-31), (13-5), (14-3), (15-2), (18-3), and (19-4) above.
(20-12) The compound or a salt thereof, which is a combination of (1-7), (4-2), (5-4), (6-1), (7-2), (8-2), (9-3), (10-2), (11-4), (13-2), (14-2), (15-2), (16-4), (18-3), and (19-2) above.
(20-13) The compound or a salt thereof, which is a combination of (1-28), (4-3), (5-7), (6-3), (7-2), (9-22), (13-5), (15-2), (18-3), and (19-4) above.
(20-14) The compound or a salt thereof, which is a combination of (1-11), (4-4), (6-3), (7-2), (9-22), (13-5), (15-2), (18-5), and (19-4) above.
(20-15) The compound or a salt thereof, which is a combination of (1-17), (4-4), (6-3), (7-2), (9-22), (13-5), (15-2), (18-5), and (19-4) above.
(20-16) The compound or a salt thereof, which is a combination of (1-17), (4-6), (6-3), (7-2), (9-22), (13-5), (15-2), (18-5), and (19-4) above.
(20-17) The compound or a salt thereof, which is a combination of (1-11), (4-6), (6-3), (7-2), (9-22), (13-5), (15-2), (18-5), and (19-4) above.
(20-18) The compound or a salt thereof, which is a combination of (1-21), (4-4), (5-7), (6-3), (7-2), (9-22), (13-5), (15-2), (18-4), and (19-4) above.
(20-19) The compound or a salt thereof, which is a combination of (1-18), (4-4), (6-3), (7-2), (9-22), (13-5), (15-2), (18-5), and (19-4) above.
(20-20) The compound or a salt thereof, which is a combination of (1-17), (4-5), (6-3), (7-2), (9-22), (13-5), (15-2), (18-5), and (19-4) above.
(20-21) The compound or a salt thereof, which is a combination of (1-16), (4-4), (6-3), (7-2), (9-22), (13-5), (15-2), (18-5), and (19-4) above.
(20-22) The compound or a salt thereof which is a combination of (1-16), (4-6), (6-3), (7-2), (9-22), (13-5), (15-2), (18-5), and (19-4) above.
(20-23) The compound or a salt thereof, which is a combination of (1-16), (4-5), (6-3), (7-2), (9-22), (13-5), (15-2), (18-5), and (19-4) above.
(20-24) The compound or a salt thereof, which is a combination of (1-31), (4-4), (6-3), (7-2), (9-22), (13-5), (15-2), (18-5), and (19-4) above.
(20-25) The compound or a salt thereof, which is a combination of (1-32), (4-4), (6-3), (7-2), (9-22), (13-5), (15-2), (18-5), and (19-4) above.
(20-26) The compound or a salt thereof, which is a combination of (1-17), (4-6), (6-3), (7-2), (9-27), (14-3), (18-5), and (19-4) above.

Examples of specific compounds included in the present invention include compounds or salts thereof selected from the group consisting of:
2-(4-{[(1R,2R)-2-hydroxycyclohexyl]amino}-5,6,7,8-tetrahydrophthalazin-1-yl)-5-(trifluoro methyl)phenol;
2-(4-{[(1R,2R)-2-hydroxycyclohexyl]amino}-7,8-dihydro-5H-pyrano[3,4-d]pyridazin-1-yl)-5 -(trifluoromethyl)phenol;
2-(4-{[(1R,2R)-2-hydroxycyclohexyl]amino}-7,8-dihydro-5H-pyrano[3,4-d]pyridazin-1-yl)-5 -(trifluoromethoxy)phenol;
2-(4-{[(1R,2S)-2-hydroxycyclohexyl]amino}-7,8-dihydro-5H-pyrano[3,4-d]pyridazin-1-yl)-5 -(trifluoromethyl)phenol;
2-(4-{[(1R,2R)-2-hydroxycyclohexyl]amino}-5,6,8,9-tetrahydrooxepino[4,5-d]pyridazin-1-yl )-5-(trifluoromethyl)phenol;
5-chloro-2-(4-{[(1R,2R)-2-hydroxycyclohexyl]amino}-7,8-dihydro-5H-pyrano[3,4-d]pyridaz in-1-yl)phenol;
2-(4-{[(1R,2R)-2-hydroxycyclohexyl]amino}-6-methyl-5,6,7,8-tetrahydropyrido[3,4-d]pyrid azin-1-yl)-5-(trifluoromethyl)phenol;
5-(difluoromethoxy)-2-(4-{[(1R,2R)-2-hydroxycyclohexyl]amino}-5,6,7,8-tetrahydrophthala zin-1-yl)phenol;
5-(difluoromethoxy)-2-(4-{[(1R,2R)-2-hydroxycyclohexyl]amino}-7,8-dihydro-5H-pyrano[3 ,4-d]pyridazin-1-yl)phenol;
5-(difluoromethoxy)-2-(4-{[(3R)-1-methylpiperidin-3-yl]amino}-7,8-dihydro-5H-pyrano[3,4 -d]pyridazin-1-yl)phenol;
2-(4-{[(1R,2R)-2-hydroxycyclohexyl]amino}-5,7-dihydrothieno[3,4-d]pyridazin-1-yl)-5-(trif luoromethyl)phenol; and
5-(difluoromethyl)-2-(4-{[(1R,3S)-3-hydroxycyclohexyl]amino}-5,7-dihydrofuro[3,4-d]pyrid azin-1-yl)phenol.

Aspects of the specific compounds included in the present invention include compounds and salts thereof selected from the group consisting of:
2-(4-{[(1R,2R)-2-hydroxycyclohexyl]amino}-5,6,7,8-tetrahydrophthalazin-1-yl)-5-(trifluoro methyl)phenol;
2-(4-{[(1R,2R)-2-hydroxycyclohexyl]amino}-7,8-dihydro-5H-pyrano[3,4-d]pyridazin-1-yl)-5 -(trifluoromethyl)phenol;
2-(4-{[(1R,2R)-2-hydroxycyclohexyl]amino}-7,8-dihydro-5H-pyrano[3,4-d]pyridazin-1-yl)-5 -(trifluoromethoxy)phenol;
2-(4-{[(1R,2S)-2-hydroxycyclohexyl]amino}-7,8-dihydro-5H-pyrano[3,4-d]pyridazin-1-yl)-5 -(trifluoromethyl)phenol;
2-(4-{[(1R,2R)-2-hydroxycyclohexyl]amino}-5,6,8,9-tetrahydrooxepino[4,5-d]pyridazin-1-yl )-5-(trifluoromethyl)phenol;
5-chloro-2-(4-{[(1R,2R)-2-hydroxycyclohexyl]amino}-7,8-dihydro-5H-pyrano[3,4-d]pyridaz in-1-yl)phenol;
2-(4-{[(1R,2R)-2-hydroxycyclohexyl]amino}-6-methyl-5,6,7,8-tetrahydropyrido[3,4-d]pyrid azin-1-yl)-5-(trifluoromethyl)phenol;
5-(difluoromethoxy)-2-(4-{[(1R,2R)-2-hydroxycyclohexyl]amino}-5,6,7,8-tetrahydrophthala zin-1-yl)phenol; and
5-(difluoromethoxy)-2-(4-{[(1R,2R)-2-hydroxycyclohexyl]amino}-7,8-dihydro-5H-pyrano[3 ,4-d]pyridazin-1-yl)phenol.

Aspects of the specific compounds included in the present invention include compounds and salts thereof selected from the group consisting of:
2-(4-{[(1R,2R)-2-hydroxycyclohexyl]amino}-7,8-dihydro-5H-pyrano[3,4-d]pyridazin-1-yl)-5 -(trifluoromethyl)phenol;
2-(4-{[(1R,2R)-2-hydroxycyclohexyl]amino}-7,8-dihydro-5H-pyrano[3,4-d]pyridazin-1-yl)-5 -(trifluoromethoxy)phenol;
2-(4-{[(1R,2S)-2-hydroxycyclohexyl]amino}-7,8-dihydro-5H-pyrano[3,4-d]pyridazin-1-yl)-5 -(trifluoromethyl)phenol;
2-(4-{[(1R,2R)-2-hydroxycyclohexyl]amino}-5,6,8,9-tetrahydrooxepino[4,5-d]pyridazin-1-yl )-5-(trifluoromethyl)phenol;
5-chloro-2-(4-{[(1R,2R)-2-hydroxycyclohexyl]amino}-7,8-dihydro-5H-pyrano[3,4-d]pyridaz in-1-yl)phenol;
2-(4-{[(1R,2R)-2-hydroxycyclohexyl]amino}-6-methyl-5,6,7,8-tetrahydropyrido[3,4-d]pyrid azin-1-yl)-5-(trifluoromethyl)phenol;
5-(difluoromethoxy)-2-(4-{[(1R,2R)-2-hydroxycyclohexyl]amino}-7,8-dihydro-5H-pyrano[3 ,4-d]pyridazin-1-yl)phenol;
5-(difluoromethoxy)-2-(4-{[(3R)-1-methylpiperidin-3-yl]amino}-7,8-dihydro-5H-pyrano[3,4 -d]pyridazin-1-yl)phenol;
2-(4-{[(1R,2R)-2-hydroxycyclohexyl]amino}-5,7-dihydrothieno[3,4-d]pyridazin-1-yl)-5-(trif luoromethyl)phenol; and
5-(difluoromethyl)-2-(4-{[(1R,3S)-3-hydroxycyclohexyl]amino}-5,7-dihydrofuro[3,4-d]pyrid azin-1-yl)phenol.

Aspects of the specific compounds included in the present invention include compounds and salts thereof selected from the group consisting of:
2-(4-{[(1R,2R)-2-hydroxycyclohexyl]amino}-7,8-dihydro-5H-pyrano[3,4-d]pyridazin-1-yl)-5 -(trifluoromethyl)phenol;
2-(4-{[(1R,2R)-2-hydroxycyclohexyl]amino}-7,8-dihydro-5H-pyrano[3,4-d]pyridazin-1-yl)-5 -(trifluoromethoxy)phenol;
2-(4-{[(1R,2S)-2-hydroxycyclohexyl]amino}-7,8-dihydro-5H-pyrano[3,4-d]pyridazin-1-yl)-5 -(trifluoromethyl)phenol;
2-(4-{[(1R,2R)-2-hydroxycyclohexyl]amino}-5,6,8,9-tetrahydrooxepino[4,5-d]pyridazin-1-yl )-5-(trifluoromethyl)phenol;
5-chloro-2-(4-{[(1R,2R)-2-hydroxycyclohexyl]amino}-7,8-dihydro-5H-pyrano[3,4-d]pyridaz in-1-yl)phenol;
5-(difluoromethoxy)-2-(4-{[(1R,2R)-2-hydroxycyclohexyl]amino}-7,8-dihydro-5H-pyrano[3 ,4-d]pyridazin-1-yl)phenol;
5-(difluoromethoxy)-2-(4-{[(3R)-1-methylpiperidin-3-yl]amino}-7,8-dihydro-5H-pyrano[3,4 -d]pyridazin-1-yl)phenol; and
5-(difluoromethyl)-2-(4-{[(1R,3S)-3-hydroxycyclohexyl]amino}-5,7-dihydrofuro[3,4-d]pyrid azin-1-yl)phenol.

Aspects of the specific compounds included in the present invention include compounds and salts thereof selected from the group consisting of:
2-(4-{[(1R,2R)-2-hydroxycyclohexyl]amino}-7,8-dihydro-5H-pyrano[3,4-d]pyridazin-1-yl)-5 -(trifluoromethyl)phenol;
2-(4-{[(1R,2R)-2-hydroxycyclohexyl]amino}-7,8-dihydro-5H-pyrano[3,4-d]pyridazin-1-yl)-5 -(trifluoromethoxy)phenol;
2-(4-{[(1R,2S)-2-hydroxycyclohexyl]amino}-7,8-dihydro-5H-pyrano[3,4-d]pyridazin-1-yl)-5 -(trifluoromethyl)phenol;
5-chloro-2-(4-{[(1R,2R)-2-hydroxycyclohexyl]amino}-7,8-dihydro-5H-pyrano[3,4-d]pyridaz in-1-yl)phenol;
5-(difluoromethoxy)-2-(4-{[(1R,2R)-2-hydroxycyclohexyl]amino}-7,8-dihydro-5H-pyrano[3 ,4-d]pyridazin-1-yl)phenol; and
5-(difluoromethoxy)-2-(4-{[(3R)-1-methylpiperidin-3-yl]amino}-7,8-dihydro-5H-pyrano[3,4 -d]pyridazin-1-yl)phenol.

Examples of specific compounds included in the present invention include the following compounds:
2-(4-{[(1R,2R)-2-hydroxycyclohexyl]amino}-5,6,7,8-tetrahydrophthalazin-1-yl)-5-(trifluoro methyl)phenol monohydrochloride;
2-(4-{[(1R,2R)-2-hydroxycyclohexyl]amino}-7,8-dihydro-5H-pyrano[3,4-d]pyridazin-1-yl)-5-(trifluoromethyl)phenol monohydrochloride;
2-(4-{[(1R,2R)-2-hydroxycyclohexyl]amino}-7,8-dihydro-5H-pyrano[3,4-d]pyridazin-1-yl)-5-(trifluoromethoxy)phenol monohydrochloride; and
5-(difluoromethoxy)-2-(4-{[(3R)-1-methylpiperidin-3-yl]amino}-7,8-dihydro-5H-pyrano[3,4 -d]pyridazin-1-yl)phenol dihydrochloride.

Examples of specific compounds included in the present invention include, in an aspect, the following compounds:
2-(4-{[(1R,2R)-2-hydroxycyclohexyl]amino}-7,8-dihydro-5H-pyrano[3,4-d]pyridazin-1-yl)-5-(trifluoromethyl)phenol monohydrochloride;
2-(4-{[(1R,2R)-2-hydroxycyclohexyl]amino}-7,8-dihydro-5H-pyrano[3,4-d]pyridazin-1-yl)-5-(trifluoromethoxy)phenol monohydrochloride; and
5-(difluoromethoxy)-2-(4-{[(3R)-1-methylpiperidin-3-yl]amino}-7,8-dihydro-5H-pyrano[3,4 -d]pyridazin-1-yl)phenol dihydrochloride.

The compound of formula (I) may have a tautomer or a geometrical isomer depending on the type of the substituent. Although the compound of formula (I) or a salt thereof may be described herein only in one form of an isomer, the present invention also includes isomers other than that as well as separated isomers or mixtures thereof.

In addition, the compound of formula (I) or a salt thereof may have an asymmetric center or an axis chirality in some cases, based on which enantiomers (optical isomers) may exist. The compound of formula (I) or a salt thereof includes any of an isolated individual enantiomer such as (R) form and (S) form, and a mixture thereof (including a racemic mixture or a non-racemic mixture). In an aspect, the enantiomer is "stereochemically pure". The term "stereochemically pure" refers to a degree of purity such that those skilled in the art can recognize an enantiomer as being substantially stereochemically pure. In another aspect, the enantiomer is, for example, a compound having stereochemical purity of 90% ee (enantiomeric excess) or more, 95% ee or more, 98% ee or more, or 99% ee or more.

The present invention further includes a pharmaceutically acceptable prodrug of the compound represented by formula (I). The pharmaceutically acceptable prodrug is a compound having a group that can be converted into, for example, an amino group, a hydroxy group, and a carboxyl group, by solvolysis or under physiological conditions.
Examples of the group to form a prodrug include groups described in Prog. Med., 5, 2157-2161 (1985) or in "Iyakuhin no Kaihatsu (Pharmaceutical Research and Development)" (Hirokawa Publishing Company, 1990), vol. 7, Bunshi Sekkei (Molecular Design), 163-198.

In addition, the salt of the compound of formula (I) is a pharmaceutically acceptable salt of the compound of formula (I), which may form an acid addition salt or a salt with a base in some cases, depending on the type of the substituent. Specific examples thereof include an acid addition salt with an inorganic acid such as hydrochloric acid, hydrobromic acid, hydroiodic acid, sulfuric acid, nitric acid, and phosphoric acid, or with an organic acid such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid, and glutamic acid; a salt with an inorganic base such as sodium, potassium, magnesium, calcium, and aluminum, or with an organic base such as methylamine, ethylamine, ethanolamine, lysine, and ornithine; a salt with various amino acids and amino acid derivatives such as acetylleucine; and an ammonium salt.

The present invention further includes substances having various hydrates, solvates, and crystalline polymorphism of the compound of formula (I) and a salt thereof.

The present invention also includes all pharmaceutically acceptable compounds of formula (I) or salts thereof, which are labeled with one or more radioactive or non-radioactive isotopes. Examples of suitable isotopes used for isotopic labeling of the compound of the present invention include isotopes such as hydrogen (e.g., ²H and ³H), carbon (e.g., ¹¹C, ¹³C, and ¹⁴C), nitrogen (e.g., ¹³N and ¹⁵N), oxygen (e.g., ¹⁵O, ¹⁷O, and ¹⁸O), fluorine (e.g., ¹⁸F), chlorine (e.g., ³⁶Cl), iodine (e.g., ¹²³I and ¹²⁵I), phosphorus (e.g., ³²P), and sulfur (e.g., ³⁵S). The isotope-labeled compound of the invention of the application can be used for research and the like on tissue distribution of drugs and/or substrates. For example, radioactive isotopes such as tritium (³H) and carbon 14 (¹⁴C) can be used for this purpose from the viewpoint of the ease of labeling and the convenience of detection. Substitution by a heavier isotope, for example, substitution of hydrogen by deuterium (²H), is therapeutically advantageous through the improvement of metabolic stability in some cases (for example, increase in in vivo half-life, decrease in required dose, or decrease in interaction between drugs). Substitution by positron emitting isotopes (e.g., ¹¹C, ¹⁸F, ¹⁵O, and ¹³N) can be used in a positron emission tomography (PET) test for testing occupancy of a substrate receptor. The isotope-labeled compound of the present invention can be generally produced by a conventional method known to a person skilled in the art or by the same preparation method as in Examples or Production Examples by using suitable reagents which are labeled with isotopes in place of unlabeled reagents. For example,
2-[4-(4-aminobutoxy)phthalazin-1-yl]-5-(trifluoromethyl)phenol (compound of Production Example 64) is subjected to a known [¹¹C] acetylation reaction (International Journal of Radiation Applications and Instrumentation. Part A. Applied Radiation and Isotopes, Vol. 39, No. 4, pp. 287-290, 1988) to prepare a compound of Example 81 in which a carbon atom of the carbonyl group is replaced with ¹¹C.

### (Production Method)

The compound of formula (I) and a salt thereof can be produced by applying various known synthetic methods using the basic structure thereof or the characteristics based on the types of substituents. Depending on the type of a functional group, it is effective for production technique in some cases to replace the functional group with an appropriate protective groups (group that can be easily converted to the functional groups) in advance at a stage from a raw material to an intermediate. Examples of such a protective group include a protective group described in "Greene's Protective Groups in Organic Synthesis (4th edition, 2006)" by Wuts (P. G. M. Wuts) and Greene (T. W. Greene), and the protective group may be appropriately selected and used according to these reaction conditions. In such a method, a desired compound can be obtained by introducing the protective group to carry out the reaction, then removing the protective group, if necessary.

Similarly to the protective group, a prodrug of the compound of formula (I) can also be produced by introducing a special group at a stage from a raw material to an intermediate or further carrying out the reaction using the obtained compound of formula (I). The reaction can be carried out by applying a method known to a person skilled in the art, such as general esterification, amidation, and dehydration.

Hereinafter, a representative method for producing the compound of formula (I) will be described. Each preparation method can also be applied with reference to reference documents attached to the description. Note that the production method of the present invention is not limited to the examples described below.

In the present specification, the following abbreviations are sometimes used.

### CN: cyano, COMU:

({[(1-cyano-2-oxoethylidene)amino]oxy}-4-morpholinomethylene)dimethylammonium hexafluorophosphate, DMF: N,N-dimethylformamide, DMSO: dimethyl sulfoxide, DIPEA: N,N-diisopropylethylamine, EDC·HCl: 1-ethyl-3-(3-dimethylaminopropyl)carbodiimide hydrochloride, HATU: 1-[bis(dimethylamino)methylene]-1H-1,2,3-triazolo[4,5-b]pyridinium 3-oxide hexafluorophosphate, HOBt: 1-hydroxybenzotriazole, NMP: 1-methylpyrrolidin-2-one, Me: methyl, Oxone (R): potassium peroxymonosulfate, Pd-118: [1,1'-bis(di-tert-butylphosphino)ferrocene]dichloropalladium(II), PdCl₂(PPh₃)₂: bis(triphenylphosphine)palladium(II) dichloride, PdCl₂(dppf): [1,1'-bis(diphenylphosphino)ferrocene]palladium(II) dichloride, PdCl₂(dppf) ·CH₂Cl₂:[1,1'-bis(diphenylphosphino)ferrocene]palladium(II) dichloride dichloromethane adduct, Pd₂(dba)₃: (1E,4E)-1,5-diphenylpenta-1,4-dien-3-one/palladium (3:2), Pd(PPh₃)₄: tetrakis(triphenylphosphine)palladium, RuPhos Pd G3: (2-dicyclohexylphosphino-2',6'-diisopropoxy-1,1'-biphenyl) [2-(2'-amino-1,1'-biphenyl)]palladium (II) methanesulfonate, SPhos Pd G3: (2-dicyclohexylphosphino-2',6'-dimethoxybiphenyl) [2-(2'-amino-1,1'-biphenyl)]palladium(II) methanesulfonate, TBS: tert-butyl(dimethyl)silyl, THF: tetrahydrofuran, Tf: trifluoromethanesulfonyl, TFA: trifluoroacetic acid, XPhos: 2-dicyclohexylphosphino-2',4',6'-triisopropylbiphenyl, and ODS: octadecylsilyl.

### (Preparation Method 1)

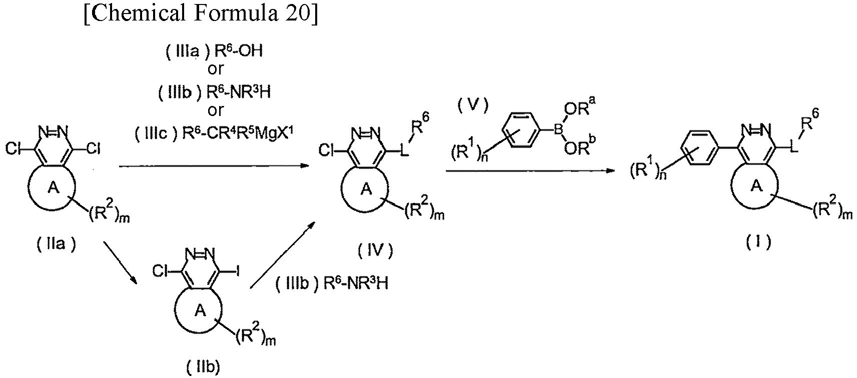

(X¹ is halogen; and R^{a} and R^{b} are each H, or R^{a} and R^{b} together with a boronic acid residue to which R^{a} and R^{b} are attached to form 4,4,5,5-tetramethyl-1,3,2-dioxaborolane. The same shall apply hereinafter.)

### (Step 1)

This step is a step of reacting a compound of formula (IIa) with compounds of formulae (IIIa) to (IIIc) to prepare a compound of formula (IV). The following steps 1 to 5 can be selected according to the type of L as a linker.

### (Step 1-1)

This step is a step of reacting the compound of formula (IIa) with the compound of formula (IIIa) to prepare a compound of formula (IV) in which L is -O-. In this reaction, the compound of formula (IIa) and the compound of formula (IIIa) are used in equivalent amounts, or either thereof in an excess amount. A mixture thereof is stirred in the presence of a base, in a reaction-inert solvent, under cooling to heating, preferably at 0 to 190°C, usually for 0.1 hours to 5 days. Examples of the solvent used here include, but are not particularly limited to, ethers such as diethyl ether, THF, 1,4-dioxane, and 1,2-dimethoxyethane, halogenated hydrocarbons such as dichloromethane and chloroform, pyridine, acetonitrile, NMP, DMF, DMSO, and a mixture thereof. Examples of the base include, but are not particularly limited to, sodium hydride, potassium-tert-butoxide, sodium hydroxide, and potassium hydroxide. In some cases, it is advantageous to carry out the reaction in the presence of a phase transfer catalyst such as benzyltrimethylammonium chloride, tetrabutylammonium bromide, and 18-crown-6-ether in order to allow the reaction to proceed smoothly. This reaction may also be carried out under microwave irradiation.

### (Step 1-2)

This step is a step of reacting the compound of formula (IIa) with the compound of formula (IIIb) to prepare a compound of formula (IV) in which L is -NR³-. In this reaction, the compound of formula (IIa) and the compound of formula (IIIb) are used in equivalent amounts, or either thereof in an excess amount. A mixture thereof is stirred in a reaction-inert solvent or without a solvent, under cooling to heating at reflux, preferably at 0 to 220°C, usually for 0.1 hours to 5 days. Examples of the solvent used here include, but are not particularly limited to, ethers such as diethyl ether, THF, 1,4-dioxane, and 1,2-dimethoxyethane, alcohols such as methanol, ethanol 1-propanol, 2-propanol, 1-butanol, and cyclopentanol, water, pyridine, acetonitrile, NMP, DMF, DMSO, and a mixture thereof. In some cases, it is advantageous to carry out the reaction in the presence of an organic base such as triethylamine and N,N-diisopropylethylamine or an inorganic base such as sodium hydride, potassium carbonate, sodium carbonate, and cesium carbonate in order to allow the reaction to proceed smoothly. This reaction may also be carried out under microwave irradiation.

### (Step 1-3)

This step is a step of reacting the compound of formula (IIa) with the compound of formula (IIIb) to prepare a compound of formula (IV) in which L is -NR³-. In this reaction, the compound of formula (IIa) and the compound of formula (IIIb) are used in equivalent amounts, or either thereof in an excess amount. A mixture thereof is stirred in the presence of a catalyst, a ligand, and a base, in a reaction-inert solvent, under cooling to heating at reflux, preferably at room temperature to 150°C, usually for 0.1 hours to 5 days. Examples of the solvent used here include, but are not particularly limited to, palladium acetate and Pd₂(dba)₃. Examples of the ligand include, but are not particularly limited to, 2,2'-bis(diphenylphosphino) -1,1'-binaphthyl, tri-tert-butylphosphine, and XPhos. Examples of the base include, but are not particularly limited to, tripotassium phosphate, sodium carbonate, potassium carbonate, cesium carbonate, sodium hydroxide, sodium tert-butoxide, and potassium tert-butoxide. Examples of the solvent include, but are not particularly limited to, ethers such as diethyl ether, THF, 1,4-dioxane, and 1,2-dimethoxyethane, aromatic hydrocarbons such as benzene, toluene, and xylene, water, pyridine, acetonitrile, NMP, DMF, DMSO, and a mixture thereof. This reaction may also be carried out under microwave irradiation.

### [Reference]

Journal of the American Chemical Society, 127, pp 4685-4696 (2005)

### (Step 1-4)

This step is a step of reacting the compound of formula (IIa) with the compound of formula (IIIb) to prepare a compound of formula (IV) in which L is -NR³-. This step consists of a first phase of converting the compound of formula (IIa) into the compound of formula (IIb) and a second phase of converting the compound of formula (IIb) into the compound of formula (IV).

### (First Phase)

In this reaction, the compound of formula (IIa) is stirred in the presence of a metal iodide, in hydriodic acid, under cooling to heating at reflux, preferably at room temperature to 100°C, usually for 0.1 hours to 5 days. Examples of the metal iodide used here include, but are not particularly limited to, sodium iodide and potassium iodide.

### (Second Phase)

In this reaction, the compound of formula (IIb) and the compound of formula (IIIb) are used in equivalent amounts, or either thereof in an excess amount. A mixture thereof is stirred in the presence of a catalyst and a base, in a reaction-inert solvent, under cooling to heating at reflux, preferably at room temperature to 190°C, usually for 0.1 hours to 5 days. Examples of the catalyst used here include, but are not particularly limited to, copper(I) iodide, copper(I) oxide, copper(I) bromide, copper(I) chloride, and copper(I) 2-thiophenecarboxylate. In some cases, it is advantageous to carry out the reaction in the presence of a ligand such as proline and ethylenediamine in order to allow the reaction to proceed smoothly. Examples of the base include, but are not particularly limited to, tripotassium phosphate, sodium carbonate, potassium carbonate, cesium carbonate, sodium hydroxide, sodium tert-butoxide, potassium-tert-butoxide, triethylamine, and N,N-diisopropylethylamine. Examples of the solvent include, but are not particularly limited to, ethers such as diethyl ether, THF, 1,4-dioxane, and 1,2-dimethoxyethane, alcohols such ethanol, 2-propanol, 1-butanol, and ethylene glycol, water, pyridine, acetonitrile, NMP, DMF, DMSO, and a mixture thereof. This reaction may also be carried out under microwave irradiation.

### (Step 1-5)

This step is a step of reacting the compound of formula (IIa) with the compound of formula (IIIc) to prepare a compound of formula (IV) in which L is -CR⁴R⁵-. In this reaction, the compound of formula (IIa) and the compound of formula (IIIc) are used in equivalent amounts, or either thereof in an excess amount. A mixture thereof is stirred in the presence of a catalyst, in a reaction-inert solvent, under cooling to heating at reflux, preferably at room temperature to 150°C, usually for 0.1 hours to 5 days. Examples of the catalyst used here include, but are not particularly limited to, copper(I) iodide, and iron (III) acetylacetonate. Examples of the solvent include, but are not particularly limited to, an ether such as diethyl ether, THF, 1,4-dioxane, and 1,2-dimethoxyethane, water, pyridine, acetonitrile, NMP, DMF, DMSO, and a mixture thereof. This reaction may also be carried out under microwave irradiation.

### (Step 2)

This step is a step of reacting the compound of formula (IV) with the compound of formula (V) to prepare a compound of formula (I). In this reaction, the compound of formula (IV) and the compound of formula (V) are used in equivalent amounts, or either thereof in an excess amount. A mixture thereof is stirred in the presence of a catalyst or a base, in a reaction-inert solvent, under cooling to heating at reflux, preferably 0 to 150°C, usually for 0.1 hours to 5 days. Examples of the catalyst used here include, but are not particularly limited to, Pd(PPh₃)₄, PdCl₂(PPh₃)₂, PdCl₂(dppf), PdCl₂(dppf)·CH₂Cl₂, Pd₂(dba)₃, RuPhos Pd G3, SPhos Pd G3, and Pd-118. Examples of the base include, but are not particularly limited to, tripotassium phosphate, sodium carbonate, potassium carbonate, cesium carbonate, potassium acetate, sodium hydroxide, and sodium tert-butoxide. Examples of the solvent include, but are not particularly limited to, an ether such as diethyl ether, THF, 1,4-dioxane, and 1,2-dimethoxyethane, an aromatic hydrocarbon such as benzene, toluene, and xylene, water, pyridine, acetonitrile, NMP, DMF, DMSO, and a mixture thereof. This reaction may also be carried out under microwave irradiation.

### (Preparation Method 2)

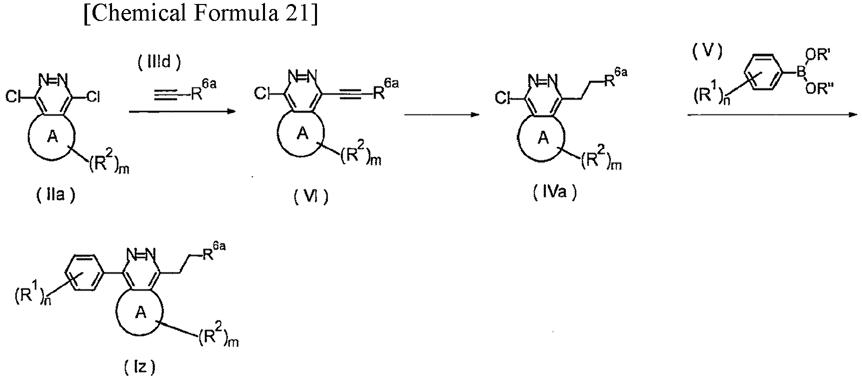

(R^{6a} is C₁₋₅ alkyl substituted with one to four identical or different R⁷,
-C₁₋₅ alkylene-(C₃₋₈ cycloalkyl optionally substituted with one to four identical or different R⁸), or -C₁₋₅ alkylene-(4- to 7-membered saturated heterocyclyl optionally substituted with one to four identical or different R⁹). The same shall apply hereinafter.)

This preparation method is a method for preparing compound (Iz) of formula (I) in which L is -CR⁴R⁵-; R⁴ and R⁵ are each H; R⁶ is -CH₂-(C₁₋₅ alkyl substituted with one to four identical or different R⁷), -CH₂-C₁₋₅ alkylene-(C₃₋₈ cycloalkyl optionally substituted with one to four identical or different R⁸), or -CH₂-C₁₋₅ alkylene-(4- to 7-membered saturated heterocyclyl optionally substituted with one to four identical or different R⁹).

### (Step 1)

This step is a step of reacting the compound of formula (IIa) with the compound of formula (IIId) to prepare a compound of formula (VI). In this reaction, the compound of formula (IIa) and the compound of formula (IIId) are used in equivalent amounts, or either thereof in an excess amount. A mixture thereof is stirred in the presence of a catalyst and a base, preferably in the presence of a copper salt, in a reaction-inert solvent, under cooling to heating at reflux, preferably at room temperature to 150°C, usually for 0.1 hours to 5 days. Examples of the catalyst used here include, but are not particularly limited to, Pd(PPh₃)₄, PdCl₂(PPh₃)₂, PdCl₂(dppf), PdCl₂(dppf)·CH₂Cl₂, and Pd₂(dba)₃. Examples of the base include, but are not particularly limited to, tripotassium phosphate, sodium carbonate, potassium carbonate, cesium carbonate, sodium hydroxide, sodium tert-butoxide, triethylamine, N,N-diisopropylethylamine, diisopropylamine, and pyrrolidine. Examples of the copper salt include copper(I) iodide. Examples of the solvent include, but are not particularly limited to, ethers such as diethyl ether, THF, 1,4-dioxane, and 1,2-dimethoxyethane, water, pyridine, acetonitrile, NMP, DMF, DMSO, and a mixture thereof. This reaction may also be carried out under microwave irradiation. Note that this reaction may be carried out after the compound of formula (IIa) has been converted into the compound of formula (IIb) by the reaction of the first phase in step 1-4 described in Preparation Method 1.

### [Reference]

Chemical Reviews, 107, pp 874-922 (2007)

### (Step 2)

This step is a step of preparing a compound of formula (IVa) by a reduction reaction of the compound of formula (VI). In this reaction, the compound of formula (VI) is stirred in the presence of a catalyst under a hydrogen atmosphere, in a reaction-inert solvent, under cooling to heating at reflux, preferably at room temperature to 50°C, usually for 0.1 hours to 5 days. Examples of the catalyst include, but are not particularly limited to, palladium on carbon, palladium hydroxide, and platinum oxide. Examples of the solvent include, but are not particularly limited to, alcohols such as methanol, ethanol, and 2-propanol, water, ethyl acetate, acetic acid, and a mixture thereof.

### (Step 3)

This step is a step of reacting the compound of formula (I Va) with the compound of formula (V) to prepare a compound of formula (Iz) in the same manner as in step 2 of Preparation Method 1.

### (Preparation Method 3)

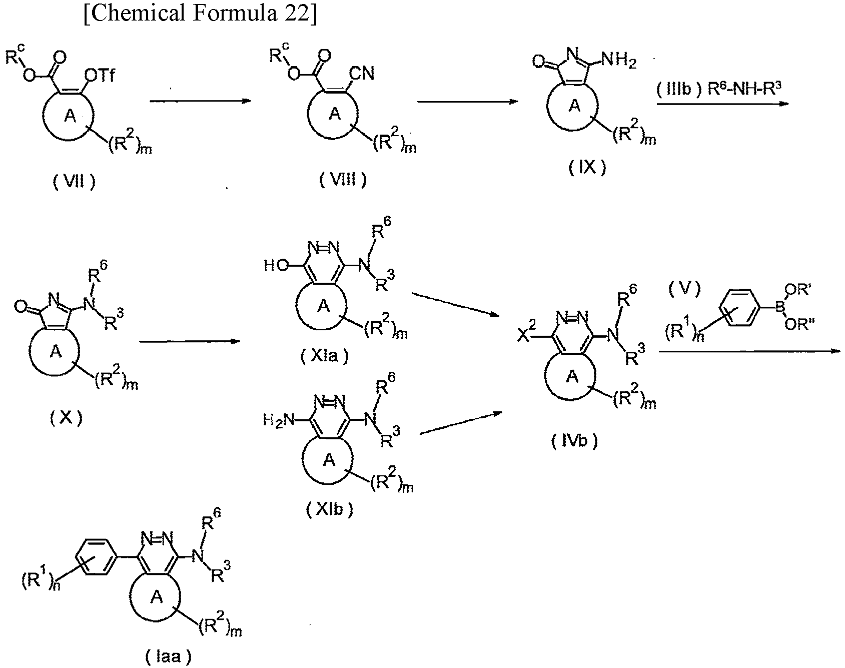

(R^{c} is C₁₋₆ alkyl; and X² is halogen or -OTf. The same shall apply hereinafter.)

This preparation method is a method for preparing compound (Iaa) of formula (I) in which L is -NR³-.

### (Step 1)

This step is a step of preparing a compound of formula (VIII) by a cyanation reaction of the compound of formula (VII). In this reaction, the compound of formula (VII) and a metal cyanide are used in equivalent amounts, or either thereof in an excess amount. A mixture thereof is stirred in the presence of a catalyst, in a reaction-inert solvent, under cooling to heating at reflux, preferably at room temperature to 150°C, usually for 0.1 hours to 5 days. Examples of the metal cyanide used here include, but are not particularly limited to, zinc cyanide, copper cyanide, sodium cyanide, and potassium cyanide. Examples of the catalyst include, but are not particularly limited to, Pd(PPh₃)₄, PdCl₂(PPh₃)₂, PdCl₂(dppf), PdCl₂(dppf)·CH₂Cl₂, and Pd₂(dba)₃. Examples of the solvent include, but are not particularly limited to, an ether such as diethyl ether, THF, 1,4-dioxane, and 1,2-dimethoxyethane, an aromatic hydrocarbon such as benzene, toluene, and xylene, water, pyridine, acetonitrile, NMP, DMF, DMSO, and a mixture thereof. This reaction may also be carried out under microwave irradiation.

### (Step 2)

This step is a step of reacting the compound of formula (VIII) with ammonia to prepare a compound o formula (IX). In this reaction, the compound of formula (VIII) and ammonia are stirred in a reaction-inert solvent, under cooling to heating at reflux, preferably at 0°C to 100°C, usually for 0.1 hours to 5 days. Examples of the solvent used here include, but are not particularly limited to, alcohols such as methanol, ethanol, and 2-propanol. This reaction may also be carried out under microwave irradiation.

### (Step 3)

This step is a step of reacting the compound of formula (IX) with the compound of formula (IIIb) to prepare a compound of formula (X). In this reaction, the compound of formula (IX) and the compound of formula (IIIb) are used in equivalent amounts, or either thereof in an excess amount. A mixture thereof is stirred in a reaction-inert solvent, under cooling to heating at reflux, preferably at room temperature to 150°C, usually for 0.1 hours to 5 days. Examples of the solvent used here include, but are not particularly limited to, an ether such as diethyl ether, THF, 1,4-dioxane, and 1,2-dimethoxyethane, an alcohol such as methanol, ethanol 1-propanol, 2-propanol, 1-butanol, and cyclopentanol, water, pyridine, acetonitrile, NMP, DMF, DMSO, and a mixture thereof. In some cases, it is advantageous to carry out the reaction in the presence of an organic base such as triethylamine and N,N-diisopropylethylamine or an inorganic base such as potassium carbonate, sodium carbonate, and cesium carbonate in order to allow the reaction to proceed smoothly. This reaction may also be carried out under microwave irradiation.

### (Step 4)

This step is a step of reacting the compound of formula (X) with hydrazine monohydrate to prepare a compound of formula (XIa) and/or a compound of formula (XIb). In this reaction, the compound of formula (X) and hydrazine monohydrate are used in equivalent amounts, or either thereof in an excess amount. A mixture thereof is stirred in a reaction-inert solvent, under cooling to heating at reflux, preferably at 0°C to 100°C, usually for 0.1 hours to 5 days. Examples of the solvent used here include, but are not particularly limited to, alcohols such as methanol, ethanol, and 2-propanol, and acetonitrile. This reaction may also be carried out under microwave irradiation.

### (Step 5)

This step is a step of preparing a compound of formula (IVb) from the compound of formula (XIa) or formula (XIb). The following steps 1 to 3 can be selected according to the type of the starting material and the X².

### (Step 5-1)

This step is a step of preparing a compound of formula (IVb) in which X² is -Otf from the compound of formula (XIa). In this reaction, the compound of formula (XIa) and a trifluoromethanesulfonylating agent are stirred in the presence of a base, in a reaction-inert solvent, under cooling to heating at reflux, preferably at 0°C to at room temperature, usually for 0.1 hours to 5 days. Examples of the trifluoromethanesulfonylating agent used here include, but are not particularly limited to, trifluoromethanesulfonic anhydride and 1,1,1-trifluoro-N-phenyl-N-(trifluoromethanesulfonyl)methanesulfonamide. Examples of the base include, but are not particularly limited to, sodium hydride, potassium-tert-butoxide, sodium hydroxide, and potassium hydroxide. Examples of the solvent include, but are not particularly limited to, ethers such as diethyl ether, THF, 1,4-dioxane, and 1,2-dimethoxyethane, halogenated hydrocarbons such as dichloromethane and chloroform, water, pyridine, acetonitrile, NMP, DMF, DMSO, and a mixture thereof.

### (Step 5-2)

This step is a step of preparing a compound of formula (IVb) in which X² is halogen from the compound of formula (XIa). In this reaction, the compound of formula (XIa) and the halogenating agent are stirred in a reaction-inert solvent, under cooling to heating at reflux, preferably at 0°C to 150°C, usually for 0.1 hours to 5 days. Examples of the halogenating agent used here include, but are not particularly limited to, phosphorus oxychloride, phosphorus oxybromide, and thionyl chloride. Examples of the solvent include, but are not particularly limited to, ethers such as diethyl ether, THF, 1,4-dioxane, and 1,2-dimethoxyethane, halogenated hydrocarbons such as dichloromethane and chloroform, NMP, DMF, and a mixture thereof.

### (Step 5-3)

This step is a step of preparing a compound of formula (IVb) in which X² is halogen from the compound of formula (XIb). In this reaction, the compound of formula (XIb) and a diazotizing agent are stirred in the presence of a copper halide, in a reaction-inert solvent, under cooling to heating at reflux, preferably at 0°C to room temperature, usually for 0.1 hours to 5 days. Examples of the diazotizing agent used here include, but are not particularly limited to, sodium nitrite and tert-butyl nitrite. Examples of the copper halide include, but are not particularly limited to, copper(I) chloride, copper(I) bromide, and copper(I) iodide. Examples of the solvent include, but are not particularly limited to, halogenated hydrocarbons such as dichloromethane and chloroform, acetonitrile, NMP, DMF, and a mixture thereof. In some cases, it is advantageous to carry out the reaction in the presence of a hydrogen halide such as hydrochloric acid, hydrobromic acid, and hydroiodic acid in order to allow the reaction to proceed smoothly.

### (Step 6)

This step is a step of reacting the compound of formula (IVb) with the compound of formula (V) to prepare a compound of formula (Iaa). In this reaction, the compound of formula (IVb) and the compound of formula (V) are used in equivalent amounts, or either thereof in an excess amount. A mixture thereof is stirred in the presence of a catalyst or a base, in a reaction-inert solvent, under cooling to heating at reflux, preferably at room temperature to 150°C, usually for 0.1 hours to 5 days. Examples of the catalyst used here include, but are not particularly limited to, Pd(PPh₃)₄, PdCl₂(PPh₃)₂, PdCl₂(dppf), PdCl₂(dppf)·CH₂Cl₂, Pd₂(dba)₃, and RuPhos Pd G3. Examples of the base include, but are not particularly limited to, tripotassium phosphate, sodium carbonate, potassium carbonate, cesium carbonate, sodium hydroxide, and sodium tert-butoxide. Examples of the solvent include, but are not particularly limited to, an ether such as diethyl ether, THF, 1,4-dioxane, and 1,2-dimethoxyethane, an aromatic hydrocarbon such as benzene, toluene, and xylene, water, pyridine, acetonitrile, NMP, DMF, DMSO, and a mixture thereof. This reaction may also be carried out under microwave irradiation.

### (Raw Material Synthesis 1)

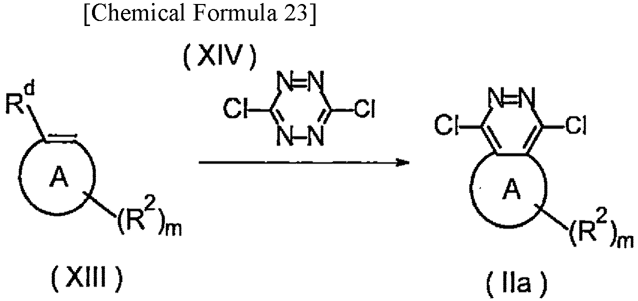

(R^{d} is a tri(C₁₋₆ alkyl)silyloxy group or a pyrrolidin-1-yl group. The same shall apply hereinafter.)

This preparation method is a method of reacting the compound of formula (XIII) with the compound of formula (XIV) to prepare a compound of formula (IIa) in which ring A is C₅₋₈ cycloalkenyl or 5- to 11-membered partially unsaturated heterocyclyl. In the reaction in this preparation method, the compound of formula (XIII) and the compound of formula (XIV) are used in equivalent amounts, or either thereof in an excess amount. A mixture thereof is stirred in a reaction-inert solvent, under cooling to heating at reflux, preferably at room temperature to 150°C, usually for 0.1 hours to 5 days. Examples of the solvent used in this reaction include, but are not particularly limited to, aromatic hydrocarbons such as benzene, toluene, and xylene, halogenated carbons such as dichloromethane and chloroform, and a mixture thereof.

### (Raw Material Synthesis 2)

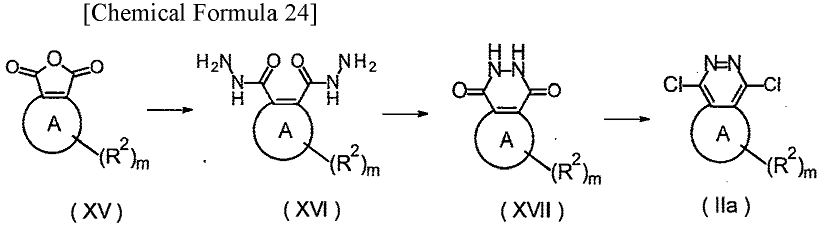

This preparation method is a method for preparing a compound of formula (IIa) from a compound of formula (XV).

### (Step 1)

This step is a step of reacting the compound of formula (XV) with hydrazine monohydrate to prepare a compound of formula (XVI). In this reaction, a mixture of hydrazine monohydrate in an excess amount and the compound of the formula (XV) is stirred without a solvent or in a reaction-inert solvent, under cooling to heating at reflux, preferably at room temperature to 200°C. usually for 0.1 hour to 5 days. Examples of the solvent used in this reaction include, but are not particularly limited to, alcohols such as ethanol, 2-propanol, 1-butanol, and ethylene glycol, acetonitrile, DMF, NMP, DMSO and a mixture thereof.

### (Step 2)

This step is a step of preparing a compound of formula (XVII) from a compound of formula (XVI). In this reaction, the compound of formula (XVI) is stirred in the presence of an acid, in a reaction-inert solvent, under cooling to heating at reflux, preferably at room temperature to 150°C, usually for 0.1 hours to 5 days. Examples of the acid used here include, but are not particularly limited to, hydrochloric acid, sulfuric acid, and acetic acid. Examples of the solvent used in this reaction include, but are not particularly limited to, alcohols such as ethanol, 2-propanol, 1-butanol, and ethylene glycol, water, acetonitrile, DMF, NMP, DMSO, and a mixture thereof.

### (Step 3)

This step is a step of reacting the compound of formula (XVII) with a chlorinating agent to prepare a compound of formula (XIIa). In this reaction, a mixture of the chlorinating agent in an excess amount and the compound of formula (XVII) is stirred under cooling to heating at reflux, preferably at room temperature to 200°C, usually for 0.1 hours to 5 days. Examples of the chlorinating agent used here include, but are not particularly limited to, phosphorus oxychloride and thionyl chloride. In some cases, it is advantageous to add a small amount of DMF in order to allow the reaction to proceed smoothly.

### (Raw Material Synthesis 3)

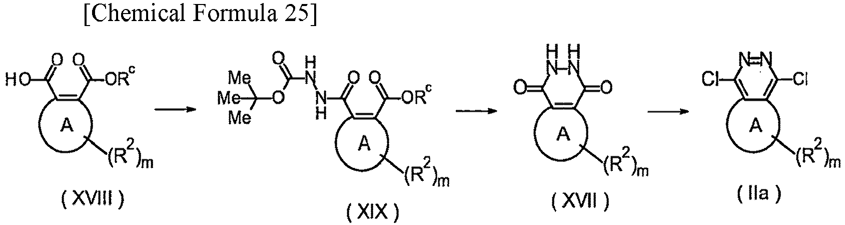

This preparation method is a method for preparing a compound of formula (IIa) from a compound of formula (XVIII).

### (Step 1)

This step is a step of reacting the compound of formula (XVIII) with tert-butyl carbazate to prepare a compound of formula (XIX). In this reaction, the compound of formula (XVIII) and tert-butyl carbazate are used in equivalent amounts, or either thereof in an excess amount. A mixture thereof is stirred in the presence of a condensing agent and a base, in a reaction-inert solvent, under cooling to heating at reflux, preferably at room temperature to 150°C, usually for 0.1 hours to 5 days. Examples of the condensing agent used here include, but are not particularly limited to, HATU, EDC·HCl, and COMU. Examples of the base used here include, but are not particularly limited to, triethylamine and DIPEA. Examples of the solvent used in this reaction include, but are not particularly limited to, ethers such as THF, 1,4-dioxane, and 1,2-dimethoxyethane, halogenated hydrocarbons such as dichloromethane and chloroform, acetonitrile, NMP, DMF, DMSO, and a mixture thereof. In some cases, it is advantageous to carry out the reaction in the presence of HOBt in order to allow the reaction to proceed smoothly.

### (Step 2)

This step is a step of preparing a compound of formula (XVII) from the compound of formula (XIX). In this reaction, the compound of formula (XIX) is stirred in the presence of an acid, in a reaction-inert solvent, under cooling to heating at reflux, preferably at room temperature to 150°C, usually for 0.1 hours to 5 days. Examples of the acid used here include, but are not particularly limited to, hydrochloric acid and sulfuric acid. Examples of the solvent used in this reaction include, but are not particularly limited to, water, ethyl acetate, and 1,4-dioxane.

### (Step 3)

This step is a step of reacting the compound formula (XVII) with a chlorinating agent to prepare a compound of formula (XIIa) in the same manner as in step 3 of Raw Material Synthesis 2.

### (Other Preparation Method)

The compound of formula (I) obtained by the above preparation method is used as a raw material and further subjected to a chemical modification reaction generally used by those skilled in the art, such as alkylation, benzylation, esterification, amidation, acylation, sulfonylation, or oxidation reaction, protection reaction, and deprotection reaction to prepare another compound of formula (I).

The compound of formula (I) is isolated and purified as a free compound, a salt thereof, a hydrate, a solvate or a substance having crystalline polymorphism. The salt of the compound of formula (I) can also be produced by subjecting the compound to a salt formation reaction of a general method. Isolation and purification are carried out by applying usual chemical operations such as extraction, fractional crystallization, and various fractionation chromatography. Various isomers can be produced by selecting an appropriate raw material compound or can be separated by using a difference in physiochemical properties between isomers. For example, an optical isomer can be obtained by a general optical resolution method of racemic form (for example, fractional crystallization leading to a diastereomeric salt with an optically active base or an acid, chromatography using a chiral column or the like), or can also be produced from an appropriate optically active raw material compound.

Pharmacological activity of the compound of formula (I) can be confirmed by the following tests. Note that the pharmacological activity can also be confirmed by well-known improved tests.

### Test Example 1: THP-1 IL-1β Production Inhibition Test

PMA (phorbol myristate acetate, SIGMA, P1585) was added to THP-1 cells in an amount of 50 ng/mL to culture the cells for 2 days at 37°C. The culture medium was replaced with a serum-free RPMI-1640 medium, and a compound with a known concentration was added thereto to culture the cells at 37°C for 15 minutes. LPS (Lipopolysaccharide, SIMGA, L2880) and ATP (Adenosine triphosphate, SIGMA, A2383) were added so that final concentrations become 50 ng/mL and 5 mM, respectively, followed by culturing at 37°C for 2 hours. The supernatants were collected to measure the concentrations of IL-10 by ELISA (DuoSet ELISA human IL-1β, R&D Systems, DY201). The IL-1β concentrations were plotted against log concentrations of test compounds, and an IC₅₀ value was calculated by sigmoid Emax model nonlinear regression analysis.

The results are shown in Table 1. Example compounds were confirmed to inhibit NLRP3 inflammasome activation and IL-10 production.

**[Table 1-1]**

| Table 1 | | | | | | | |
|---|---|---|---|---|---|---|---|
| Ex | IC₅₀ (nM) | Ex | IC₅₀ (nM) | Ex | IC₅₀ (nM) | Ex | IC₅₀ (nM) |
| 1 | 10 | 25 | 4.6 | 52 (2) | 27 | 75 | 54 |
| 2 (1) | 3.4 | 26 | 15 | 53 | 15 | 76 | 23 |
| 2 (2) | 14 | 27 | 4.1 | 54 | 21 | 77 | 33 |
| 3 (1) | 2.2 | 28 | 14 | 55 | 36 | 78 | 54 |
| 3 (2) | 7.0 | 29 | 18 | 56(1) | 7.0 | 79 | 4800 |
| 4 (1) | 2.5 | 30 | 22 | 56(2) | 36 | 80 | 5200 |
| 4 (2) | 15 | 31 | 44 | 57(1) | 19 | 81 | 33 |
| 5 | 4.7 | 32 | 76 | 57(2) | 94 | 82 | 28 |
| 6 | 9000 | 33 | 100 | 58 | 88 | 83 | 4. 8 |
| 7 | 8500 | 34 | 3.9 | 59 | 40 | 84 | 18 |
| 8 | 210 | 35 | 16 | 60 | 1600 | 85 | 58 |
| 9 | 1300 | 36 | 32 | 61 | 96 | 86 | 11 |
| 10 | 24 | 37 | 18 | 62(1) | 6.1 | 87 | 6.5 |
| 11 | 16 | 38 | 54 | 62(2) | 27 | 88 | 1. 8 |
| 12 | 58 | 39 | 19 | 63 | 9.4 | 89 | 31 |
| 13 | 15 | 40 | 50 | 64 | 6.3 | 90 | 12 |
| 14 | 7.6 | 41 | 40 | 65 | 48 | 91 | 560 |
| 15 | 2.8 | 42 | 37 | 66(1) | 990 | 92 | 47 |
| 16(1) | 1.9 | 43 | 8.7 | 66(2) | 180 | 93 | 200 |
| 16(2) | 20 | 44 | 6.8 | 67 | 13 | 94 | 5. 1 |
| 17 | 20 | 45 | 14 | 68 | 11 | 95 | 61 |
| 18 | 34 | 46 | 11 | 69 | 6.5 | 96 | 45 |
| 19 | 81 | 47 | 16 | 70 | 2.1 | 97 | 3.4 |
| 20 | 22 | 48 | 36 | 71 | 150 | 98 | 95 |
| 21 | 81 | 49 | 22 | 72 (1) | 27 | 99 | 16 |
| 22 | 69 | 50 | 8.2 | 72(2) | 14 | | |
| 23 | 17 | 51 | 13 | 73 | 7.6 | | |
| 24 | 74 | 52(1) | 4.1 | 74 | 1400 | | |

**[Table 1-2]**

| Table 1 (continued) | | | | | | | |
|---|---|---|---|---|---|---|---|
| Ex | IC₅₀ (nM) | Ex | IC₅₀ (nM) | Ex | IC₅₀ (nM) | Ex | IC₅₀ (nM) |
| 100 | 4.9 | 108 (2) | 3.0 | 118 | 4.8 | 128 | 3.3 |
| 101(1) | 2. 0 | 109 | 1.5 | 119 | 6.0 | 129 | 86 |
| 101(2) | 8.6 | 110 | 1.4 | 120 | 1.0 | 130 | 22 |
| 102 | 5.1 | 111 | 2. 9 | 121 | 8. 5 | 131(1) | 3.0 |
| 103 | 4.4 | 112 | 1.3 | 122 | 8.4 | 131(2) | 79 |
| 104 | 3.5 | 113 | 1.2 | 123 | 47 | 132 | 95 |
| 105 | 8.9 | 114 | 15 | 124 | 0.62 | 133 | 1.3 |
| 106 | 4.8 | 115 | 11 | 125 | 1.5 | 134 | 4.6 |
| 107 | 3.6 | 116 | 4.4 | 126 | 7.1 | 135 | 2.9 |
| 108 (1) | 27 | 117 | 130 | 127 | 5.6 | 136 | 2.2 |

### Test Example 2 TNF-α Production Inhibition Test

PMA (phorbol myristate acetate, SIGMA, P1585) was added to THP-1 cells in an amount of 50 ng/mL to culture the cells for 2 days at 37°C. The culture medium was replaced with a serum-free RPMI-1640 medium, and a compound with a known concentration was added thereto to culture the cells at 37°C for 15 minutes. LPS (Lipopolysaccharide SIMGA, L2880) was added so that each final concentration becomes 50 ng/mL, followed by culturing at 37°C for 2 hours. The supernatants were collected to measure the concentration of TNF--α by ELISA (DuoSet ELISA human TNF-α, R&D Systems, DY210). The TNF-α concentrations were plotted against log concentrations of test compounds, and an IC₅₀ value was calculated by sigmoid Emax model nonlinear regression analysis.

It was confirmed that Examples 1, 2(1), 3(1), 4(1), 5, 70, 73, 83, 88, 99, 111, 124, and 125, which are compounds of formula (I), each had a IC₅₀ value of 10 µM or more in this test.

### Test Example 3: Rat Central Nervous System IL-1β Production Test

Under isoflurane anesthesia, 10 to 14 weeks-old male Wistar rats were administered with 12.5 µg/5 µL of LPS (SIGMA, L2880) in the cisterna magna, followed by an oral administration of the test compound 2 hours later. After an additional hour, 50 µg/5 µL of BzATP(2'(3')-O-(4-benzoylbenzoyl)adenosine 5'-triphosphate triethylammonium salt, SIGMA, B6396) was administered into the cisterna magna, and cerebrospinal fluid was collected after 30 minutes. The cerebrospinal fluid was subjected to Western blotting using an anti-IL-10 antibody (Millipore, AB1832P) to quantify IL-1β p17, and the percent inhibition relative to the solvent-treated group was calculated.

The percent inhibition of IL-10 p17 for Examples 1, 2(1), 3(1) and 124, which are compounds of formula (I), relative to the solvent-treated group is shown in the following table. In the table, Dose refers to the dosage of test compounds; Ex1, Ex2(1), Ex3(1), and Ex124 refer to Example 1, Example 2(1), Example 3(1); and Example 124; and "NT" denotes the unmeasured case. These compounds were confirmed to exhibit inhibitory effects on IL-1β production.

**[Table 2]**

| Dose | Ex1 | Ex2 (1) | Ex3 (1) | Ex124 |
|---|---|---|---|---|
| 0.1 mg/kg | NT | NT | NT | 55% |
| 0.3 mg/kg | -12% | 81% | 75% | 49% |
| 1 mg/kg | 74% | 75% | 82% | 96% |
| 3 mg/kg | 84% | 88% | 72% | NT |

### Test Example 4: Evaluation of Motor Function in Mouse α-Synuclein Fibril-Induced Neuroinflammation Model

To male C57BL/6J mice, 8 µg of mouse α-synuclein fibrotic protein (StressMarq Biosciences Inc., SPR-324) was administered at the left striatum. After 14 to 15 weeks, a test compound suspended in a 0.5% methylcellulose solution was orally administered once per day. To a negative control group, a 0.5% methylcellulose solution was administered. After 4 weeks from the start of administration, motor function was evaluated by a hanging wire test. The mice were allowed to catch a wire horizontally stretched. When the mice fell, they were allowed to catch the wire again. This operation was continued for 3 minutes to record the number of falls. In this test, it was confirmed that a specific compound of formula (I) or a salt thereof has improvement effects on movement disorder.

### Test Example 5: Mouse ex vivo Il-1β Production Test

Male C57BL/6J mice are orally administered with a compound suspended in 0.5% methylcellulose at an arbitrary dose at single or multiple times, and blood is collected at arbitrary times. LPS is added to the blood so that a final concentration becomes 50 ng/mL, followed by culturing at 37°C for 3 hours. Thereafter, ATP is added thereto so that a final concentration becomes 5 mM, followed by culturing at 37°C for 30 minutes. After removal of blood cells by centrifugation, the concentration of IL-10 is measured by ELISA (DuoSet ELISA mouse IL-1β, R&D Systems, DY401).

### Test Example 6: In Vitro Phototoxicity Test

The evaluation of in vitro phototoxicity tests was based on ICH S10: Guidelines for Evaluation of Photosafety of Drugs (Notification No. 0521-1) using a method described in OECD Guidelines for the Testing of Chemicals 432: In vitro 3T3 NRU Phototoxicity Test, 2019). In this test, it was confirmed that the compounds of Examples 1, 2(1), 3(1), and 124 had no phototoxic effect.

### Test Example 7: Safety Pharmacology Test

For the safety pharmacology test, evaluation of a human Ether-a-go-go Related Gene (hereinafter referred to as hERG) channel inhibitory effect was carried out. The hERG channel inhibitory effect was evaluated using a modified method described in Combinatorial Chemistry & High Throughput Screening, 12, 1, 78-95 (2009). In this test, it showed that the compounds of Examples 1, 2(1), 3(1), and 124 had a IC₅₀ value of 10 µM or more.

From the above results, the compound of formula (I) or a salt thereof is expected to be used as an highly safe pharmaceutical for prevention and/or treatment of inflammatory diseases and/or neurodegenerative diseases.

The compound of formula (I) or a salt thereof shown in Table 1 exhibited to inhibit the production of IL-10 by Test Example 1, and the compounds of Example 1, Example 2(1), Example 3(1), and Example 124 also exhibited to have inhibitory effects on the production of IL-1β in the central nervous system by Test Example 3. Furthermore, a specific compound of formula (I) or a salt thereof exhibited to improve motor function in the mouse model of α-synucleinopathy induced by α-synuclein fibrils described in Test example 4. From the above results, it is highly expected that the compound of formula (I) or a salt thereof can be used for the prevention and/or treatment of inflammatory diseases and/or neurodegenerative diseases, in particular α-synucleinopathies, including Parkinson's disease, multiple system atrophy, and Lewy body dementia.

In addition, the results of Test Examples 6 and 7 showed that the compounds of Example 1, Example 2(1), Example 3(1), and Example 124 had no phototoxic effects and weak hERG channel inhibitory effects. From the above results, the compounds of Example 1, Example 2(1), Example 3(1), and Example 124 are highly expected to be highly safe pharmaceuticals.

A pharmaceutical composition containing one or more compounds of formula (I) or a salt thereof as active ingredients can be prepared by using an excipient commonly used in the art, i.e., a pharmaceutical excipient and a pharmaceutical carrier through a method commonly used.

The administration may be either oral administration with tablets, pills, capsules, granules, powders, solutions and the like or parenteral administration with injections such as intraarticular, intravenous, and intramuscular, suppositories, eye drops, ophthalmic ointments, transdermal liquids, ointments, transdermal patches, transmucosal solutions, transmucosal patches, and inhalants.

As a solid composition for the oral administration, tablets, powders, granules and the like are used. In such a solid composition, one or more active ingredients are mixed with at least one inert excipient. The composition may contain an inert additive, such as a lubricant, a disintegrant, a stabilizer and a solubilizing agent according to a conventional method. The tablets, powders, granules, or pills may be coated with a sugar coating or a film of a gastric or enteric substance, if necessary.

A liquid composition for the oral administration includes a pharmaceutically acceptable emulsion, solution, suspension, syrup, and elixir agent and contains a generally used inert diluent such as purified water or ethanol. The liquid composition may contain, in addition to the inert diluent, a solubilizing agent, a wetting agent, an adjuvant such as a suspending agent, a sweetening agent, a flavoring agent, an aromatic, or a preservative.

An injection for the parenteral administration contains a sterile aqueous or nonaqueous solution, a suspension, or an emulsion. Examples of the aqueous solvent include distilled water for injection or physiological saline. Examples of the nonaqueous solvent include alcohols such as ethanol. Such a composition may further includes an isotonizing agent, a preservative, a wetting agent, an emulsifier, a dispersant, a stabilizer, or a solubilizing agent. These compositions are sterilized, for example, by filtration through a bacteria-retaining filter, incorporation of a microbicide or irradiation. In addition, they are used to produce a sterile solid composition, and can be used by being dissolved or suspended in sterile water or a sterile injectable solvent before use.

A topical medication includes an ointment, a plaster, a cream, a jelly, a poultice, a spray, a lotion, an eye drop, and an ophthalmic ointment. The topical medication contains a generally used ointment base, lotion base, aqueous or non-aqueous liquid, suspension, and emulsion.

A transmucosal agent such as an inhalant or a transnasal agent is used in a form of solid, liquid, or semisolid, and can be produced according to a conventionally known method. In addition to a known excipient, for example, a pH adjuster, a preservative, a surfactant, a lubricant, a stabilizer, or a thickener may be appropriately added. The administration can be performed using a device for appropriate inhalation or insufflation. For example, using a known device such as a metering and administering inhalation device or an atomizer, the compound may be administered alone or as a powder of a mixture formulated, or as a solution or a suspension in combination with a pharmaceutically acceptable carrier. A dry powder inhaler or the like may be used for a single administration or multiple administrations, and dry powder or powder-containing capsules can be used. Alternatively, a suitable ejection agent, for example, a form of pressurized aerosol spray using suitable gases such as chlorofluoroalkane or carbon dioxide may be employed.

In the case of the common oral administration, a suitable dosage per day is appropriately about 0.001 to 100 mg/kg per body weight, preferably 0.1 to 30 mg/kg, and still more preferably 0.1 to 10 mg/kg, which is given once or two to four times a day. In the case of intravenous administration, a suitable dosage per day is appropriately about 0.0001 to 10 mg/kg per body weight, which is given once to several times a day. In addition, the transmucosal agent is administered at about 0.001 to 100 mg/kg per body weight once to several times a day. The dosage is appropriately decided according to individual cases in consideration of, for example, symptoms, age, and sex.

Depending on an administration route, a dosage form, an administration site, the types of excipients and additives, the pharmaceutical composition of the present invention contains one or more compounds of formula (I) or salts thereof having active ingredients in a range of 0.01 to 100% by weight, and in an aspect, in a range of 0.01 to 50% by weight.

The compound of formula (I) can be used in combination with various therapeutic or prophylactic agents for diseases in which the compound of formula (I) is considered to exhibit efficacy. The combination use may be simultaneous administration or separate administration in succession, or administered at a desired interval. A simultaneous administration preparation may be a formulated agent or may be separately formulated.

### EXAMPLES

Hereinafter, the production methods for the compound of formula (I) will be described in more detail with reference to Examples. Note that the present invention is not limited to compounds described in Examples below. In addition, the production method of starting compounds will be described in Production Examples. The production method for the compound of formula (I) is not limited to only the production methods of specific Examples described below, and the compound of formula (I) may also be produced by using a combination of the production methods or a method obvious to a person skilled in the art.

Note that onset temperatures of DSC curves obtained by measurement under the following conditions are described in Tables below. The DSC measurement was carried out using DSC Q2000 (manufactured by TA Instruments), under the conditions of a measurement temperature range from 25 to 300 °C, a temperature elevating rate of 10°C/min, a nitrogen flow rate of 50 mL/min, with an aluminum sample pan without a lid thereon. Since the onset temperatures of DSC curves may vary more or less depending on the measurement conditions, the values should not be strictly interpreted.

The powder X-ray diffraction was measured using Empyrean (manufactured by PANalytical) under the conditions of a vacuum tube of Cu, a tube current of 40 mA, a tube voltage of 45 kV, a step width of 0.013°, a wavelength of 1.5418 Å, and a measurement diffraction angle (2θ) of 2.5° to 40° or using SmartLab (manufactured by Rigaku) under the conditions of a vacuum tube of Cu, a tube current of 200 mA, a tube voltage of 45 kV, a step width of 0.005°, a wavelength of 1.5418 Å, and a measurement diffraction angle (2θ) of 2.5° to 40°. Note that for powder X-ray diffraction patterns, crystal lattice spacings or overall patterns are important in identity certification of crystals due to the nature of the data, and the error range of the diffraction angle (2θ(°)) in the powder X-ray diffraction is usually ± 0.2°. However, the diffraction angle and diffraction intensity may vary more or less depending on the orientation of the crystal growth, particle size, and measurement conditions, and thus, the values should not be strictly interpreted.

The following abbreviations are sometimes used in Examples, Production Examples, and Tables as described later:
PEx: Production Example No., Ex: Example No., PSyn: Production Example No. where a compound prepared by the same method, Syn: Example No. where a compound prepared by the same method, Str: chemical structural formula, DAT: physicochemical data, EI+: m/z value in mass spectrometry (electron ionization method EI, [M]+ unless otherwise specified), ESI+: m/z value in mass spectrometry (ionization method ESI, [M+H]+ unless otherwise specified), FAB: m/z value in mass spectrometry (ionization method FAB, [M+H]+ unless otherwise specified), CI: m/z value in mass spectrometry (ionization method CI, [M+H]+ unless otherwise specified), ¹H-NMR (400 MHz, CDCl₃): δ value (ppm) of signals in ¹H-NMR in CDCl₃, ¹H-NMR (500 MHz, CDCl₃): δ value (ppm) of a signal in ¹H-NMR in CDCl₃, ¹H-NMR (400 MHz, DMSO-d₆): δ value (ppm) of a signal in ¹H-NMR in DMSO-d₆, ¹H-NMR (500 MHz, DMSO-d₆): δ value (ppm) of a signal in ¹H-NMR in DMSO-d₆, J: coupling constant, s: singlet, d: doublet, t: triplet, q: quartet, dd: double doublet, br: broad (e.g., br s), m: multiplet, DSC¹: temperature (onset temperature) at which an endothermic peak (decomposition point) is observed in DSC measurement, DSC²: temperature (onset temperature) at which an endothermic peak (amorphous decomposition point) is observed in DSC measurement, and 2θ: diffraction angle of peaks in powder X-ray diffraction.

In the Tables described later, hyphen (-) in the columns of PSyn and Syn indicates that the production method is described in sentences in Production Examples or Examples.

For the purpose of convenience, the concentration mol/L is expressed as M. For example, a 1 M aqueous sodium hydroxide solution means a 1 mol/L aqueous sodium hydroxide solution.

### Production Example 1

A mixture of 1,4-dichloro-5,6,7,8-tetrahydrophthalazine (10.92 g), (1R,2R)-2-aminocyclohexan-1-ol monohydrochloride (16.3 g), DIPEA (28 mL), and cyclopentanol (55 mL) was stirred at 160°C for 2 days. After the reaction mixture was allowed to cool to room temperature, water/saturated aqueous sodium chloride solution (1/1) and chloroform/methanol (9/1) were added thereto, and the organic layer was separated.
The aqueous layer was extracted with chloroform/methanol (9/1), combined with the organic layer, and dried over anhydrous magnesium sulfate. Then, the solution was concentrated under reduced pressure. The resulting residue was purified by silica gel column chromatography (chloroform/ethyl acetate) to afford (1R,2R)-2-[(4-chloro-5,6,7,8-tetrahydrophthalazin-1-yl)amino]cyclohexan-1-ol (8.24 g) as a solid.

### Production Example 2

To a mixture of 1,4-dichloro-7,8-dihydro-5H-pyrano[3,4-d]pyridazine (1 g) and cyclopentanol (5 mL) were added (1R,2R)-2-aminocyclohexan-1-ol monohydrochloride (1.48 g) and DIPEA (1.7 mL) at room temperature, and the mixture was stirred at 160°C for 44 hours. After the reaction mixture was cooled to room temperature, chloroform and water were added thereto, and the mixture was stirred at room temperature for 10 minutes. The organic layer was separated and dried over anhydrous sodium sulfate, and the solution was then concentrated. The resulting residue was purified by silica gel column chromatography (hexane/ethyl acetate) to afford a mixture of (1R,2R)-2-[(1-chloro-7,8-dihydro-5H-pyrano[3,4-d]pyridazin-4-yl)amino]cyclohexan-1-ol and (1R,2R)-2-[(4-chloro-7,8-dihydro-5H-pyrano[3,4-d]pyridazin-1-yl)amino]cyclohexan-1-ol (386 mg) as a solid.

### Production Example 3

A mixture of 6-benzyl-1,4-dichloro-5,6,7,8-tetrahydropyrido[3,4-d]pyridazine (1.145 g), (1R,2R)-2-aminocyclohexan-1-ol monohydrochloride (890 mg), DIPEA (2.7 mL), and NMP (15 mL) was stirred at 160°C for 24 hours. To the reaction mixture were added (1R,2R)-2-aminocyclohexan-1-ol monohydrochloride (420 mg) and DIPEA (1.3 mL), followed by stirring at 160°C for 20 hours. After the reaction mixture was allowed to cool to room temperature, ethyl acetate and water were added thereto, and the organic layer was separated. The aqueous layer was extracted with ethyl acetate, combined with the organic layer, washed with water/saturated aqueous sodium chloride solution (1/1) and saturated aqueous sodium chloride solution, and then dried over anhydrous magnesium sulfate. The solution was concentrated under reduced pressure, and the resulting residue was purified by silica gel column chromatography (hexane/ethyl acetate) to afford (1R,2R)-2-[(6-benzyl-1-chloro-5,6,7,8-tetrahydropyrido[3,4-d]pyridazin-4-yl)amino]cyclohe xan-1-ol (36.1 mg) as a solid from the low polar fraction and (1R,2R)-2-[(6-benzyl-4-chloro-5,6,7,8-tetrahydropyrido[3,4-d]pyridazin-1-yl)amino]cyclohe xan-1-ol (504.8 mg) as an oil from the high polar fraction.

### Production Example 4

To a mixture of 1,4-dichloro-7,8-dihydro-5H-pyrano[3,4-d]pyridazine (1 g) and cyclopentanol (5 mL) were added (1S,2R)-2-aminocyclohexan-1-ol monohydrochloride (1.48 g) and DIPEA (1.68 mL) at room temperature, and the mixture was stirred at 160°C for 12 hours. After the reaction mixture was cooled to room temperature, DIPEA (1 mL) was added thereto, and the mixture was stirred at room temperature for 12 hours. After the reaction mixture was cooled to room temperature, chloroform/methanol (9/1) and water/saturated aqueous sodium chloride solution (1/1) were added thereto, and the mixture was stirred at room temperature for 10 minutes. The resulting organic layer was separated and dried over anhydrous sodium sulfate. After the solution was concentrated, the resulting residue was purified by silica gel column chromatography (hexane/ethyl acetate) to afford a mixture (774 mg) of (1S,2R)-2-[(1-chloro-7,8-dihydro-5H-pyrano[3,4-d]pyridazin-4-yl)amino]cyclohexan-1-ol and (1S,2R)-2-[(4-chloro-7,8-dihydro-SH-pyrano[3,4-d]pyridazin-1-yl)amino]cyclohexan-1-ol as a solid.

### Production Example 5

To a mixture of 1,4-dichloro-5,6,8,9-tetrahydrooxepino[4,5-d]pyridazine (502.7 mg) and cyclopentanol (3 mL) were added (1R,2R)-2-aminocyclohexan-1-ol monohydrochloride (394 mg) and DIPEA (0.393 mL) at room temperature, and the mixture was stirred at 160°C under an argon atmosphere for 2 days. After the reaction mixture was cooled to room temperature, chloroform/methanol (9/1) and saturated aqueous sodium chloride solution/water (1/1) were added thereto, and the mixture was stirred at room temperature for 10 minutes. The organic layer was separated and concentrated, and the resulting residue was then purified by silica gel column chromatography (chloroform/ethyl acetate) to afford (1R,2R)-2-[(4-chloro-5,6,8,9-tetrahydrooxepino[4,5-d]pyridazin-1-yl)amino]cyclohexan-1-ol (57.4 mg) as an oil.

### Production Example 42

To a mixture of 2,3-dihydropyrido[3,4-d]pyridazin-1,4-dione (1 g) and methanol (20 mL) were added hydrochloric acid (12 M, 0.6 mL) and platinum oxide (100 mg), and the mixture was stirred at room temperature under a hydrogen atmosphere overnight. Methanol (20 mL) and DMF (20 mL) were added to the reaction mixture, followed by stirring under an argon atmosphere for 1 hour. The reaction mixture was filtered through Celite (R), and the filtrate was concentrated under reduced pressure. Toluene was added to the resulting residue, and the mixture was concentrated under reduced pressure. The resulting solid was triturated with ethyl acetate. The solid was collected by filtration and washed with ethyl acetate to afford 2,3,5,6,7,8-hexahydropyrido[3,4-d]pyridazin-1,4-dione monohydrochloride (1.19 g) as a solid.

### Production Example 43

To a mixture of 2,3,5,6,7,8-hexahydropyrido[3,4-d]pyridazin-1,4-dione monohydrochloride(800 mg) and dichloromethane (16 mL) were added DIPEA (0.250 mL), benzaldehyde (0.500 mL), and sodium triacetoxyborohydride (1.1 g), and the mixture was stirred at room temperature overnight. Water was added to the reaction mixture, and the mixture was extracted with chloroform/methanol (5/1). After the organic layer was dried over anhydrous magnesium sulfate, the solution was concentrated under reduced pressure. The resulting residue was purified by silica gel column chromatography (chloroform/methanol) to afford 6-benzyl-2,3,5,6,7,8-hexahydropyrido[3,4-d]pyridazin-1,4-dione (515 mg) as a solid.

### Production Example 44

A mixture of 6-benzyl-2,3,5,6,7,8-hexahydropyrido[3,4-d]pyridazin-1,4-dione (100 mg) and phosphoryl chloride(2 mL) was stirred at 120°C for 2 hours. After the reaction mixture was concentrated under reduced pressure, toluene was added thereto, and the mixture was concentrated under reduced pressure. Chloroform (3 mL) was added to the resulting residue, and DIPEA (1 mL) was added thereto under ice cooling. Water was added to the reaction mixture, followed by extraction with chloroform. The organic layer was dried over anhydrous magnesium sulfate, and the solution was concentrated under reduced pressure. The resulting residue was purified by silica gel column chromatography (hexane/ethyl acetate) to afford 6-benzyl-1,4-dichloro-5,6,7,8-tetrahydropyrido[3,4-d]pyridazine(87 mg) as an oil.

### Production Example 45

To a mixture of 1,4-dichlorophthalazine (720 mg), 4-hydroxy-1λ⁶-thiane-1,1-dione (540 mg), and THF (14 mL) under a nitrogen atmosphere were added sodium hydride (55%, liquid paraffin dispersion, 190 mg) under ice cooling, and the mixture was stirred at the same temperature for 30 minutes, then warmed to room temperature, and stirred for 3 days. Water and ethyl acetate were added to the reaction mixture, and the solid was collected by filtration and washed with water and ethyl acetate to afford 4-[(4-chlorophthalazin-1-yl)oxy]-1 λ⁶-thiane-1,1-dione (681 mg) as a solid.

### Production Example 47

To a mixture of (1R,2R)-2-[(4-chloro-5,6,7,8-tetrahydrophthalazin-1-yl)amino]cyclohexan-1-ol(100 mg) and 1,4-dioxane (3.2 mL) were added 2-[4-(difluoromethoxy)-2-(methoxymethoxy)phenyl]-4,4,5,5-tetramethyl-1,3,2-dioxaborolan e(350 mg), RuPhos Pd G3(45 mg), potassium carbonate (147 mg), and water (0.64 mL), and the mixture was stirred under microwave irradiation at 100°C for 1.5 hours. After the reaction mixture was cooled to room temperature, chloroform and water were added thereto, and the organic layer was separated. The solution was concentrated, and the resulting residue was then purified by silica gel column chromatography (hexane/ethyl acetate) to afford (1R,2R)-2-({4-[4-(difluoromethoxy)-2-(methoxymethoxy)phenyl]-5,6,7,8-tetrahydrophthalaz in-1-yl}amino)cyclohexan-1-ol (106 mg) as a solid.

### Production Example 48

Under an argon atmosphere, a mixture of (1R,2R)-2-[(6-benzyl-1-chloro-5,6,7,8-tetrahydropyrido[3,4-d]pyridazin-4-yl)amino]cyclohe xan-1-ol (34 mg), [2-hydroxy-4-(trifluoromethyl)phenyl]boronic acid (38 mg), RuPhos Pd G3 (3 mg), potassium carbonate (38 mg), 1,4-dioxane (1 mL), and water (0.100 mL) was stirred under microwave irradiation at 130°C for 60 minutes. Water and chloroform were added to the reaction mixture, and the organic layer was separated. The aqueous layer was extracted with chloroform, then combined with the organic layer, and dried over anhydrous magnesium sulfate. The solution was concentrated under reduced pressure. The resulting residue was purified by silica gel column chromatography (hexane/ethyl acetate) to afford 2-(6-benzyl-4-{[(1R,2R)-2-hydroxycyclohexyl]amino}-5,6,7,8-tetrahydropyrido[3,4-d]pyrida zin-1-yl)-5-(trifluoromethyl)phenol (15.6 mg) as a solid.

### Production Example 63

To a mixture of tert-butyl [4-({4-[2-hydroxy-4-(trifluoromethyl)phenyl]phthalazin-1-yl}oxy)butyl]carbamate (1.38g), dichloromethane (14 mL), and triethylamine (1.21 mL) were added acetic anhydride (0.330 mL) and 4-dimethylaminopyridine (71 mg), and the mixture was stirred at room temperature overnight. Water was added to the reaction mixture, and the mixture was extracted with ethyl acetate. After the organic layer was dried over anhydrous magnesium sulfate, the solution was concentrated under reduced pressure. The resulting residue was purified by silica gel column chromatography (hexane/ethyl acetate), and the resulting solid was then washed with hexane/ethyl acetate (4/1) to afford 2-(4-{4-[(tert-butoxy carbonyl)amino]butoxy}phthal azin-1-yl)-5-(trifluoromethyl)phenyl acetate (1.24 g) as a solid.

### Production Example 64

To a mixture of 2-(4-{4-[(tert-butoxycarbonyl)amino]butoxy}phthalazin-1-yl)-5-(trifluoromethyl)phenyl acetate (1.24 g) and dichloromethane (13 mL) was added trifluoroacetic acid (1.83 mL), and the mixture was stirred at room temperature overnight. To the reaction mixture were added a 1 M aqueous sodium hydroxide solution and saturated aqueous sodium hydrogen carbonate solution, followed by extraction with chloroform. After the organic layer was dried over anhydrous magnesium sulfate, the solution was concentrated under reduced pressure. The resulting residue was purified by silica gel column chromatography (chloroform/methanol/ammonia water). The resulting residue was mixed with chloroform and methanol and dried over anhydrous magnesium sulfate. The solution was concentrated under reduced pressure, and the resulting solid was washed with hexane/ethyl acetate (4/1) to afford 2-[4-(4-aminobutoxy)phthalazin-1-yl]-5-(trifluoromethyl)phenol (607 mg) as a solid.

### Production Example 65

A mixture of 2-[4-(4-aminobutoxy)phthalazin-1-yl]-5-(trifluoromethyl)phenol (403 mg), acetyl chloride (0.075 mL), DIPEA (0.495 mL), and dichloromethane (4 mL) was stirred at room temperature for 1 hour. Acetyl chloride (0.075 mL) was added to the reaction mixture, followed by stirring at room temperature for 1 hour. DIPEA (0.495 mL) and acetyl chloride (0.075 mL) were added to the reaction mixture, followed by stirring at room temperature for 30 minutes. Acetyl chloride (0.075 mL) was added to the reaction mixture, followed by stirring at room temperature for 4 days. Water was added to the reaction mixture, and the mixture was extracted with ethyl acetate. The organic layer was washed with saturated aqueous sodium chloride solution and then dried over anhydrous magnesium sulfate, and the solution was concentrated under reduced pressure. The resulting residue was purified by silica gel column chromatography (chloroform/methanol) to afford 2-[4-(4-acetamidobutoxy)phthalazin-1-yl]-5-(trifluoromethyl)phenyl acetate (401 mg) as a solid.

### Production Example 67

To a mixture of 1-chloro-4-iodo-6,7-dihydro-5H-cyclopenta[d]pyridazine (500 mg), (2R)-but-3-yn-2-ol (0.150 mL), and THF (8 mL) under an argon atmosphere were added PdCl₂(PPh₃)₂ (126 mg), copper(I) iodide (17 mg), and diisopropylamine (0.755 mL) under ice cooling, and the mixture was then warmed to room temperature and stirred for 5 hours. Water and saturated aqueous ammonium chloride solution were added to the reaction mixture, followed extraction with chloroform. The aqueous layer was extracted with chloroform, combined with the organic layer, and dried over anhydrous magnesium sulfate. Then, the solution was concentrated under reduced pressure. The resulting residue was purified by silica gel column chromatography (chloroform/methanol) to afford (2R)-4-(4-chloro-6,7-dihydro-5H-cyclopenta[d]pyridazin-1-yl)but-3-yn-2-ol (267 mg) as an oil.

### Production Example 69

To a mixture of (2R)-4-(4-chloro-6,7-dihydro-5H-cyclopenta[d]pyridazin-1-yl)but-3-yn-2-ol (267 mg) and ethanol (6 mL) was added platinum oxide (67 mg), and the mixture was stirred at room temperature under a hydrogen atmosphere for 1.5 hours. Celite (R) was added to the reaction mixture, followed by stirring, the mixture was then filtered through Celite (R), and the filtrate was concentrated under reduced pressure to afford (2R)-4-(4-chloro-6,7-dihydro-5H-cyclopenta[d]pyridazin-1-yl)butan-2-ol (260 mg) as an oil.

### Production Example 70

Under an argon atmosphere, a mixture of 1,4-dichloro-6,7-dihydro-5H-cyclopenta[d]pyridazine (1.50 g), sodium iodide (3.1 g), and 57% hydriodic acid (9 mL) was stirred under microwave irradiation at 100°C for 30 minutes. The reaction mixture was cooled to room temperature, water was added thereto, and the mixture was extracted with ethyl acetate. The organic layer was washed with saturated aqueous sodium hydrogen carbonate solution, saturated aqueous sodium thiosulfate solution, and saturated aqueous sodium chloride solution and then dried over anhydrous magnesium sulfate. The solution was concentrated under reduced pressure, and the resulting residue was purified by silica gel column chromatography (hexane/ethyl acetate) to afford 1-chloro-4-iodo-6,7-dihydro-5H-cyclopenta[d]pyridazine (1.9 g) as a solid.

### Production Example 72

Under an argon atmosphere, a mixture of 1,4-dichloro-5,6,7,8-tetrahydrophthalazin (100 mg), (3-aminobicyclo[1.1.1]pentan-1-yl)methanol monohydrochloride (95 mg), palladium acetate (22 mg), (+/-)-2,2'-bis(diphenylphosphino)-1,1'-binaphthyl (90 mg), cesium carbonate (385 mg), and 1,4-dioxane (2 mL) was stirred at 100°C for 24 hours. The reaction mixture was filtered through Celite (R), and the filtrate was concentrated under reduced pressure. The resulting residue was purified by silica gel column chromatography (chloroform/methanol) to afford {3-[(4-chloro-5,6,7,8-tetrahydrophthalazin-1-yl)amino]bicyclo[1.1.1]pentan-1-yl}methanol (46.2 mg) as a solid.

### Production Example 73

To a mixture of tert-butyl [(3,6-dihydro-2H-pyran-4-yl)oxy]di(methyl)silane (1.42 g) and toluene (20 mL) was added 3,6-dichloro-1,2,4,5-tetrazine (1 g) under ice cooling, and the mixture was stirred at room temperature for 0.5 hours and at 120°C for 16 hours.
The reaction mixture was cooled to room temperature and then concentrated under reduced pressure, and the resulting residue was purified by silica gel column chromatography (hexane/ethyl acetate) to afford 1,4-dichloro-7,8-dihydro-5H-pyrano[3,4-d]pyridazine (1.08 g) as a solid.

### Production Example 74

To a mixture (11.67 g) of tert-butyl di(methyl)[(2,3,6,7-tetrahydrooxepin-4-yl)oxy]silane and tert-butyldi(methyl)[(2,5,6,7-tetrahydrooxepin-4-yl)oxy]silane under an argon atmosphere were added toluene (121 mL) and 3,6-dichloro-1,2,4,5-tetrazine (6.61 g), the mixture was stirred at 120°C for 24 hours. The reaction mixture was cooled to room temperature and then filtered using methanol and chloroform. The solution was concentrated under reduced pressure, and the resulting residue was purified by silica gel column chromatography (chloroform/ethyl acetate) to afford 1,4-dichloro-5,6,8,9-tetrahydrooxepino[4,5-d]pyridazine (502.7 mg) as a solid.

### Production Example 76

To a mixture of 2-amino-5-cyclopropylphenol (3.50 g) and ethanol (50 mL) was added hydrochloric acid (12 M, 15.8 mL) at -15°C, and the mixture was stirred at the same temperature for 10 minutes. Amyl nitrite (9.6 mL) was added to the reaction mixture at -15°C, followed by stirring at the same temperature for 30 minutes, and a mixture of potassium iodide (50.6 g) and water (50 mL) was then added dropwise at -15°C. The mixture was warmed to room temperature and stirred for 20 hours. Ethyl acetate was added to the reaction mixture, and the organic layer was separated. The organic layer was washed with saturated aqueous sodium thiosulfate solution and saturated aqueous sodium chloride solution and dried over anhydrous sodium sulfate. The solution was concentrated under reduced pressure, and the resulting residue was purified by silica gel column chromatography (hexane/ethyl acetate) to afford 5-cyclopropyl-2-iodophenol (5 g) as an oil.

### Production Example 77

To a mixture of 1-bromo-4-(difluoromethoxy)-2-(methoxymethoxy)benzene (920 mg) and 1,4-dioxane (9.2 mL) were added 4,4,4',4',5,5,5',5'-octamethyl-2,2'-bi-1,3,2-dioxaborolane (1.24 g), PdCl₂(dppf) (238 mg), and potassium acetate (957 mg), and the mixture was stirred at 100°C for 3 hours. After the reaction mixture was cooled to room temperature, PdCl₂(dppf) (238 mg) was added thereto, and the mixture was stirred at 100°C for 2 hours. After the reaction mixture was cooled to room temperature, 4,4,4',4',5,5,5',5'-octamethyl-2,2'-bi-1,3,2-dioxaborolane (620 mg) was added thereto, and the mixture was stirred at 100°C for 3 hours. After the reaction mixture was cooled to room temperature, hexane was added thereto, and the mixture was stirred at room temperature for 30 minutes. The reaction mixture was filtered through Celite (R), and the filtrate was concentrated under reduced pressure to afford 2-[4-(difluoromethoxy)-2-(methoxymethoxy)phenyl]-4,4,5,5-tetramethyl-1,3,2-dioxaborolan e (1.98 g) as an oil.

### Production Example 81

To a mixture of 4-bromo-3-hydroxybenzaldehyde (2.86 g) and dichloromethane (30 mL) were added DIPEA (2.93 mL) and chloromethyl methyl ether (1.19 mL), and the mixture was stirred at room temperature for 1 hour. Water was added to the reaction mixture, and the organic layer was separated and then concentrated under reduced pressure. The resulting residue was purified by silica gel column chromatography (hexane/ethyl acetate) to afford 4-bromo-3-(methoxymethoxy)benzaldehyde (1.35 g) as an oil.

### Production Example 83

To a mixture of 4-bromo-3-(methoxymethoxy)benzaldehyde (1.35 g) and dichloromethane(20 mL) was added diethylaminosulfur trifluoride (2.2 mL) under ice cooling, and the mixture was stirred at the same temperature for 1 hour, then warmed to room temperature, and stirred for 3 hours. The reaction mixture was added to a mixture of ice water, sodium hydrogen carbonate, and chloroform and stirred. The organic layer was separated and concentrated under reduced pressure. The resulting residue was purified by silica gel column chromatography (hexane/ethyl acetate) to afford 1-bromo-4-(difluoromethyl)-2-(methoxymethoxy)benzene (1.3 g) as an oil.

### Production Example 84

To a mixture of potassium carbonate (1.65 g) and DMF(7.4 mL) were added dropwise a mixture of 4-bromo-3-(methoxymethoxy)phenol (1.86 g), chlorodi(fluoro)sodium acetate (2.43 g), and DMF (11 mL) at 95°C over 1 hour, and the mixture was stirred at the same temperature for 15 minutes. After the reaction mixture was cooled to room temperature, potassium carbonate (1.65 g) and chlorodi(fluoro)sodium acetate (2.43 g) added thereto, and the mixture was stirred at 95°C for 1 hour. After the reaction mixture was cooled to room temperature, ethyl acetate and water were added thereto, and the organic layer was separated. The aqueous layer was extracted with ethyl acetate, and the combined organic layer was washed with water and saturated aqueous sodium chloride solution and then dried over anhydrous sodium sulfate. The solution was concentrated under reduced pressure, and the resulting residue was purified by silica gel column chromatography (hexane/ethyl acetate) to afford 1-bromo-4-(difluoromethoxy)-2-(methoxymethoxy)benzene (920 mg) as an oil.

### Production Example 85

Under an argon atmosphere, a mixture of 1-chloro-4-iodo-5,6,7,8-tetrahydrophthalazine (500 mg), (1R,2R)-2-amino-1-methylcyclohexan-1-ol (270 mg), copper(I) iodide (30 mg), potassium phosphate (725 mg), ethylene glycol (0.100 mL), and 1-propanol (5 mL) was stirred at 90°C for 24 hours. After the reaction mixture was allowed to cool to room temperature, chloroform and saturated aqueous ammonium chloride solution were added thereto, and the organic layer was separated. The aqueous layer was extracted with chloroform, combined with the organic layer, and dried over anhydrous magnesium sulfate. Then, the solution was concentrated under reduced pressure. The resulting residue was purified by silica gel column chromatography (chloroform/methanol) to afford (1R,2R)-2-[(4-chloro-5,6,7,8-tetrahydrophthalazin-1-yl)amino]-1-methylcyclohexan-1-ol (292 mg) as a solid.

### Production Example 86

Under an argon atmosphere, a mixture of ethyl 1-benzyl-5-[(trifluoromethanesulfonyl)oxy]-1,2,3,6-tetrahydropyridine-4-carboxylate (3.37 g), zinc cyanide (1.5 g), Pd(PPh₃)₄ (1.2 g), and DMF (42 mL) was stirred at 100°C for 1.5 hours. After being allowed to cool to room temperature, water and ethyl acetate were added to the reaction mixture, and the organic layer was separated. The aqueous layer was extracted with ethyl acetate, then combined with the organic layer, washed with water/saturated aqueous sodium chloride solution (1/1) and saturated aqueous sodium chloride solution, and dried over anhydrous magnesium sulfate. The solution was concentrated under reduced pressure, and the resulting residue was purified by silica gel column chromatography (hexane/ethyl acetate) to afford ethyl 1-benzyl-5-cyano-1,2,3,6-tetrahydropyridine-4-carboxylate (2.04 g) as an oil.

### Production Example 87

Under an argon atmosphere, a mixture of 5-[(trifluoromethanesulfonyl)oxy]-3,6-dihydro-2H-pyran-4-carboxylateethyl (7.4 g), zinc cyanide (4.3 g), Pd(PPh₃)₄ (2.8 g), and DMF (74 mL) was stirred at 100°C for 2 hours. The reaction mixture was cooled to room temperature, followed by addition of ethyl acetate and water, and was filtered through Celite (R). Two layers were separated, and the aqueous layer was extracted with ethyl acetate. The combined organic layer was washed with water/saturated aqueous sodium chloride solution (1/1) and dried over anhydrous sodium sulfate. The solution was concentrated under reduced pressure, and the resulting residue was purified by silica gel column chromatography (hexane/ethyl acetate) to afford ethyl 5-cyano-3,6-dihydro-2H-pyran-4-carboxylate (4 g) as an oil.

### Production Example 88

A mixture of ethyl 1-benzyl-5-cyano-1,2,3,6-tetrahydropyridine-4-carboxylate (930 mg) and ammonia (7M methanol solution, 8 mL) was stirred under ice cooling for 6 hours. The reaction mixture was allowed to stand in a refrigerator for 18 hours, then warmed to room temperature, and stirred for 10 minutes. The reaction mixture was concentrated under reduced pressure to afford 3-amino-5-benzyl-4,5,6,7-tetrahydro-1H-pyrrolo[3,4-c]pyridin-1-one (828 mg) as a solid.

### Production Example 89

Under ice cooling, ammonia (7M methanol solution, 44 mL) was added to ethyl 5-cyano-3,6-dihydro-2H-pyran-4-carboxylate (4 g), and the mixture was stirred at 0°C for 3 days. The reaction mixture was concentrated under reduced pressure to afford 3-amino-6,7-dihydropyrano[3,4-c]pyrrol-1(4H)-one (3.261 g) as a solid.

### Production Example 90

A mixture of 3-amino-5-benzyl-4,5,6,7-tetrahydro-1H-pyrrolo[3,4-c]pyridin-1-one (825 mg), (1R,2R)-2-aminocyclohexan-1-ol monohydrochloride (530 mg), DIPEA (0.7 mL), and acetonitrile (20 mL) was stirred at 70°C for 19 hours. The reaction mixture was allowed to cool to room temperature and then concentrated under reduced pressure. The resulting residue was purified by silica gel column chromatography (chloroform/methanol) to afford 5-benzyl-3-{[(1R,2R)-2-hydroxycyclohexyl]amino}-4,5,6,7-tetrahydro-1H-pyrrolo[3,4-c]pyr idin-1-one (1.12 g) as a solid.

### Production Example 91

A mixture of 3-amino-6,7-dihydropyrano[3,4-c]pyrrol-1(4H)-one (3.26 g), (1R,2R)-2-aminocyclohexan-1-ol monohydrochloride (3.4 g), DIPEA (5.5 mL), and acetonitrile (70 mL) was stirred at 70°C for 18 hours. The reaction mixture was allowed to cool to room temperature and then concentrated under reduced pressure. The resulting residue was purified by silica gel column chromatography (chloroform/methanol) to afford 3-{[(1R,2R)-2-hydroxycyclohexyl]amino}-6,7-dihydropyrano[3,4-c]pyrrol-1(4H)-one (5.63 g) as a solid.

### Production Example 92

Hydrazine monohydrate (0.16 mL) was added to a mixture of 5-benzyl-3-{[(1R,2R)-2-hydroxycyclohexyl]amino}-4,5,6,7-tetrahydro-1H-pyrrolo[3,4-c]pyr idin-1-one (560 mg) and ethanol (10 mL), and the mixture was stirred at room temperature for 3 days. After the reaction mixture was concentrated under reduced pressure, the resulting residue was purified by silica gel column chromatography (chloroform/methanol) to afford 6-benzyl-4-{[(1R,2R)-2-hydroxycyclohexyl]amino}-5,6,7,8-tetrahydropyrido[3,4-d]pyridazi n-1-ol (201 mg) as a solid.

### Production Example 93

Sodium hydride (55%, liquid paraffin dispersion, 12 mg) was added to a mixture of 6-benzyl-4-{[(1R,2R)-2-hydroxycyclohexyl]amino}-5,6,7,8-tetrahydropyrido[3,4-d]pyridazi n-1-ol (90 mg) and THF (3 mL) under an argon atmosphere under ice cooling, and the mixture was stirred at the same temperature for 20 minutes. To the reaction mixture was added 1,1,1-trifluoro-N-phenyl-N-(trifluoromethanesulfonyl)methanesulfonamide (108 mg), and the mixture was stirred under ice cooling for 1 hour. Water and chloroform were added to the reaction mixture, and the organic layer was separated. The aqueous layer was extracted with chloroform, then combined with the organic layer, and dried over anhydrous magnesium sulfate. The solution was concentrated under reduced pressure. The resulting residue was purified by silica gel column chromatography (hexane/ethyl acetate, then chloroform/ethyl acetate) to afford 6-benzyl-4-{[(1R,2R)-2-hydroxycyclohexyl]amino}-5,6,7,8-tetrahydropyrido[3,4-d]pyridazi n-1-yl trifluoromethanesulfonate (40 mg) as a solid.

### Production Example 94

To a mixture of tert-butyl (3R)-3-hydroxypiperidine-1-carboxylate (2 g) and dichloromethane (5 mL) were added sodium hydroxide (0.596 g) and benzyltriethylammonium chloride (0.922 g), and the mixture was stirred at room temperature for 30 minutes. To the reaction mixture was added 1,4-dichlorophthalazine(2.37 g), followed by stirring at room temperature for 24 hours. Water and dichloromethane were added to the reaction mixture, the organic layer was separated, and the aqueous layer was extracted with dichloromethane. The combined organic layer was washed with water and then dried over anhydrous sodium sulfate, and the solution was concentrated under reduced pressure. The resulting residue was purified by silica gel column chromatography (hexane/ethyl acetate)to afford tert-butyl (3R)-3-[(4-chlorophthalazin-1-yl)oxy]piperidine-1-carboxylate (0.960 g) as a solid.

### Production Example 96

To a mixture of tert-butyl (3R)-3-[(4-chlorophthalazin-1-yl)oxy]piperidine-1-carboxylate (960 mg) and dichloromethane(10 mL) was added a solution of hydrogen chloride in 1,4-dioxane (4 M, 10 mL), and the mixture was stirred at room temperature for 16 hours. After the reaction mixture was concentrated under reduced pressure, the resulting solid was triturated with diethyl ether. The solid was collected by filtration to afford 1-chloro-4-{[(3R)-piperidin-3-yl]oxy}phthalazine monohydrochloride (850 mg) as a solid.

### Production Example 97

Triethylamine (0.26 mL) was added to a mixture of 1-chloro-4-{[(3R)-piperidin-3-yl]oxy}phthalazine monohydrochloride (100 mg) and dichloromethane (2 mL), followed by stirring at room temperature for 30 minutes. Acetyl chloride (0.04 mL) was added thereto, and the mixture was stirred at the same temperature for 2 hours. Water and ethyl acetate were added to the reaction mixture, the organic layer was separated, and the aqueous layer was then extracted with ethyl acetate. The combined organic layer was washed with saturated aqueous sodium chloride solution and dried over anhydrous sodium sulfate, and the solution was then concentrated under reduced pressure (referred to as mixture A). Triethylamine (0.70 mL) was added to a mixture of 1-chloro-4-{[(3R)-piperidin-3-yl]oxy}phthalazine monohydrochloride (300 mg) and dichloromethane (10 mL), followed by stirring at room temperature for 30 minutes. Acetyl chloride (0.10 mL) was added thereto, and the mixture was stirred at the same temperature for 2 hours. Water and ethyl acetate were added to the reaction mixture, the organic layer was separated, and the aqueous layer was then extracted with ethyl acetate. The combined organic layer was washed with saturated aqueous sodium chloride solution and dried over anhydrous sodium sulfate, and the solution was then concentrated under reduced pressure (referred to as mixture B). Mixture A was combined with mixture B and purified by silica gel column chromatography (hexane/ethyl acetate) to afford 1-{(3R)-3-[(4-chlorophthalazin-1-yl)oxy]piperidin-1-yl}ethan-1-one (280 mg).

### Production Example 98

To a mixture of oxepan-4-one (5 g) and diethyl ether (50 mL) was added triethylamine (6.3 mL) under ice cooling, followed by tert-butyldi(methyl)silyl trifluoromethanesulfonate (12.3 mL), and the mixture was stirred at the same temperature for 10 minutes. To the reaction mixture was added triethylamine (1.4 mL), followed by stirring under ice cooling for 1.5 hours. Hexane and water were added to the reaction mixture, and the organic layer was separated. The organic layer was washed with saturated aqueous sodium chloride solution and dried over anhydrous sodium sulfate. The solution was concentrated to afford a mixture (11.7 g) of tert-butyldi(methyl)[(2,3,6,7-tetrahydrooxepin-4-yl)oxy]silane and tert-butyldi(methyl)[(2,5,6,7-tetrahydrooxepin-4-yl)oxy]silane as an oil.

### Production Example 99

Hydrazine monohydrate (0.300 mL) was added to a mixture of 3-{[(1R,2R)-2-hydroxycyclohexyl]amino}-6,7-dihydropyrano[3,4-c]pyrrol-1(4H)-one (500 mg) and 2-propanol (20 mL), and the mixture was stirred at room temperature for 20 hours. After the reaction mixture was concentrated under reduced pressure, the resulting residue was purified by silica gel column chromatography (chloroform/methanol) to afford (1R,2R)-2-[(1-amino-7,8-dihydro-5H-pyrano[3,4-d]pyridazin-4-yl)amino]cyclohexan-1-ol (200 mg) as a solid.

### Production Example 100

Under an argon atmosphere, sodium nitrite (80 mg) was added to a mixture of (1R,2R)-2-[(1-amino-7,8-dihydro-5H-pyrano[3,4-d]pyridazin-4-yl)amino]cyclohexan-1-ol (198 mg), copper(I) chloride (75 mg), and hydrochloric acid (12 M, 2.5 mL) under ice cooling, and the mixture was stirred at the same temperature for 1 hour. To the reaction mixture were added dichloromethane (10 mL), water, and triethylamine (4.2 mL) under ice cooling, and the mixture was stirred. The reaction mixture was filtered through Celite (R) and washed with dichloromethane/methanol (10/1). The organic layer was separated, and the aqueous layer was extracted with dichloromethane. The combined organic layer was dried over anhydrous magnesium sulfate, and the solution was concentrated under reduced pressure. The resulting residue was purified by silica gel column chromatography (chloroform/methanol) to afford (1R,2R)-2-[(1-chloro-7,8-dihydro-5H-pyrano[3,4-d]pyridazin-4-yl)amino]cyclohexan-1-ol (170 mg) as a solid.

### Production Example 101

A mixture of 2-(6-benzyl-4-{[(1R,2R)-2-hydroxycyclohexyl]amino}-5,6,7,8-tetrahydropyrido[3,4-d]pyrida zin-1-yl)-5-(trifluoromethyl)phenol (131.6 mg), 10% palladium on carbon (51% wet, 84.5 mg), and methanol (3 mL) was stirred under a hydrogen atmosphere at room temperature for 4 hours. Chloroform was added to the reaction mixture, followed by filtration through Celite (R), and the filtrate was concentrated under reduced pressure to afford 2-(4-{[(1R,2R)-2-hydroxycyclohexyl]amino}-5,6,7,8-tetrahydropyrido[3,4-d]pyridazin-1-yl)-5-(trifluoromethyl)phenol (89.3 mg) as a solid.

### Production Example 102

To a mixture of cyclobutanone (0.640 g) and dichloromethane (25 mL) were added tert-butyl [(3R)-piperidin-3-yl]carbamate (1.524 g) and sodium triacetoxyborohydride (2.416 g) at room temperature, and the mixture was stirred at room temperature for 5 hours. The reaction mixture was poured into saturated aqueous sodium hydrogen carbonate solution, followed by extraction with dichloromethane. The organic layer was dried over anhydrous sodium sulfate and concentrated under reduced pressure. The resulting residue was purified by silica gel column chromatography (dichloromethane/methanol) to afford tert-butyl [(3R)-1-cyclobutylpiperidin-3-yl]carbamate (1.940 g) as a solid.

### Production Example 103

To a mixture of tert-butyl [(3R)-1-cyclobutylpiperidin-3-yl]carbamate (225 mg) and dichloromethane(1.8 mL) was added a solution of hydrogen chloride in 1,4-dioxane (4 M, 1.8 mL) with stirring at room temperature, and the mixture was stirred at room temperature for 3 hours. The reaction mixture was concentrated under reduced pressure to afford (3R)-1-cyclobutylpiperidin-3-amine dihydrochloride (250 mg) as a solid.

### Production Example 104

To a mixture (393 mg) of (1R,2R)-N¹-(1-chloro-7,8-dihydro-5H-pyrano[3,4-d]pyridazin-4-yl)cyclohexane -1,2-diamine and (1R,2R)-N¹-(4-chloro-7,8-dihydro-5H-pyrano[3,4-d]pyridazin-1-yl)cyclohexane-1,2-diamine were added dichloromethane (14 mL), triethylamine (0.23 mL), and di-tert-butyl dicarbonate (0.34 mL), and the mixture was stirred at room temperature for 1.5 hours. Water was added to the reaction mixture, followed by extraction with dichloromethane. The organic layer was dried over anhydrous sodium sulfate and concentrated under reduced pressure. The resulting residue was purified by silica gel column chromatography (hexane/ethyl acetate) to afford a mixture (352 mg) of tert-butyl {(1R,2R)-2-[(1-chloro-7,8-dihydro-5H-pyrano[3,4-d]pyridazin-4-yl)amino]cyclohexyl}carba mate and tert-butyl {(1R,2R)-2-[(4-chloro-7,8-dihydro-5H-pyrano[3,4-d]pyridazin-1-yl)amino]cyclohexyl}carba mate as a solid.

### Production Example 105

To a mixture of 1,4-dichloro-6-(triphenylmethyl)-6,7-dihydro-5H-pyrrolo[3,4-d]pyridazine (1.569 g) and dichloromethane (18 mL) was added a solution of hydrogen chloride in 1,4-dioxane (4 M, 6 mL) with stirring at room temperature, and the mixture was then stirred at room temperature for 18.5 hours. The reaction mixture was filtered, and the solid collected by filtration was washed with diethyl ether and then dried under reduced pressure to afford 1,4-dichloro-6, 7-dihydro-5H-pyrrolo[3,4-d]pyridazine monohydrochloride (770 mg).

### Production Example 106

To a mixture of 1,4-dichloro-6,7-dihydro-5H-pyrrolo[3,4-d]pyridazine monohydrochloride (765 mg) and dichloromethane (23 mL) were added triethylamine (1.4 mL) and di-tert-butyl dicarbonate (1.2 mL) with stirring under ice cooling, and the mixture was stirred for 17 hours while the temperature was naturally raised to room temperature. Water was poured into the reaction mixture, followed by extraction with dichloromethane. The organic layer was dried over anhydrous sodium sulfate and concentrated under reduced pressure. The resulting solid was triturated with diethyl ether. The precipitated solid was collected by filtration and dried under reduced pressure to afford tert-butyl 1,4-dichloro-5,7-dihydro-6H-pyrrolo[3,4-d]pyridazin-6-carboxylate (788 mg) as a solid. The filtrate was concentrated under reduced pressure, and the resulting residue was purified by silica gel column chromatography (hexane/ethyl acetate) to afford tert-butyl 1,4-dichloro-5,7-dihydro-6H-pyrrolo[3,4-d]pyridazin-6-carboxylate (142 mg) as a solid.

### Production Example 114

A mixture of 1,4-dichloro-7,8-dihydro-5H-pyrano[3,4-d]pyridazine (700 mg) and tert-butyl (3R)-3-aminopiperidine-1-carboxylate (1.4 g) was stirred at 120°C for 24 hours. After being allowed to cool to room temperature, the mixture was purified by silica gel column chromatography (hexane/ethyl acetate) to afford a mixture (481 mg) of tert-butyl (3R)-3-[(1-chloro-7,8-dihydro-5H-pyrano[3,4-d]pyridazin-4-yl)amino]piperidine-1-carboxyla te and tert-butyl (3R)-3-[(4-chloro-7,8-dihydro-5H-pyrano[3,4-d]pyridazin-1-yl)amino]piperidine-1-carboxyla te as a solid.

### Production Example 116

A mixture of 1,4-dichloro-5,7-dihydrothieno[3,4-d]pyridazine (384 mg), (1R,2R)-2-aminocyclohexan-1-ol (426 mg), DIPEA (0.77 mL), and cyclopentanol (7.4 mL) was stirred at 145 to 155°C under an argon atmosphere for 18.5 hours. After the reaction mixture was cooled to room temperature, water and saturated aqueous sodium chloride solution were added thereto, followed by extraction with ethyl acetate. The organic layer was dried over anhydrous sodium sulfate and concentrated under reduced pressure. The resulting residue was purified by silica gel column chromatography (hexane/ethyl acetate) to afford (1R,2R)-2-[(4-chloro-5,7-dihydrothieno[3,4-d]pyridazin-1-yl)amino]cyclohexan-1-ol (236 mg) as a solid.

### Production Example 120

A mixture of 1,4-dichloro-5,7-dihydrofuro[3,4-d]pyridazine (500 mg), (1S,3R)-3-aminocyclohexan-1-ol monohydrochloride (795 mg), DIPEA (1.8 mL), and cyclopentanol (5 mL) was stirred at 120 to 130°C for 22 hours. The reaction mixture was cooled to room temperature, then poured into water, and extracted with ethyl acetate. The organic layer was washed with saturated aqueous sodium chloride solution, then dried over anhydrous sodium sulfate, and concentrated under reduced pressure. The resulting residue was purified by silica gel column chromatography (hexane/ethyl acetate, then ethyl acetate/methanol) to afford (1S,3R)-3-[(4-chloro-5,7-dihydrofuro[3,4-d]pyridazin-1-yl)amino]cyclohexan-1-ol (556 mg) as a solid.

### Production Example 122

A mixture of 2,3,5,7-tetrahydrothieno[3,4-d]pyridazin-1,4-dione (78 mg) and phosphorus oxychloride (1 mL) was stirred at 98 to 100°C for 2.5 hours. The reaction mixture was cooled to room temperature, then poured into ice water, and thoroughly washed with ice water and dichloromethane. The mixture was adjusted to a pH of about 8 by dropwise addition of saturated aqueous sodium hydrogen carbonate solution with stirring at room temperature and extracted with dichloromethane. The organic layer was dried over anhydrous sodium sulfate and concentrated under reduced pressure to afford 1,4-dichloro-5,7-dihydrothieno[3,4-d]pyridazine (92 mg) as a solid.

### Production Example 128

A mixture (478 mg) of tert-butyl (3R)-3-[(1-chloro-7,8-dihydro-5H-pyrano[3,4-d]pyridazin-4-yl)amino]piperidine-1-carboxyla te and tert-butyl (3R)-3-[(4-chloro-7,8-dihydro-5H-pyrano[3,4-d]pyridazin-1-yl)amino]piperidine-1-carboxyla te and a mixture of 2-[4-(difluoromethoxy)-2-(methoxymethoxy)phenyl]-4,4,5,5-tetramethyl-1,3,2-dioxaborolan e (680 mg), RuPhos Pd G3 (163 mg), potassium carbonate (450 mg), 1,4-dioxane (10 mL), and water (2 mL) were stirred at 100°C for 4 hours. The mixture was allowed to cool to room temperature, and water was then added thereto, followed by extraction with ethyl acetate. The organic layer was washed with saturated aqueous sodium chloride solution and then dried over anhydrous magnesium sulfate. After the solution was concentrated under reduced pressure, the resulting residue was purified by silica gel column chromatography (hexane/ethyl acetate) to afford tert-butyl (3R)-3-({1-[4-(difluoromethoxy)-2-(methoxymethoxy)phenyl]-7,8-dihydro-5H-pyrano[3,4-d] pyridazin-4-yl}amino)piperidine-1-carboxylate (150 mg) and tert-butyl (3R)-3-({4-[4-(difluoromethoxy)-2-(methoxymethoxy)phenyl]-7,8-dihydro-5H-pyrano[3,4-d] pyridazin-1-yl}amino)piperidine-1-carboxylate (200 mg) each as a solid.

### Production Example 134

Under an argon atmosphere, a mixture of (1S,3R)-3-[(4-chloro-5,7-dihydrofuro[3,4-d]pyridazin-1-yl)amino]cyclohexan-1-ol (155 mg), 2-[4-(difluoromethyl)-2-(methoxymethoxy)phenyl]-4,4,5,5-tetramethyl-1,3,2-dioxaborolane (361 mg), PdCl₂(dppf)· CH₂Cl₂ (94 mg), potassium carbonate (159 mg), 1,4-dioxane (6 mL), and water (1.4 mL) was stirred at 90 to 110°C for 20 hours. The reaction mixture was cooled to room temperature, poured into water, and then filtered through Celite (R), and the filtrate was extracted with ethyl acetate. The organic layer was washed with saturated aqueous sodium chloride solution, then dried over anhydrous sodium sulfate, and concentrated under reduced pressure. The resulting residue was purified by silica gel column chromatography (hexane/ethyl acetate, then ethyl acetate/methanol) to afford (1S,3R)-3-({4-[4-(difluoromethyl)-2-(methoxymethoxy)phenyl]-5,7-dihydrofuro[3,4-d]pyrid azin-1-yl}amino)cyclohexan-1-ol (128 mg) as a solid.

### Production Example 144

In the same reaction as in Production Example 74, 1,4-dichloro-5, 7,8,9-tetrahydrooxepino[3,4-d]pyridazine was obtained as a solid by-product.

### Production Example 152

To a mixture of 4-{[(1R,2S)-2-{[tert-butyldi(methyl)silyl]oxy}cyclohexyl]amino}-7,8-dihydro-5H-pyrano[3, 4-d]pyridazin-1-ol (261 mg), 2,6-lutidine (0.16 mL), and dichloromethane (6 mL) was added trifluoromethanesulfonic anhydride (0.17 mL) under ice cooling, and the mixture was stirred at the same temperature for 1.5 hours. Dichloromethane and water were added to the reaction mixture, followed by stirring, and two layers were separated. The aqueous layer was extracted with dichloromethane, and the combined organic layer was dried over anhydrous magnesium sulfate. The solution was concentrated under reduced pressure, and the resulting residue was purified by silica gel column chromatography (hexane/ethyl acetate) to afford 4-{[(1R,2S)-2-{[tert-butyldi(methyl)silyl]oxy}cyclohexyl]amino}-7,8-dihydro-5H-pyrano[3, 4-d]pyridazin-1-yl trifluoromethanesulfonate (311 mg) as a solid.

### Production Example 154

After trifluoromethanesulfonic anhydride (14.1 mL) was added dropwise to a mixture of ethyl 5,5-dimethyl-4-oxothiolane-3-carboxylate (16.169 g), DIPEA (15.3 mL), and dichloromethane (80 mL) under an argon atmosphere with stirring at -30°C, the reaction mixture was stirred at -45 to -15°C for 1 hour. Water was added to the reaction mixture, and the temperature was raised to room temperature. Then, the mixture was extracted with dichloromethane. The organic layer was dried over anhydrous sodium sulfate and concentrated under reduced pressure. The resulting residue was purified by silica gel column chromatography (hexane/ethyl acetate) to afford ethyl 5,5-dimethyl-4-[(trifluoromethanesulfonyl)oxy]-2,5-dihydrothiophene-3-carboxylate (17.559 g) as an oil.

### Production Example 159

To a mixture of (1S,2R)-2-aminocyclohexan-1-ol monohydrochloride (608 mg), dichloromethane (8 mL), and 2,6-lutidine (1.1 mL) was added tert-butyldimethylsilyl trifluoromethanesulfonate (1.2 mL) under ice cooling, and the mixture was stirred at the same temperature for 1 hour and at room temperature for 3 hours. To the resulting mixture was added 2,6-lutidine (0.16 mL) and tert-butyldimethylsilyl trifluoromethanesulfonate (0.3 mL) at room temperature, followed by stirring at the same temperature for 20 hours. To the reaction mixture were added dichloromethane and saturated aqueous sodium hydrogen carbonate solution, followed by stirring, and two layers were separated. The aqueous layer was extracted with dichloromethane, and the combined organic layer was dried over anhydrous magnesium sulfate. The solution was concentrated under reduced pressure, and the resulting residue was purified by silica gel column chromatography (chloroform/methanol) to afford (1R,2S)-2-{[tert-butyldi(methyl)silyl]oxy}cyclohexan-1-amine (445 mg) as an oil.

### Production Example 160

To a mixture of tert-butyl (3R)-3-({1-[4-(difluoromethoxy)-2-(methoxymethoxy)phenyl]-7,8-dihydro-5H-pyrano[3,4-d] pyridazin-4-yl}amino)piperidine-1-carboxylate (150 mg) and methanol (3 mL) was added hydrochloric acid (12 M, 0.5 mL) at room temperature, and the mixture was stirred at 60°C for 1 hour. After being allowed to cool to room temperature, the reaction mixture was concentrated under reduced pressure. To the resulting residue were added chloroform/methanol (9/1) and basic silica gel, and the mixture was concentrated under reduced pressure. The resulting residue was purified by silica gel column chromatography (basic silica, chloroform/methanol) to afford 5-(difluoromethoxy)-2-(4-{[(3R)-piperidin-3-yl]amino}-7,8-dihydro-5H-pyrano[3,4-d]pyrida zin-1-yl)phenol (102 mg) as a solid.

### Production Example 162

A mixture of (1R,2R)-2-[(1-chloro-5,7,8,9-tetrahydrooxepino[3,4-d]pyridazin-4-yl)amino]cyclohexan-1-ol and (1R,2R)-2-[(4-chloro-5,7,8,9-tetrahydrooxepino[3,4-d]pyridazin-1-yl)amino]cyclohexan-1-ol (113 mg) was purified by reversed-phase preparative HPLC (ODS column, 0.1% formic acid/methanol) to afford (1R,2R)-2-[(1-chloro-5,7,8,9-tetrahydrooxepino[3,4-d]pyridazin-4-yl)amino]cyclohexan-1-ol (62.4 mg) as an oil.

### Production Example 163

To a mixture of 1-chloro-N-[(3R)-piperidin-3-yl]-7,8-dihydro-5H-pyrano[3,4-d]pyridazin-4-amine dihydrochloride (110 mg) and DMF (6 mL) were added potassium carbonate (197.99 mg) and methyl iodide (58.10 mg) at 25°C, and the mixture was stirred at the same temperature for 16 hours. The reaction mixture was added to water and extracted with ethyl acetate. The organic layer was washed with saturated aqueous sodium chloride solution, then dried over anhydrous sodium sulfate, and concentrated under reduced pressure to afford 1-chloro-N-[(3R)-1-methylpiperidin-3-yl]-7,8-dihydro-5H-pyrano[3,4-d]pyridazin-4-amine (110 mg) as an oil.

### Production Example 164

To a mixture of 3-cyclopropylphenol (20 g) and chloroform (200 mL) was added N-bromosuccinimide (23.88 g) at 0°C, and the mixture was stirred at 15°C for 16 hours. The reaction mixture was concentrated under reduced pressure and purified by silica gel column chromatography (petroleum ether) to afford 2-bromo-5-cyclopropylphenol (7.7 g) as an oil.

### Production Example 165

A mixture of 2,5-dihydrothiophene-3,4-dicarbonohydrazide (127 mg) and 10% sulfuric acid (8 mL) was stirred at 115 to 120°C for 1 hour. The reaction mixture was cooled to room temperature and then filtered. The solid collected by filtration was washed with water and dried to afford 2,3,5,7-tetrahydrothieno[3,4-d]pyridazin-1,4-dione (94 mg) as a solid.

### Production Example 166

A mixture of 4,6-dihydro-1H,3H-thieno[3,4-c]furan-1,3-dione (308 mg), hydrazine monohydrate (0.96 mL), and 2-propanol (20 mL) was stirred at 100°C for 14.5 hours with heating at reflux. The reaction mixture was cooled to room temperature and filtered. The solid collected by filtration was washed with 2-propanol and dried to afford 2,5-dihydrothiophene-3,4-dicarbonohydrazide (246 mg) as a solid.

### Production Example 167

A mixture of tert-butyl 2-[4-(ethoxycarbonyl)-2,2-dimethyl-2,5-dihydrothiophene-3-carbonyl]hydrazine-1-carboxyla te (6.259 g), a solution of hydrogen chloride in 1,4-dioxane (4 M, 9.1 mL), and ethyl acetate (90 mL) was stirred at 60 to 67°C for 19 hours. The reaction mixture was cooled to room temperature and filtered. The solid collected by filtration was washed with ethyl acetate and dried to afford 5,5-dimethyl-2,3,5,7-tetrahydrothieno[3,4-d]pyridazin-1,4-dione (2.717 g) as a solid.

### Production Example 168

A mixture of 4-(ethoxycarbonyl)-2,2-dimethyl-2,5-dihydrothiophene-3-carboxylate (5.095 g), tert-butyl carbazate (3.509 g), HATU(10.095 g), DIPEA (7.73 mL), and DMF (110 mL) was stirred at room temperature for 2.5 hours. Water was added to the reaction mixture, followed by extraction with diethyl ether. The organic layer was washed with water and saturated aqueous sodium chloride solution, dried over anhydrous sodium sulfate, and concentrated under reduced pressure. The resulting residue was purified by silica gel column chromatography (hexane/ethyl acetate) to afford tert-butyl 2-[4-(ethoxycarbonyl)-2,2-dimethyl-2,5-dihydrothiophene-3-carbonyl]hydrazine-1-carboxyla te (6.259 g) as an oil.

### Production Example 169

Under an argon atmosphere, a mixture of ethyl 5,5-dimethyl-4-[(trifluoromethanesulfonyl)oxy]-2,5-dihydrothiophene-3 -carboxylate (15.556 g), sodium formate (9.493 g), acetic anhydride (8.8 mL), DIPEA (16.3 mL), lithium chloride (5.917 g), palladium acetate (1.045 g), and DMF (230 mL) was stirred at room temperature for 17 hours. The reaction mixture was filtered, and the filtrate was adjusted to a pH of about 1 by addition of 10% hydrochloric acid and extracted with ethyl acetate. The organic layer was washed with saturated aqueous sodium chloride solution, dried over anhydrous sodium sulfate, and concentrated under reduced pressure. The resulting residue was purified by silica gel column chromatography (dichloromethane/methanol). To the resulting purified product was added saturated aqueous sodium hydrogen carbonate solution, followed by stirring at room temperature to adjust the pH to about 8. Then, the mixture was washed with ethyl acetate. To the aqueous layer was added 10% hydrochloric acid with stirring at room temperature to adjust the pH to about 1, followed by extraction with ethyl acetate. The organic layer was washed with saturated aqueous sodium chloride solution, dried over anhydrous sodium sulfate, and then concentrated under reduced pressure to afford 4-(ethoxycarbonyl)-2,2-dimethyl-2,5-dihydrothiophene-3-carboxylate (9.659 g) as an oil.

### Example 1

Under an argon atmosphere, a mixture of (1R,2R)-2-[(4-chloro-5,6,7,8-tetrahydrophthalazin-1-yl)amino]cyclohexan-1-ol (4.4 g), [2-hydroxy-4-(trifluoromethyl)phenyl]boronic acid (4.5 g), RuPhos Pd G3(1.3 g), potassium carbonate (5.4 g), 1,4-dioxane (40 mL), and water (8 mL) was stirred at 100°C for 2 hours. To the reaction mixture were added [2-hydroxy-4-(trifluoromethyl)phenyl]boronic acid (965 mg) and RuPhos Pd G3 (260 mg), and the mixture was stirred at 100°C for 3 hours. To the reaction mixture were added [2-hydroxy-4-(trifluoromethyl)phenyl]boronic acid (965 mg) and RuPhos Pd G3 (260 mg), and the mixture was stirred at 100°C for 19 hours. After the reaction mixture was allowed to cool to room temperature, water and chloroform were added thereto, and the organic layer was separated. The aqueous layer was extracted with chloroform, and the combined organic layer was washed with saturated aqueous sodium chloride solution and then dried over anhydrous magnesium sulfate. After the solution was concentrated under reduced pressure, the resulting residue was purified by silica gel column chromatography (basic and neutral silica gel, chloroform/methanol). To the resulting purified product were added ethyl acetate (10 mL) and hexane (50 mL), followed by stirring at room temperature for 18 hours. The solid was collected by filtration to afford 2-(4-{[(1R,2R)-2-hydroxycyclohexyl]amino}-5,6,7,8-tetrahydrophthalazin-1-yl)-5-(trifluoro methyl)phenol (3.97 g) as a solid.

### Example 2

To a mixture (386 mg) of (1R,2R)-2-[(1-chloro-7,8-dihydro-5H-pyrano[3,4-d]pyridazin-4-yl)amino]cyclohexan-1-ol and (1R,2R)-2-[(4-chloro-7,8-dihydro-5H-pyrano[3,4-d]pyridazin-1-yl)amino]cyclohexan-1-ol were added 1,4-dioxane (12 mL), [2-hydroxy-4-(trifluoromethyl)phenyl]boronic acid (562 mg), RuPhos Pd G3 (171 mg), potassium carbonate (563 mg), and water (2.5 mL), and the mixture was stirred under microwave irradiation at 100°C for 1.5 hours. After the reaction mixture was cooled to room temperature, chloroform/methanol (9/1) and water were added thereto, and the organic layer was separated. The organic layer was concentrated, and the resulting residue was then purified by silica gel column chromatography (chloroform/ethyl acetate using neutral silica gel, then chloroform/methanol using basic silica gel). Diethyl ether (3 mL) and hexane (12 mL) were each added to the two purified products obtained, and the mixture was stirred for 15 minutes. The solid was collected by filtration to afford 2-(4-{[(1R,2R)-2-hydroxycyclohexyl]amino}-7,8-dihydro-5H-pyrano[3,4-d]pyridazin-1-yl)-5-(trifluoromethyl)phenol (108 mg) from the low polar fraction and 2-(1-{[(1R,2R)-2-hydroxycyclohexyl]amino}-7,8-dihydro-5H-pyrano[3,4-d]pyridazin-4-yl)-5-(trifluoromethyl)phenol (172 mg) from the high polar fraction each as a solid.

### Example 3

To a mixture (440 mg) of (1R,2R)-2-[(1-chloro-7,8-dihydro-5H-pyrano[3,4-d]pyridazin-4-yl)amino]cyclohexan-1-ol and (1R,2R)-2-[(4-chloro-7,8-dihydro-5H-pyrano[3,4-d]pyridazin-1-yl)amino]cyclohexan-1-ol were added 1,4-dioxane (14 mL), [2-hydroxy-4-(trifluoromethoxy)phenyl]boronic acid (690 mg), RuPhos Pd G3 (195 mg), potassium carbonate (643 mg), and water (2.9 mL), and the mixture was stirred under microwave irradiation at 100°C for 1.5 hours. After the reaction mixture was cooled to room temperature, chloroform/methanol (9/1) and water were added thereto, and the organic layer was separated. After the organic layer was concentrated, the resulting residue was purified by silica gel column chromatography (hexane/ethyl acetate using neutral silica gel, then chloroform/methanol using basic silica gel). Diethyl ether (2 mL) and hexane (8 mL) were each added to the two purified products obtained, and the mixture was stirred for 30 minutes. The solid was collected by filtration to afford 2-(4-{[(1R,2R)-2-hydroxycyclohexyl]amino}-7,8-dihydro-5H-pyrano[3,4-d]pyridazin-1-yl)-5-(trifluoromethoxy)phenol (124 mg) from the low polar fraction and 2-(1-{[(1R,2R)-2-hydroxycyclohexyl]amino}-7,8-dihydro-5H-pyrano[3,4-d]pyridazin-4-yl)-5-(trifluoromethoxy)phenol (242 mg) from the high polar fraction each as a solid.

### Example 4

To a mixture (380 mg) of (1S,2R)-2-[(1-chloro-7,8-dihydro-5H-pyrano[3,4-d]pyridazin-4-yl)amino]cyclohexan-1-ol and (1S,2R)-2-[(4-chloro-7,8-dihydro-5H-pyrano[3,4-d]pyridazin-1-yl)amino]cyclohexan-1-ol were added 1,4-dioxane (12.2 mL), [2-hydroxy-4-(trifluoromethyl)phenyl]boronic acid (553 mg), RuPhos Pd G3 (168 mg), potassium carbonate (555 mg), and water (2.4 mL), and the mixture was stirred under microwave irradiation at 100°C for 1.5 hours. After the reaction mixture was cooled to room temperature, chloroform/methanol (9/1) and water were added thereto, and the organic layer was separated. The organic layer was concentrated, and the resulting residue was then purified by silica gel column chromatography (chloroform/ethyl acetate using neutral silica gel, then chloroform/methanol using basic silica gel). Diethyl ether (2 mL) and hexane (6 mL) were each added to the two purified products obtained, and the mixture was stirred for 1 hour. The solid was collected by filtration to afford 2-(4-{[(1R,2S)-2-hydroxycyclohexyl]amino}-7,8-dihydro-5H-pyrano[3,4-d]pyridazin-1-yl)-5 -(trifluoromethyl)phenol (93 mg) from the low polar fraction and 2-(1-{[(1R,2S)-2-hydroxycyclohexyl]amino}-7,8-dihydro-5H-pyrano[3,4-d]pyridazin-4-yl)-5 -(trifluoromethyl)phenol (103 mg) from the high polar fraction each as a solid.

### Example 5

Under an argon atmosphere, RuPhos Pd G3 (17 mg) was added to a mixture of (1R,2R)-2-[(4-chloro-5,6,8,9-tetrahydrooxepino[4,5-d]pyridazin-1-yl)amino]cyclohexan-1-ol (57.4 mg), [2-hydroxy-4-(trifluoromethyl)phenyl]boronic acid (59.8 mg), potassium carbonate (80 mg), 1,4-dioxane (2.4 mL), and water (0.600 mL), and the mixture was stirred at 100°C for 3 hours. RuPhos Pd G3 (16 mg) was added to the reaction mixture, followed by stirring at 100°C for 1 hour. Then, RuPhos Pd G3 (17.7 mg), 1,4-dioxane (0.800 mL), and water (0.200 mL) were added thereto, and the mixture was stirred at 100°C for 6 hours. After the reaction mixture was cooled to room temperature, chloroform/methanol (9/1), saturated aqueous sodium chloride solution, and water were added thereto, and the mixture was stirred at room temperature for 10 minutes. After the organic layer was separated and concentrated, the resulting residue was purified by silica gel column chromatography (chloroform/ethyl acetate using neutral silica gel, then chloroform/methanol using basic silica gel) to afford 2-(4-{[(1R,2R)-2-hydroxycyclohexyl]amino}-5,6,8,9-tetrahydrooxepino[4,5-d]pyridazin-1-yl )-5-(trifluoromethyl)phenol (9.1 mg) as a solid.

### Example 70

Under an argon atmosphere, a mixture of (1R,2R)-2-[(1-chloro-7,8-dihydro-5H-pyrano[3,4-d]pyridazin-4-yl)amino]cyclohexan-1-ol (150 mg), (4-chloro-2-hydroxyphenyl)boronic acid (120 mg), RuPhos Pd G3 (46 mg), potassium carbonate (150 mg), 1,4-dioxane (4 mL), and water (0.5 mL) was stirred at 100°C for 5 hours. To the reaction mixture was added (4-chloro-2-hydroxyphenyl)boronic acid (120 mg) and RuPhos Pd G3 (22 mg), followed by stirring at 100°C for 8 hours. After the reaction mixture was allowed to cool to room temperature, basic silica gel was added, and the mixture was concentrated under reduced pressure. The resulting residue was purified by silica gel column chromatography (chloroform/methanol using basic silica gel, then chloroform/methanol using neutral silica gel) to afford a solid. To the resulting solid was added hexane/ethyl acetate (10/1), and the solid was collected by filtration to afford 5-chloro-2-(4-{[(1R,2R)-2-hydroxycyclohexyl]amino}-7,8-dihydro-5H-pyrano[3,4-d]pyridaz in-1-yl)phenol (25.2 mg) as a solid.

### Example 73

A mixture of 2-(6-benzyl-4-{[(1R,2R)-2-hydroxycyclohexyl]amino}-5,6,7,8-tetrahydropyrido[3,4-d]pyrida zin-1-yl)-5-(trifluoromethyl)phenol (14 mg), formaldehyde (37% aqueous solution, 0.020 mL), 10% palladium on carbon (50% wet, 10 mg), and methanol (1 mL) was stirred under a hydrogen atmosphere at room temperature for 24 hours. Chloroform was added to the reaction mixture, followed by filtration through Celite (R), and the filtrate was concentrated under reduced pressure. The resulting residue was purified by silica gel column chromatography (chloroform/methanol) to afford 2-(4-{[(1R,2R)-2-hydroxycyclohexyl]amino}-6-methyl-5,6,7,8-tetrahydropyrido[3,4-d]pyrid azin-1-yl)-5-(trifluoromethyl)phenol (5.6 mg) as a solid.

### Example 79

To a mixture of (2R)-1-({6-benzyl-1-[2,4-bis(trifluoromethyl)phenyl]-5,6,7,8-tetrahydropyrido[3,4-d]pyridazi n-4-yl}amino)propan-2-ol (30 mg) and methanol (1 mL) was added 10% palladium on carbon (50% wet, 6 mg), and the mixture was stirred at room temperature under a hydrogen atmosphere for 8 hours. The reaction mixture was filtered through Celite (R), and the filtrate was concentrated under reduced pressure. To a mixture of the resulting residue and dichloromethane (1 mL) were added triethylamine (0.020 mL) and acetyl chloride (6 mg) under ice cooling, and the mixture was stirred at the same temperature for 2 hours. Water was added to the reaction mixture, and the mixture was extracted with chloroform. The organic layer was dried over anhydrous magnesium sulfate, and the solution was concentrated under reduced pressure. The resulting residue was purified by silica gel column chromatography (chloroform/methanol) to afford 1-{1-[2,4-bis(trifluoromethyl)phenyl]-4-{[(2R)-2-hydroxypropyl]amino}-7,8-dihydropyrido[ 3,4-d]pyridazin-6(5H)-yl}ethan-1-one (18 mg) as a solid.

### Example 80

To a mixture of (2R)-1-({6-benzyl-1-[2,4-bis(trifluoromethyl)phenyl]-5,6,7,8-tetrahydropyrido[3,4-d]pyridazi n-4-yl}amino)propan-2-ol (30 mg) and methanol (1 mL) was added 10% palladium on carbon (50% wet, 6 mg), and the mixture was stirred at room temperature under a hydrogen atmosphere for 8 hours. The reaction mixture was filtered through Celite (R), and the filtrate was concentrated under reduced pressure. To a mixture of the resulting residue and dichloromethane (1 mL) were added triethylamine (0.020 mL) and methanesulfonyl chloride (8 mg) under ice cooling, and the mixture was stirred at the same temperature for 30 minutes. Water was added to the reaction mixture, and the mixture was extracted with chloroform. The organic layer was dried over anhydrous magnesium sulfate, and the solution was concentrated under reduced pressure. The resulting residue was purified by silica gel column chromatography (chloroform/methanol) to afford (2R)-1-({1-[2,4-bis(trifluoromethyl)phenyl]-6-(methanesulfonyl)-5,6,7,8-tetrahydropyrido[3, 4-d]pyridazin-4-yl}amino)propan-2-ol (15 mg) as a solid.

### Example 81

To a mixture of 2-[4-(4-acetamidobutoxy)phthalazin-1-yl]-5-(trifluoromethyl)phenyl acetate(398 mg) and methanol (8 mL) was added aqueous sodium hydroxide solution (1 M, 1.73 mL), and the mixture was stirred at room temperature for 3 hours. To the reaction mixture were added hydrochloric acid (1 M, 1.73 mL) and water, followed by extraction with chloroform/2-propanol (4/1). The organic layer was washed with saturated aqueous sodium chloride solution and dried over anhydrous magnesium sulfate. After the solution was concentrated under reduced pressure, the resulting solid was washed with ethyl acetate to afford N-[4-({4-[2-hydroxy-4-(trifluoromethyl)phenyl]phthalazin-1-yl}oxy)butyl]acetamide (309 mg) as a solid.

### Example 82

To a mixture of [(1R,2R)-2-({4-[2-hydroxy-4-(trifluoromethyl)phenyl]phthalazin-1-yl}amino)cyclohexyl]car bamate tert-butyl(0.440 g) and 1,4-dioxane (10 mL) was added a solution of hydrogen chloride in 1,4-dioxane (4 M, 4 mL) at 0°C, and the mixture was warmed to room temperature and stirred for 4 hours. To the reaction mixture was added a solution of hydrogen chloride in 1,4-dioxane (4 M, 4 mL) at 5°C, followed by stirring at the same temperature for 1 hour. The reaction mixture was concentrated under reduced pressure, and the resulting solid was washed with diethyl ether to afford 2-(4-{[(1R,2R)-2-aminocyclohexyl]amino}phthalazin-1-yl)-5-(trifluoromethyl)phenol dihydrochloride (0.375 g) as a solid.

### Example 83

To a mixture of (1R,2R)-2-({4-[4-(difluoromethoxy)-2-(methoxymethoxy)phenyl]-5,6,7,8-tetrahydrophthalaz in-1-yl}amino)cyclohexan-1-ol (106 mg) and methanol (1.1 mL) was added hydrochloric acid (12 M, 0.060 mL), and the mixture was stirred at 60°C for 10 hours. To the reaction mixture were added ethyl acetate and saturated aqueous sodium hydrogen carbonate solution, the organic layer was separated, and the aqueous layer was then extracted with ethyl acetate. The combined organic layer was washed with water and saturated aqueous sodium chloride solution and dried over anhydrous sodium sulfate. The solution was concentrated, and the resulting residue was purified by silica gel column chromatography (hexane/ethyl acetate using neutral silica gel, then chloroform/methanol using basic silica gel). To the resulting solid were added diethyl ether (1 mL) and hexane (3 mL), the mixture was stirred for 15 minutes, and the solid was then collected by filtration to afford 5-(difluoromethoxy)-2-(4-{[(1R,2R)-2-hydroxycyclohexyl]amino}-5,6,7,8-tetrahydrophthala zin-1-yl)phenol (62 mg) as a solid.

### Example 88

To a mixture of (1R,2R)-2-[(1-chloro-7,8-dihydro-5H-pyrano[3,4-d]pyridazin-4-yl)amino]cyclohexan-1-ol (162 mg), 1,4-dioxane (2 mL), and water (0.500 mL) were added potassium carbonate (159 mg), 2-[4-(difluoromethoxy)-2-(methoxymethoxy)phenyl]-4,4,5,5-tetramethyl-1,3,2-dioxaborolan e(564 mg), and RuPhos Pd G3 (49.3 mg) under an argon atmosphere, and the mixture was stirred at 100°C for 1.5 hours. The reaction mixture was cooled to room temperature, methanol (2 mL) and hydrochloric acid (12 M, 0.590 mL) were added thereto, and the mixture was stirred at 60°C for 2 hours. The reaction mixture was allowed to cool to room temperature and then concentrated by addition of basic silica gel. The resulting residue was purified by silica gel column chromatography (chloroform/methanol using basic silica gel, then chloroform/methanol using neutral silica gel). After ethyl acetate and diisopropyl ether were added to the resulting residue and sonicated, the solid was collected by filtration to afford 5-(difluoromethoxy)-2-(4-{[(1R,2R)-2-hydroxycyclohexyl]amino}-7,8-dihydro-5H-pyrano[3 ,4-d]pyridazin-1-yl)phenol (46.3 mg) as a solid.

### Example 90

To a mixture of tert-butyl 4-{[(1R,2R)-2-hydroxycyclohexyl]amino}-1-[2-hydroxy-4-(trifluoromethyl)phenyl]-7,8-dihy dropyrido[3,4-d]pyridazin-6(5H)-carboxylate (171 mg) and dichloromethane (2.6 mL) was added a solution of hydrogen chloride in 1,4-dioxane (4 M, 0.84 mL) under ice cooling, and the mixture was stirred at room temperature for 6 hours. The reaction mixture was concentrated under reduced pressure to afford 2-(4-{[(1R,2R)-2-hydroxycyclohexyl]amino}-5,6,7,8-tetrahydropyrido[3,4-d]pyridazin-1-yl)-5-(trifluoromethyl)phenol dihydrochloride (162 mg) as a solid.

### Example 92

To a mixture of 2-(4-{[(1R,2R)-2-hydroxycyclohexyl]amino}-5,6,7,8-tetrahydropyrido[3,4-d]pyridazin-1-yl)-5-(trifluoromethyl)phenol (46.7 mg), dichloromethane (2 mL), and THF (1 mL) under an argon atmosphere were added acetyl chloride (0.041 mL) and pyridine (0.010 mL) under ice cooling, and the mixture was stirred at the same temperature for 1 hour. The reaction mixture was warmed to room temperature, followed by addition of aqueous sodium hydroxide solution (1 M, 1 mL), and was stirred at the same temperature for 7 hours. The reaction mixture was cooled in ice bath, hydrochloric acid (1 M, 1 mL) was added thereto, and the mixture was then warmed to room temperature, followed by addition of chloroform, and stirred at the same temperature for 10 minutes. The organic layer was separated and concentrated. The resulting residue was purified by silica gel column chromatography (chloroform/methanol) to afford 1-[4-{[(1R,2R)-2-hydroxycyclohexyl]amino}-1-[2-hydroxy-4-(trifluoromethyl)phenyl]-7,8-d ihydropyrido[3,4-d]pyridazin-6(5H)-yl]ethan-1-one (17.2 mg) as a solid.

### Example 93

To a mixture of 2-(4-{[(1R,2R)-2-hydroxycyclohexyl]amino}-5,6,7,8-tetrahydropyrido[3,4-d]pyridazin-1-yl)-5-(trifluoromethyl)phenol (18.5 mg) and dichloromethane (2 mL) under an argon atmosphere were added methanesulfonyl chloride (0.004 mL) and DIPEA (0.039 mL) under ice cooling, and the mixture was stirred at the same temperature for 30 minutes. To the reaction mixture were added saturated aqueous sodium hydrogen carbonate solution and chloroform, and the mixture was warmed to room temperature and stirred for 5 minutes. The organic layer was separated and concentrated (referred to as mixture A). To a mixture of 2-(4-{[(1R,2R)-2-hydroxycyclohexyl]amino}-5,6,7,8-tetrahydropyrido[3,4-d]pyridazin-1-yl)-5-(trifluoromethyl)phenol (22.9 mg) and dichloromethane (3 mL) under an argon atmosphere were added methanesulfonyl chloride (0.005 mL) and DIPEA (0.048 mL) under ice cooling, and the mixture was stirred at the same temperature for 30 minutes. To the reaction mixture were added saturated aqueous sodium hydrogen carbonate solution and chloroform, and the mixture was warmed to room temperature and stirred for 5 minutes. The organic layer was separated and concentrated (referred to as mixture B). Mixture A was combined with mixture B and purified by silica gel column chromatography (chloroform/methanol) to afford 2-[4-{[(1R,2R)-2-hydroxycyclohexyl]amino}-6-(methanesulfonyl)-5,6,7,8-tetrahydropyrido[ 3,4-d]pyridazin-1-yl]-5-(trifluoromethyl)phenol (13.8 mg) as a solid.

### Example 94

Under an argon atmosphere, boron tribromide (17% dichloromethane solution, 2.5 mL) was added dropwise to a mixture of (1R,2R)-2-({4-[2-methoxy-4-(trifluoromethyl)phenyl]thieno[2,3-d]pyridazin-7-yl}amino)cyc lohexan-1-ol (210 mg) and dichloromethane (2.5 mL) with stirring under ice cooling, and the mixture was stirred at 0°C for 2 hours. To the reaction mixture were added saturated aqueous sodium hydrogen carbonate solution and methanol, followed by extraction with dichloromethane/methanol. The organic layer was dried over anhydrous sodium sulfate and concentrated under reduced pressure. The resulting residue was purified by silica gel column chromatography (hexane/ethyl acetate), and the resulting purified product was triturated with dichloromethane. The precipitated solid was collected by filtration, washed with dichloromethane, and then dried under reduced pressure to afford 2-(7-{[(1R,2R)-2-hydroxycyclohexyl]amino}thieno[2,3-d]pyridazin-4-yl)-5-(trifluoromethyl) phenol (75 mg) as a solid.

### Example 95

To a mixture of tert-butyl [(1R,2R)-2-({4-[2-methoxy-4-(trifluoromethyl)phenyl]-7,8-dihydro-5H-pyrano[3,4-d]pyridaz in-1-yl}amino)cyclohexyl]carbamate (73 mg) and dichloromethane (1 mL) was added boron tribromide (17% dichloromethane solution, 0.7 mL) with stirring under ice cooling, and the mixture was stirred for 5 hours while the temperature was naturally raised to room temperature. To the reaction mixture was added saturated aqueous sodium hydrogen carbonate solution, followed by extraction with dichloromethane. The organic layer was dried over anhydrous sodium sulfate and concentrated under reduced pressure. The resulting residue was purified by silica gel column chromatography (methanol) to afford 2-(1-{[(1R,2R)-2-aminocyclohexyl]amino}-7,8-dihydro-5H-pyrano[3,4-d]pyridazin-4-yl)-5-( trifluoromethyl)phenol (44 mg) as a solid.

### Example 96

A mixture of tert-butyl (3R)-3-({4-[2-hydroxy-4-(trifluoromethyl)phenyl]-5,6,7,8-tetrahydrophthalazin-1-yl}amino) piperidine-1-carboxylate (194 mg), dichloromethane (4 mL), and trifluoroacetic acid (0.66 mL) was stirred at room temperature for 5.5 hours. Water was added to the reaction mixture, and the aqueous layer was washed with dichloromethane. The organic layer was extracted with 10% hydrochloric acid, combined with the aqueous layer, and made basic by addition of potassium carbonate and saturated aqueous sodium hydrogen carbonate solution. The aqueous layer was extracted with dichloromethane/methanol. The organic layer was dried over anhydrous sodium sulfate and concentrated under reduced pressure. The resulting residue was triturated with dichloromethane and hexane. The precipitated solid was collected by filtration and dried under reduced pressure to afford 2-(4-{[(3R)-piperidin-3-yl]amino}-5,6,7,8-tetrahydrophthalazin-1-yl)-5-(trifluoromethyl)phe nol (80 mg) as a solid. The filtrate was concentrated under reduced pressure to afford 2-(4-{[(3R)-piperidin-3-yl]amino}-5,6,7,8-tetrahydrophthalazin-1-yl)-5-(trifluoromethyl)phe nol (42 mg) as a solid.

### Example 97

A mixture of 2-(4-{[(3R)-piperidin-3-yl]amino}-5,6,7,8-tetrahydrophthalazin-1-yl)-5-(trifluoromethyl)phe nol (42 mg), dichloromethane(1 mL), methanol (0.1 mL), 3-oxetanone (19.9 µ L), and sodium triacetoxyborohydride(68 mg) was stirred at room temperature for 5 hours. To the reaction mixture were added 3-oxetanone (39.9 µL) and sodium triacetoxyborohydride(136 mg), followed by stirring at room temperature for 14 hours. To the reaction mixture was added saturated aqueous sodium hydrogen carbonate solution, followed by extraction with dichloromethane/methanol. The organic layer was dried over anhydrous sodium sulfate and concentrated under reduced pressure. The resulting residue was purified by silica gel column chromatography (dichloromethane/methanol) to afford 2-(4-{[(3R)-1-(oxetan-3-yl)piperidin-3-yl]amino}-5,6,7,8-tetrahydrophthalazin-1-yl)-5-(triflu oromethyl)phenol (45 mg) as a solid.

### Example 98

A mixture of tert-butyl 1-{[(1R,2R)-2-hydroxycyclohexyl]amino}-4-[2-hydroxy-4-(trifluoromethyl)phenyl]-5,7-dihy dro-6H-pyrrolo[3,4-d]pyridazin-6-carboxylate (264 mg), dichloromethane (5 mL), and trifluoroacetic acid (0.9 mL) was stirred at room temperature for 5 hours. To the reaction mixture were added saturated aqueous sodium hydrogen carbonate solution, dichloromethane, and methanol with stirring under ice cooling. The mixture was extracted with dichloromethane, dried over anhydrous sodium sulfate, and concentrated under reduced pressure. The resulting residue was triturated with ethyl acetate. The precipitated solid was collected by filtration and dried under reduced pressure to afford 2-(4-{[(1R,2R)-2-hydroxycyclohexyl]amino}-6,7-dihydro-5H-pyrrolo[3,4-d]pyridazin-1-yl)-5-(trifluoromethyl)phenol (72 mg) as a solid. The filtrate was concentrated under reduced pressure to afford 2-(4-{[(1R,2R)-2-hydroxycyclohexyl]amino}-6,7-dihydro-5H-pyrrolo[3,4-d]pyridazin-1-yl)-5-(trifluoromethyl)phenol (21 mg) as a solid.

### Example 99

To a mixture of 2-(4-{[(1R,2R)-2-hydroxycyclohexyl]amino}-6,7-dihydro-5H-pyrrolo[3,4-d]pyridazin-1-yl)-5-(trifluoromethyl)phenol (70 mg), methanol (1.8 mL) and 37% aqueous formaldehyde solution (20 µL) was added sodium triacetoxyborohydride (75 mg) with stirring at room temperature, and the mixture was stirred at room temperature for 4 hours. To the reaction mixture was added saturated aqueous sodium hydrogen carbonate solution, and the precipitated solid was collected by filtration and washed with water. The solid collected by filtration was dried and then triturated with hexane/ethyl acetate. The precipitated solid was collected by filtration, washed with hexane, and then dried to afford 2-(4-{[(1R,2R)-2-hydroxycyclohexyl]amino}-6-methyl-6,7-dihydro-5H-pyrrolo[3,4-d]pyrida zin-1-yl)-5-(trifluoromethyl)phenol (35 mg) as a solid.

### Example 111

Under an argon atmosphere, a mixture of (1R,2R)-2-[(4-chloro-5,7-dihydrothieno[3,4-d]pyridazin-1-yl)amino]cyclohexan-1-ol (236 mg), [2-hydroxy-4-(trifluoromethyl)phenyl]boronic acid (342 mg), PdCl₂(dppf)·CH₂Cl₂ (68 mg), potassium carbonate (229 mg), 1,4-dioxane (8 mL), and water (2 mL) was stirred at 107 to 112°C for 14 hours. After the reaction mixture was cooled to room temperature, [2-hydroxy-4-(trifluoromethyl)phenyl]boronic acid (171 mg) and PdCl₂(dppf)·CH₂Cl₂ (68 mg) were added thereto, and the mixture was stirred at 110 to 120°C for another 2 hours. The reaction solution was poured into a mixture of water and saturated aqueous sodium chloride solution, and the mixture was extracted with ethyl acetate. The organic layer was dried over anhydrous sodium sulfate and concentrated under reduced pressure, and the resulting residue was purified by silica gel column chromatography (hexane/ethyl acetate). After the resulting purified product was dissolved in a small amount of dichloromethane, hexane was added thereto, and the precipitated solid was collected by filtration and dried to afford 2-(4-{[(1R,2R)-2-hydroxycyclohexyl]amino}-5,7-dihydrothieno[3,4-d]pyridazin-1-yl)-5-(trif luoromethyl)phenol (39 mg) as a solid.

### Example 112

To a mixture of 1-[4-cyclopropyl-2-(methoxymethoxy)phenyl]-N-[(3R)-1-methylpiperidin-3-yl]-7,8-dihydro-5H-pyrano[3,4-d]pyridazin-4-amine (250 mg) and ethyl acetate (5 mL) was added at a solution of hydrogen chloride in ethyl acetate at 25°C (4 M, 4 mL), and the mixture was stirred at the same temperature for 1 hour. To the reaction mixture were added water and saturated aqueous sodium hydrogen carbonate solution, followed by extraction with ethyl acetate. The organic layer was washed with saturated aqueous sodium chloride solution and then dried over anhydrous sodium sulfate. After the solution was concentrated under reduced pressure, the resulting residue was purified by reversed-phase preparative HPLC (ODS column, aqueous ammonia + aqueous ammonium hydrogen carbonate solution/acetonitrile) to afford 5-cyclopropyl-2-(4-{[(3R)-1-methylpiperidin-3-yl]amino}-7,8-dihydro-5H-pyrano[3,4-d]pyri dazin-1-yl)phenol (29.3 mg) as a solid.

### Example 114

A mixture of (1S,3R)-3-({4-[4-(difluoromethyl)-2-(methoxymethoxy)phenyl]-5,7-dihydrofuro[3,4-d]pyrid azin-1-yl}amino)cyclohexan-1-ol (122 mg), trifluoroacetic acid (0.48 mL), and dichloromethane (2 mL) was stirred at room temperature for 5 hours. While the reaction mixture was stirred under ice cooling, saturated aqueous sodium hydrogen carbonate solution was added thereto until bubbling ceased. The mixture was then extracted with dichloromethane. The organic layer was dried over anhydrous sodium sulfate and concentrated under reduced pressure. The resulting residue was purified by silica gel column chromatography (hexane/ethyl acetate) to afford 5-(difluoromethyl)-2-(4-{[(1R,3S)-3-hydroxycyclohexyl]amino}-5,7-dihydrofuro[3,4-d]pyrid azin-1-yl)phenol (58 mg) as a solid.

### Example 117

To a mixture of 2-(4-{[(1R,2R)-2-hydroxycyclohexyl]amino}-6,7-dihydro-5H-pyrrolo[3,4-d]pyridazin-1-yl)-5-(trifluoromethyl)phenol (62 mg), DIPEA (33 µL), and dichloromethane (1.6 mL) was added acetic anhydride (18 µL) with stirring under ice cooling, and the mixture was then stirred at room temperature for 3 hours. The reaction mixture was filtered, and the solid collected by filtration was washed with dichloromethane and then dried to afford 1-(1-{[(1R,2R)-2-hydroxycyclohexyl]amino}-4-[2-hydroxy-4-(trifluoromethyl)phenyl]-5,7-d ihydro-6H-pyrrolo[3,4-d]pyridazin-6-yl)ethan-1-one (58 mg) as a solid.

### Example 124

To a mixture of 5-(difluoromethoxy)-2-(4-{[(3R)-piperidin-3-yl]amino}-7,8-dihydro-5H-pyrano[3,4-d]pyrida zin-1-yl)phenol (100 mg) and THF (4 mL) were added (1H-benzotriazol-1-yl)methanol (86 mg) and sodium acetate (55 mg) at room temperature, and the mixture was stirred at the same temperature for 10 minutes. Sodium triacetoxyborohydride (125 mg) was added to the resulting mixture, followed by stirring at room temperature for 1 hour. To the reaction mixture were added water and saturated aqueous sodium hydrogen carbonate solution, followed by extraction with chloroform/methanol (9/1). After the organic layer was dried over anhydrous magnesium sulfate, the solution was concentrated under reduced pressure. The resulting residue was purified by silica gel chromatography (basic silica gel, chloroform/methanol). The resulting purified product was triturated with hexane/ethyl acetate. The solid was collected by filtration and dried under reduced pressure to afford 5-(difluoromethoxy)-2-(4-{[(3R)-1-methylpiperidin-3-yl]amino}-7,8-dihydro-5H-pyrano[3,4 -d]pyridazin-1-yl)phenol (72 mg) as a solid.

### Example 126

A mixture of 2-(4-{[(3R)-piperidin-3-yl]amino}-5,7-dihydrothieno[3,4-d]pyridazin-1-yl)-5-(trifluoromethy l)phenol (71 mg), [(1-ethoxycyclopropyl)oxy]tri(methyl)silane (72 µL), sodium cyanoborohydride (17 mg), acetic acid (16 µL), THF (1 mL), and methanol (1 mL) was stirred at 49 to 54°C for 15.5 hours. The reaction mixture was cooled to room temperature and adjusted to a pH of about 7 to 8 by addition of 10% aqueous sodium hydroxide solution, followed by extraction with dichloromethane. The organic layer was washed with saturated aqueous sodium hydrogen carbonate solution and saturated aqueous sodium chloride solution, dried over anhydrous sodium sulfate, and concentrated under reduced pressure. The resulting residue was purified by silica gel column chromatography (dichloromethane/methanol) to afford 2-(4-{[(3R)-1-cyclopropylpiperidin-3-yl]amino}-5,7-dihydrothieno[3,4-d]pyridazin-1-yl)-5-( trifluoromethyl)phenol (15 mg) as a solid.

### Example 127

To a mixture of 2-(4-{[(1R,2S)-2-{[tert-butyldi(methyl)silyl]oxy}cyclohexyl]amino}-7,8-dihydro-5H-pyrano [3,4-d]pyridazin-1-yl)-5-(trifluoromethoxy)phenol (60 mg) and methanol (2 mL) was added hydrochloric acid (12 M, 0.5 mL) at room temperature, and the mixture was stirred at the same temperature for 2 hours. After the reaction mixture was cooled with ice bath, aqueous sodium hydroxide solution (1 M, 6 mL) was added thereto, and the mixture was extracted with chloroform and chloroform/methanol (9/1). After the combined organic layer was dried over anhydrous magnesium sulfate, the solution was concentrated under reduced pressure. The resulting residue was purified by silica gel chromatography (basic silica gel, chloroform/methanol). The resulting purified product was triturated with hexane/ethyl acetate. The solid was collected by filtration and dried under reduced pressure to afford 2-(4-{[(1R,2S)-2-hydroxycyclohexyl]amino}-7,8-dihydro-5H-pyrano[3,4-d]pyridazin-1-yl)-5 -(trifluoromethoxy)phenol (25.7 mg) as a solid.

### Example 128

TFA (0.5 mL) was added to a mixture of tert-butyl (3R)-3-({1-[2-hydroxy-4-(trifluoromethoxy)phenyl]-7,8-dihydro-5H-pyrano[3,4-d]pyridazin-4-yl}amino)piperidine-1-carboxylate (29 mg) and dichloromethane (2 mL) at room temperature, and the mixture was stirred at the same temperature for 1 hour. After the reaction mixture was concentrated under reduced pressure, THF (4 mL), sodium acetate (15 mg), and (1H-benzotriazol-1-yl)methanol (25 mg) were added to the residue, and the mixture was then stirred at room temperature for 10 minutes. Sodium triacetoxyborohydride (36 mg) was added to the resulting mixture, followed by stirring at room temperature for 2 hours. To the reaction mixture were added saturated aqueous sodium hydrogen carbonate solution and saturated aqueous sodium chloride solution, followed by extraction with chloroform/methanol (9/1). The aqueous layer was neutralized by addition of 1 M hydrochloric acid, followed by extraction with chloroform/methanol (9/1). After the combined organic layer was dried over anhydrous magnesium sulfate, the solution was concentrated under reduced pressure. The resulting residue was purified by silica gel chromatography (basic silica gel, chloroform/methanol). The resulting purified product was triturated with hexane/ethyl acetate. The solid was collected by filtration and dried under reduced pressure to afford 2-(4-{[(3R)-1-methylpiperidin-3-yl]amino}-7,8-dihydro-5H-pyrano[3,4-d]pyridazin-1-yl)-5-( trifluoromethoxy)phenol (14.3 mg) as a solid.

### Example 129

To a mixture of 2-(4-{[(1R,2R)-2-hydroxycyclohexyl]amino}-7,8-dihydro-5H-thiopyrano[3,4-d]pyridazin-1-yl)-5-(trifluoromethyl)phenol (300 mg), acetonitrile (5 mL), and water (5 mL) was added Oxone (R) (1.3 g) at 25°C, and the mixture was stirred at the same temperature for 2 hours. Water was added to the reaction mixture, and the mixture was extracted with ethyl acetate. The organic layer was washed with water and then dried over anhydrous sodium sulfate, and the solution was concentrated under reduced pressure. Acetonitrile and water were added to the resulting residue, and the mixture was lyophilized to afford 4-{[(1R,2R)-2-hydroxycyclohexyl]amino}-1-[2-hydroxy-4-(trifluoromethyl)phenyl]-7,8-dihy dro-6λ⁶-thiopyrano[3,4-d]pyridazin-6,6(5H)-dione (185 mg) as a solid.

### Example 132

A mixture of 2-(7,7-dimethyl-4-{[(3R)-piperidin-3-yl]amino}-5,7-dihydrothieno[3,4-d]pyridazin-1-yl)-5-(t rifluoromethyl)phenol (50 mg), 2,2-difluoroethyl trifluoromethanesulfonate (19 µL), triethylamine (49 µL), and ethanol (1.2 mL) was stirred at 54 to 62°C for 17 hours. After the reaction mixture was cooled to room temperature and concentrated under reduced pressure, water was added thereto, and the mixture was extracted with dichloromethane. The organic layer was dried over anhydrous sodium sulfate and concentrated under reduced pressure. The resulting residue was purified by silica gel column chromatography (hexane/ethyl acetate, then dichloromethane/methanol). The resulting purified product was dissolved in toluene and extracted with 5% aqueous sodium hydroxide solution, water, and saturated aqueous sodium chloride solution. The aqueous layer was washed with toluene, 10% hydrochloric acid was added thereto, and the mixture was then extracted with ethyl acetate. The organic layer was washed with saturated aqueous sodium chloride solution, dried over anhydrous sodium sulfate, and concentrated under reduced pressure. The resulting residue was purified by silica gel column chromatography (dichloromethane/hexane/methanol, then hexane/ethyl acetate) to afford 2-(4-{[(3R)-1-(2,2-difluoroethyl)piperidin-3-yl]amino}-7,7-dimethyl-5,7-dihydrothieno[3,4-d]pyridazin-1-yl)-5-(trifluoromethyl)phenol (6 mg) as a solid.

### Example 133

To a mixture of 2-(4-{[(1R,2R)-2-hydroxycyclohexyl]amino}-7,8-dihydro-5H-pyrano[3,4-d]pyridazin-1-yl)-5-(trifluoromethyl)phenol (150 mg) and dichloromethane (1.5 mL) was added a solution of hydrogen chloride in 1,4-dioxane (4 M, 0.11 mL) at room temperature, and the mixture was stirred at the same temperature for 1 hour. The reaction mixture was concentrated under reduced pressure, then ethyl acetate (3 mL) was added thereto, and the mixture was stirred at 50°C for 48 hours. After the mixture was cooled to room temperature, the solid was collected by filtration, washed with cold ethyl acetate (1 mL), and dried under reduced pressure to afford 2-(4-{[(1R,2R)-2-hydroxycyclohexyl]amino}-7,8-dihydro-5H-pyrano[3,4-d]pyridazin-1-yl)-5-(trifluoromethyl)phenol monohydrochloride (149 mg) as a crystal.

### Example 134

To a mixture of 2-(4-{[(1R,2R)-2-hydroxycyclohexyl]amino}-5,6,7,8-tetrahydrophthalazin-1-yl)-5-(trifluoro methyl)phenol (100 mg) and dichloromethane (2 mL) was added a solution of hydrogen chloride in 1,4-dioxane (4 M, 0.074 mL) at room temperature, and the mixture was stirred at the same temperature for 1 hour. The reaction mixture was concentrated under reduced pressure, then ethyl acetate (2 mL) was added thereto, and the mixture was stirred at room temperature for 120 hours. After the mixture was cooled in ice bath, the solid was collected by filtration, washed with cold ethyl acetate (1 mL), and dried under reduced pressure to afford 2-(4-{[(1R,2R)-2-hydroxycyclohexyl]amino}-5,6,7,8-tetrahydrophthalazin-1-yl)-5-(trifluoro methyl)phenol monohydrochloride (103 mg) as a solid.

### Example 135

To a mixture of 2-(4-{[(1R,2R)-2-hydroxycyclohexyl]amino}-7,8-dihydro-5H-pyrano[3,4-d]pyridazin-1-yl)-5-(trifluoromethoxy)phenol (100 mg) and dichloromethane (2 mL) was added a solution of hydrogen chloride in 1,4-dioxane (4 M, 0.071 mL) at room temperature, and the mixture was stirred at the same temperature for 1 hour. The reaction mixture was concentrated under reduced pressure, then ethyl acetate (2 mL) was added thereto, and the mixture was stirred at room temperature for 120 hours. After the mixture was cooled in ice bath, the solid was collected by filtration, washed with cold ethyl acetate (1 mL), and dried under reduced pressure to afford 2-(4-{[(1R,2R)-2-hydroxycyclohexyl]amino}-7,8-dihydro-5H-pyrano[3,4-d]pyridazin-1-yl)-5-(trifluoromethoxy)phenol monohydrochloride (95 mg) as a crystal.

### Example 136

To a mixture of 5-(difluoromethoxy)-2-(4-{ [(3 R)-1-methylpiperidin-3-yl] amino}-7, 8-dihydro-SH-pyrano[3,4 -d]pyridazin-1-yl)phenol (100 mg) and dichloromethane (2 mL) was added a solution of hydrogen chloride in 1,4-dioxane (4 M, 0.147 mL) at room temperature, and the mixture was stirred at the same temperature for 1 hour. The reaction mixture was concentrated under reduced pressure, then ethyl acetate (2 mL) was added thereto, and the mixture was stirred at room temperature for 120 hours. After the mixture was cooled in ice bath, the solid was collected by filtration, washed with cold ethyl acetate (1 mL), and dried under reduced pressure to afford 5-(difluoromethoxy)-2-(4-{[(3R)-1-methylpiperidin-3-yl]amino}-7,8-dihydro-5H-pyrano[3,4 -d]pyridazin-1-yl)phenol dihydrochloride (122 mg) as a solid.

In the same manner as in the methods of Production Examples or Examples described above, compounds of Production Examples and Examples shown in Tables below were produced.

**[Table 3-1]**

| PEx | Str | PEx | Str |
|---|---|---|---|
| 1 | 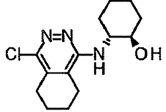 | 10 | 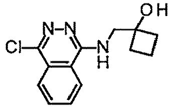 |
| 2 | 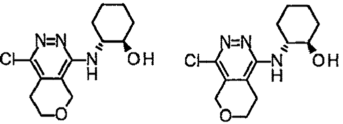 | 11 | 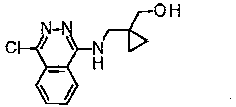 |
| 3 (1) | 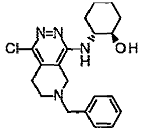 | 12 | 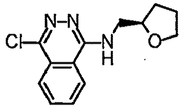 |
| 3 (2) | 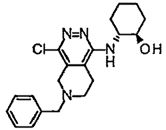 | 13 | 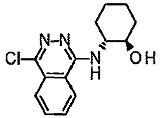 |
| 4 | 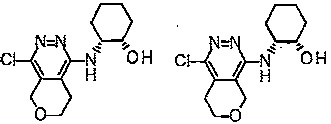 | 14 | 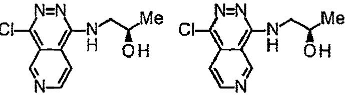 I |
| 5 | 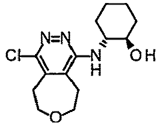 | 15 | 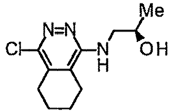 |
| 6 | 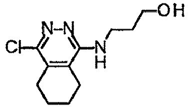 | 16 | 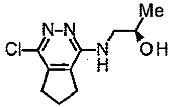 |
| 7 | 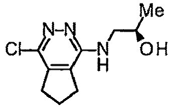 | 17 | 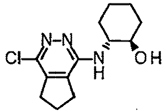 |
| 8 | 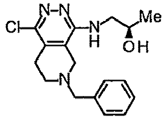 | 18 | 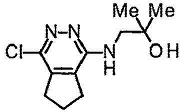 |
| 9 | 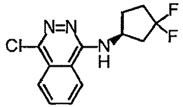 | 19 | 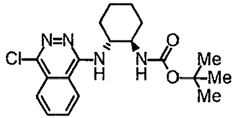 |

**[Table 3-2]**

| PEx | Str | PEx | Str |
|---|---|---|---|
| 20 | 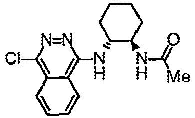 | 29 | 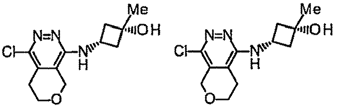 |
| 21 | 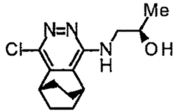 | 30 | 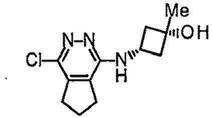 |
| 22 | 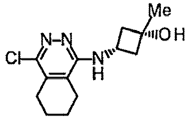 | 31 | 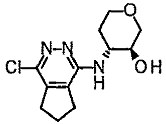 |
| 23 | 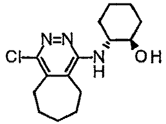 | 32 | 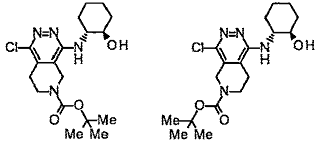 |
| 24 | 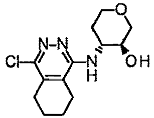 | 33 | 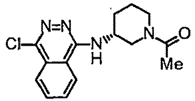 |
| 25 (1) | 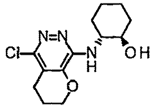 | 34 | 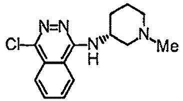 |
| 25 (2) | 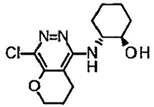 | 35 | 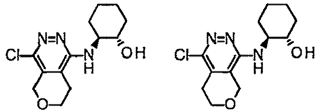 |
| 26 | 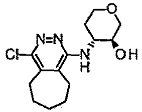 | 36 | 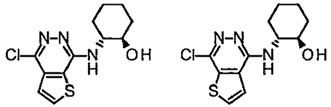 |
| 27 | 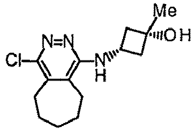 | 37 | 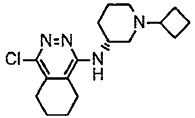 |
| 28 | 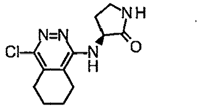 | 38 | 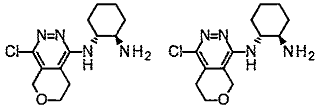 |

**[Table 3-3]**

| PEx | Str | PEx | Str |
|---|---|---|---|
| 39 | 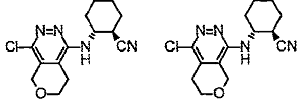 | 49 | 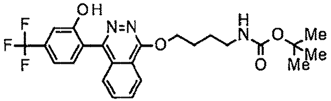 |
| 40 | 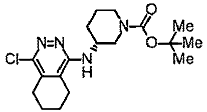 | 50 | 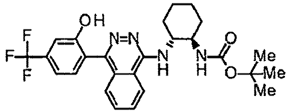 |
| 41 | 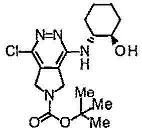 | 51 | 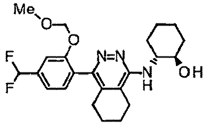 |
| 42 | 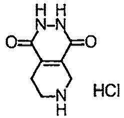 | 52 | 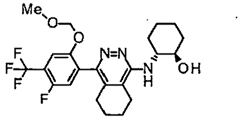 |
| 43 | 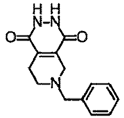 | 53 | 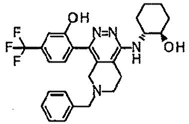 |
| 44 | 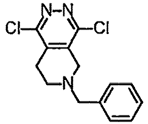 | 54 (1) | 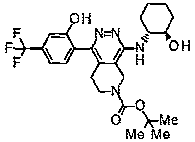 |
| 45 | 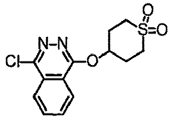 | 54 (2) | 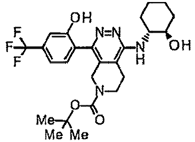 |
| 46 | 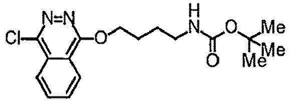 | 55 | 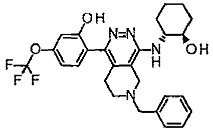 |
| 47 | 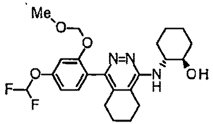 | 56 | 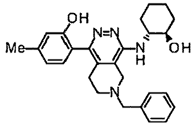 |
| 48 | 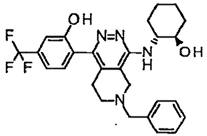 | 57 | 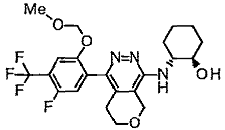 |

**[Table 3-4]**

| PEx | Str | PEx | Str |
|---|---|---|---|
| 58 | 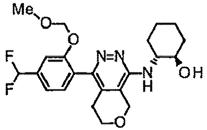 | 68 | 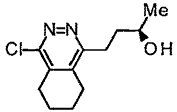 |
| 59 | 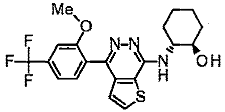 | 69 | 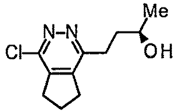 |
| 60 | 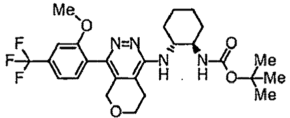 | 70 | 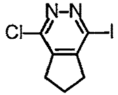 |
| 61 | 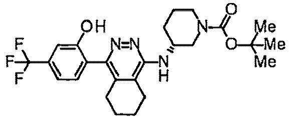 | 71 | 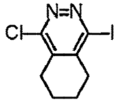 |
| 62 | 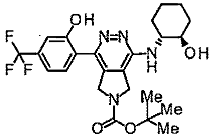 | 72 | 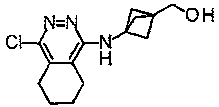 |
| 63 | 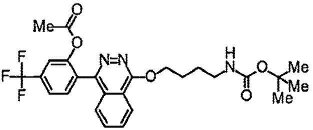 | 73 | 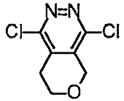 |
| 64 | 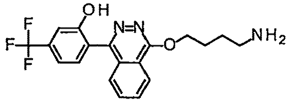 | 74 | 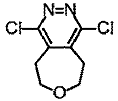 |
| 65 | 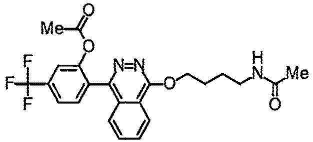 | 75 | 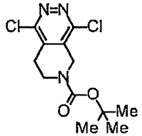 |
| 66 | 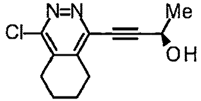 | 76 | 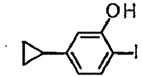 |
| 67 | 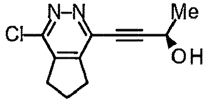 | 77 | 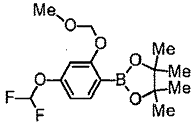 |

**[Table 3-5]**

| PEx | Str | PEx | Str |
|---|---|---|---|
| 78 | 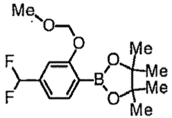 | 88 | 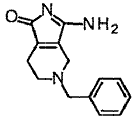 |
| 79 | 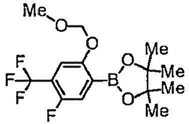 | 89 | 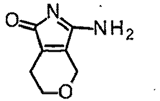 |
| 80 | 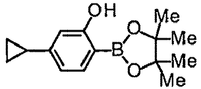 | 90 | 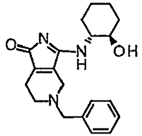 |
| 81 | 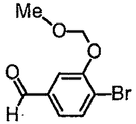 | 91 | 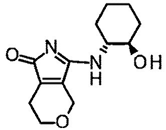 |
| 82 | 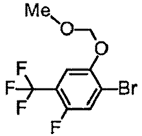 | 92 | 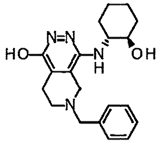 |
| 83 | 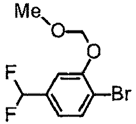 | 93 | 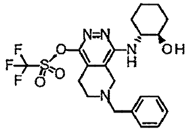 |
| 84 | 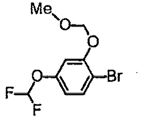 | 94 | 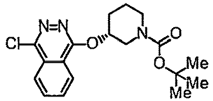 |
| 85 | 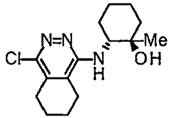 | 95 | 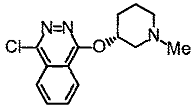 |
| 86 | 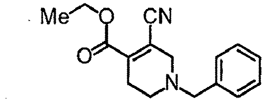 | 96 | 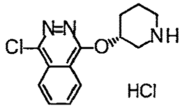 |
| 87 | 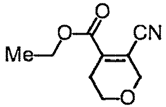 | 97 | 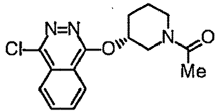 |

**[Table 3-6]**

| PEx | Str | PEx | Str |
|---|---|---|---|
| 98 | 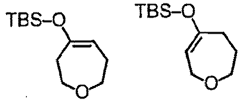 | 103 | 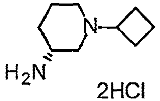 |
| 99 | 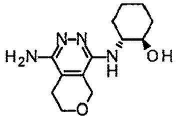 | 104 | 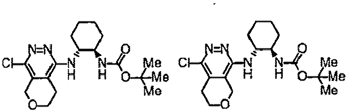 |
| 100 | 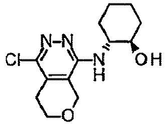 | 105 | 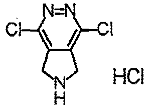 |
| 101 | 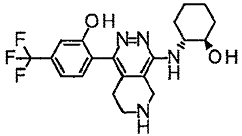 | 106 | 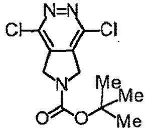 |
| 102 | 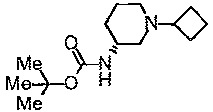 | | |

**[Table 3-7]**

| PEx | Str | PEx | Str |
|---|---|---|---|
| 107 | 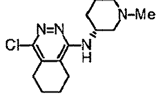 | 116 | 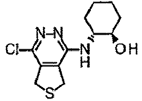 |
| 108 | 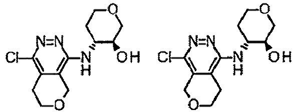 | 117 | 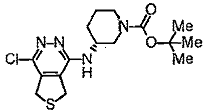 |
| 109 (1) | 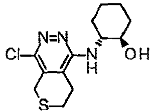 | 118 | 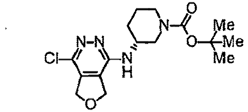 |
| 109 (2) | 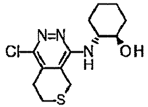 | 119 | 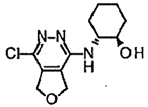 |
| 110 | 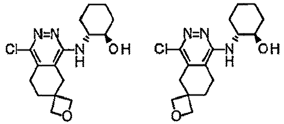 | 120 | 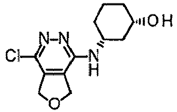 |
| 111 | 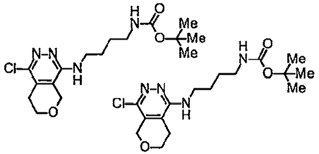 | 121 | 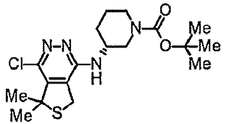 |
| 112 | 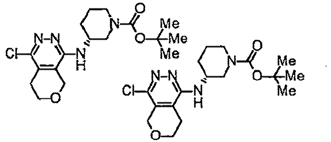 | 122 | 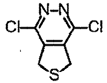 |
| 113 | 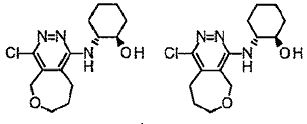 | 123 | 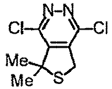 |
| 114 | 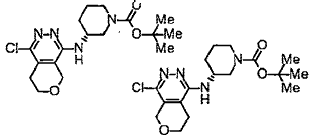 | 124 | 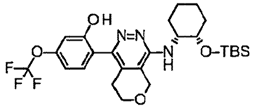 |
| 115 | 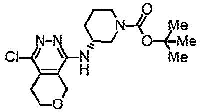 | 125 | 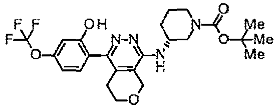 |

**[Table 3-8]**

| PEx | Str | PEx | Str |
|---|---|---|---|
| 126 | 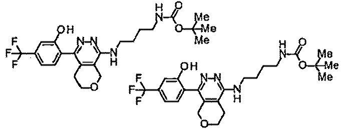 | 135 | 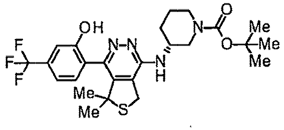 |
| 127 | 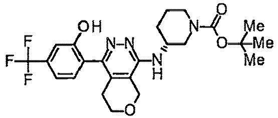 | 136 | 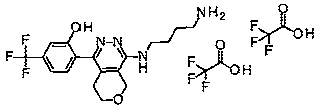 |
| 128 (1) | 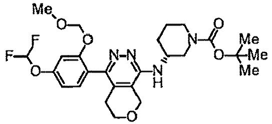 | | 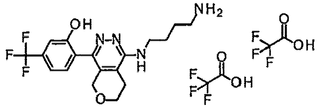 |
| 128 (2) | 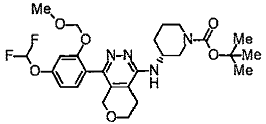 | 137 | 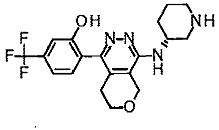 |
| 129 | 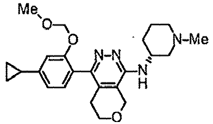 | 138 | 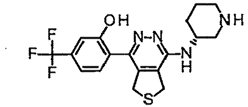 |
| 130 | 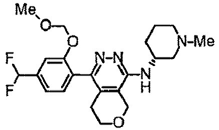 | 139 | 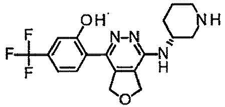 |
| 131 | 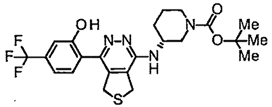 | 140 | 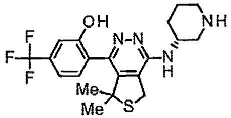 |
| 132 | 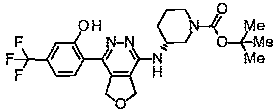 | 141 | 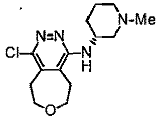 |
| 133 | 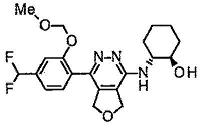 | 142 | 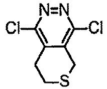 |
| 134 | 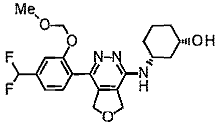 | 143 | 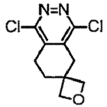 |

**[Table 3-9]**

| PEx | Str | PEx | Str |
|---|---|---|---|
| 144 | 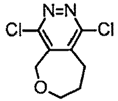 | 154 | 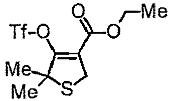 |
| 145 | 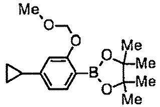 | 155 | 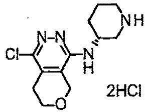 |
| 146 | 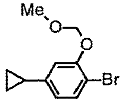 | 156 | 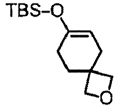 |
| 147 | 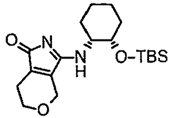 | 157 | 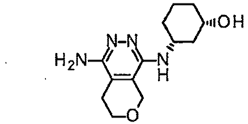 |
| 148 | 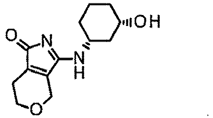 | 158 | 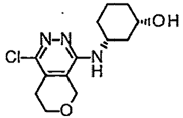 |
| 149 | 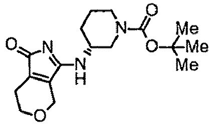 | 159 | 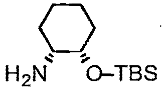 |
| 150 | 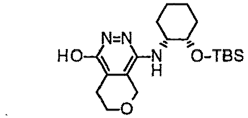 | 160 | 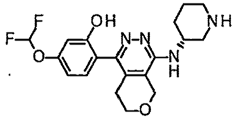 |
| 151 | 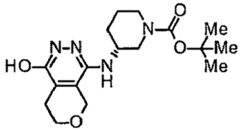 | 161 | 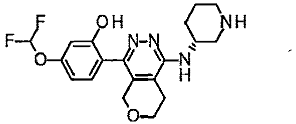 |
| 152 | 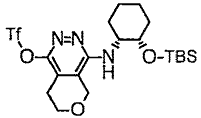 | 162 | 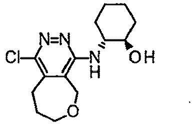 |
| 153 | 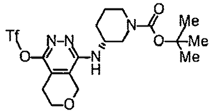 | 163 | 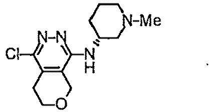 |

**[Table 3-10]**

| PEx | Str | PEx | Str |
|---|---|---|---|
| 164 | 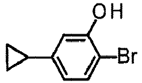 | 167 | 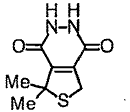 |
| 165 | 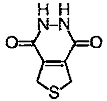 | 168 | 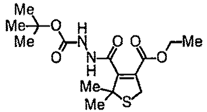 |
| 166 | 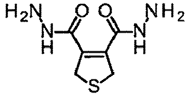 | 169 | 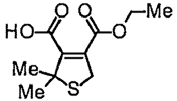 |

**[Table 4-1]**

| Ex | Str | Ex | Str |
|---|---|---|---|
| 1 | 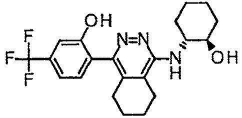 | 8 | 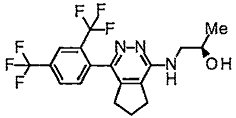 |
| 2 (1) | 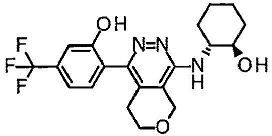 | 9 | 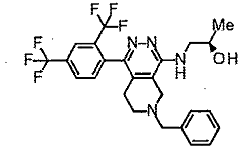 |
| 2 (2) | 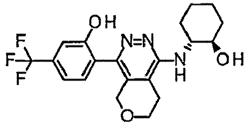 | 10 | 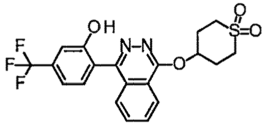 |
| 3 (1) | 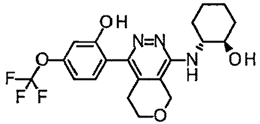 | 11 | 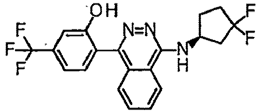 |
| 3 (2) | 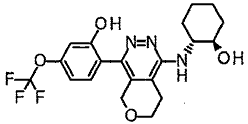 | 12 | 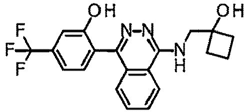 |
| 4 (1) | 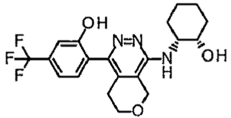 | 13 | 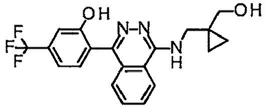 |
| 4 (2) | 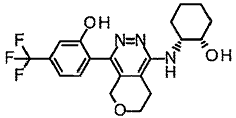 | 14 | 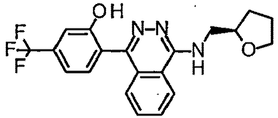 |
| 5 | 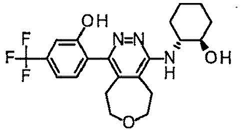 | 15 | 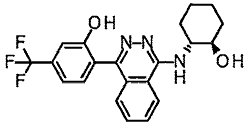 |
| 6 | 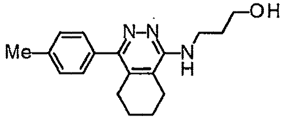 | 16 (1) | 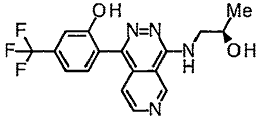 |
| 7 | 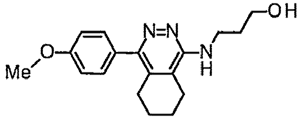 | 16 (2) | 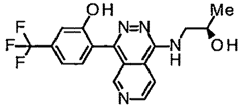 |

**[Table 4-2]**

| Ex | Str | Ex | Str |
|---|---|---|---|
| 17 | 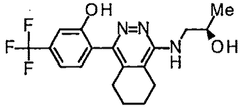 | 27 | 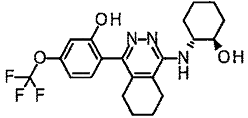 |
| 18 | 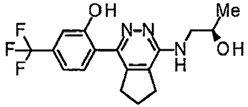 | 28 | 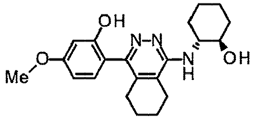 |
| 19 | 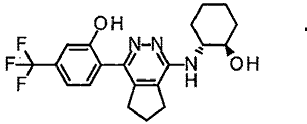 | 29 | 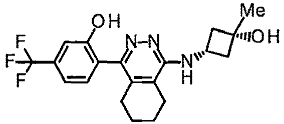 |
| 20 | 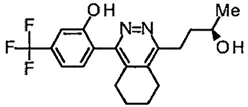 | 30 | 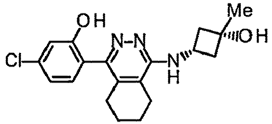 |
| 21 | 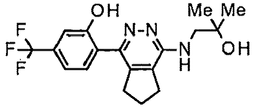 | 31 | 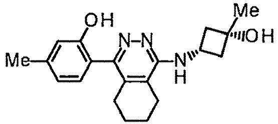 |
| 22 | 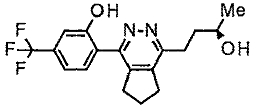 | 32 | 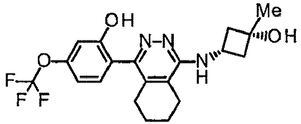 |
| 23 | 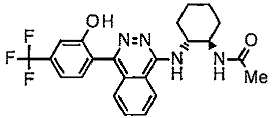 | 33 | 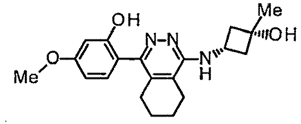 |
| 24 | 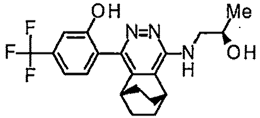 | 34 | 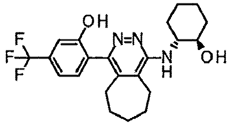 |
| 25 | 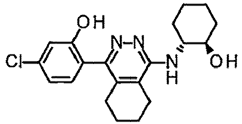 | 35 | 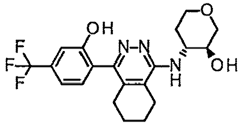 |
| 26 | 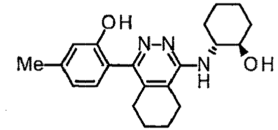 | 36 | 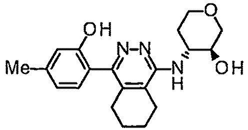 |

**[Table 4-3]**

| Ex | Str | Ex | Str |
|---|---|---|---|
| 37 | 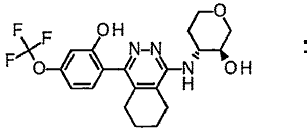 | 47 | 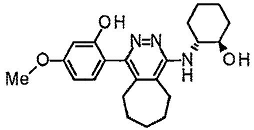 |
| 38 | 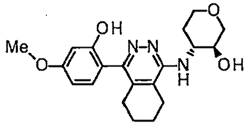 | 48 | 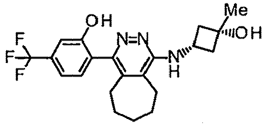 |
| 39 | 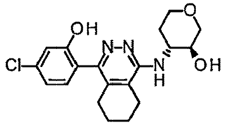 | 49 | 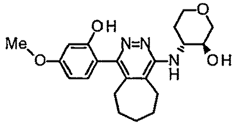 |
| 40 | 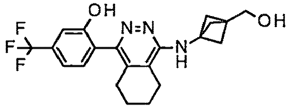 | 50 | 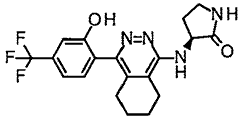 |
| 41 | 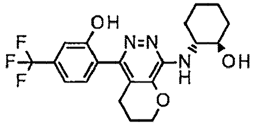 | 51 | 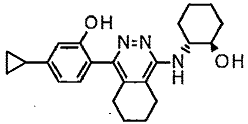 |
| 42 | 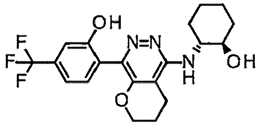 | 52 (1) | 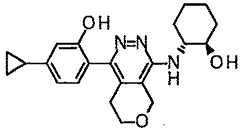 |
| 43 | 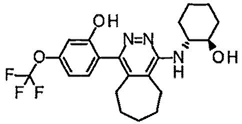 | 52 (2) | 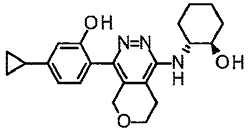 |
| 44 | 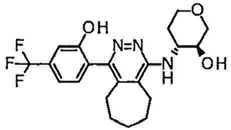 | 53 | 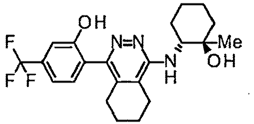 |
| 45 | 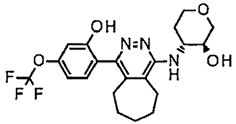 | 54 | 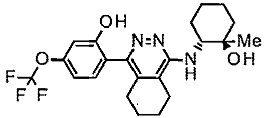 |
| 46 | 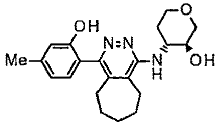 | 55 | 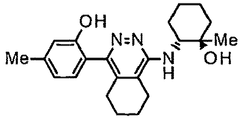 |

**[Table 4-4]**

| Ex | Str | Ex | Str |
|---|---|---|---|
| 56 (1) | 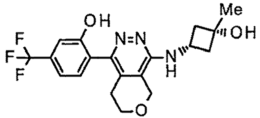 | 63 | 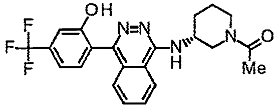 |
| 56 (2) | 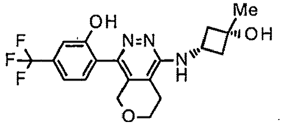 | 64 | 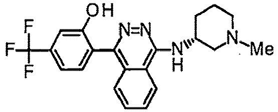 |
| 57 (1) | 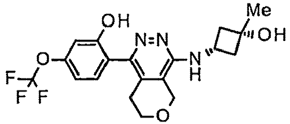 | 65 | 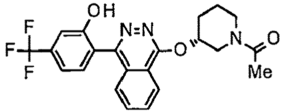 |
| 57 (2) | 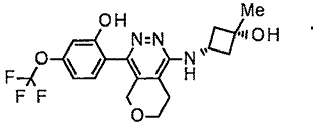 | 66 (1) | 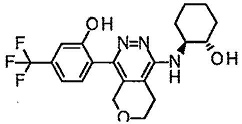 |
| 58 | 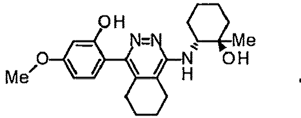 | 66 (2) | 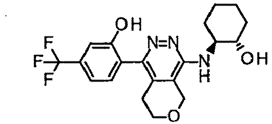 |
| 59 | 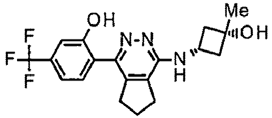 | 67 | 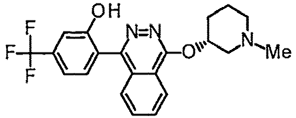 |
| 60 | 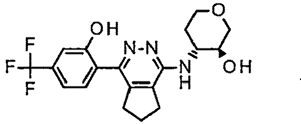 | 68 | 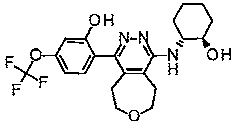 |
| 61 | 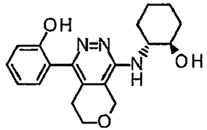 | 69 | 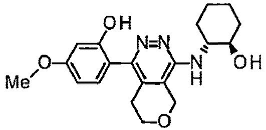 |
| 62 (1) | 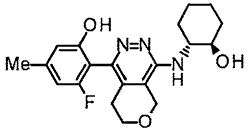 | 70 | 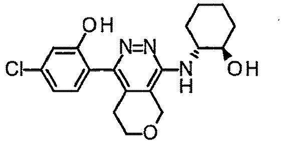 |
| 62 (2) | 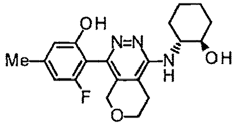 | 71 | 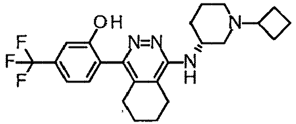 |

**[Table 4-5]**

| Ex | Str | Ex | Str |
|---|---|---|---|
| 72 (1) | 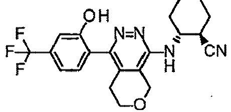 | 81 | 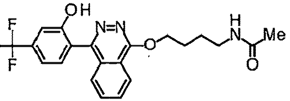 |
| 72 (2) | 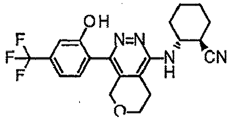 | 82 | 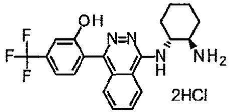 |
| 73 | 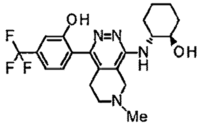 | 83 | 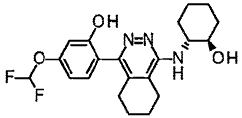 |
| 74 | 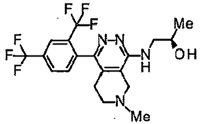 | 84 | 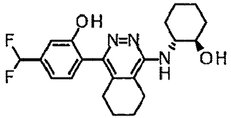 |
| 75 | 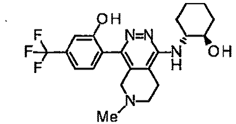 | 85 | 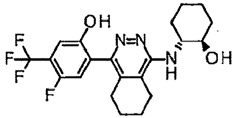 |
| 76 | 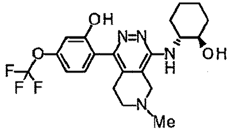 | 86 | 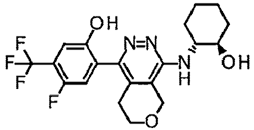 |
| 77 | 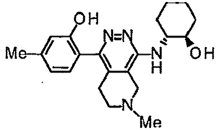 | 87 | 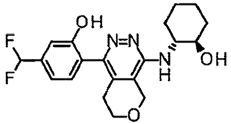 |
| 78 | 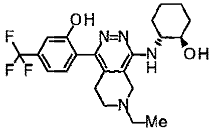 | 88 | 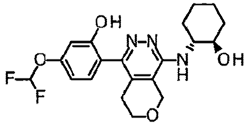 |
| 79 | 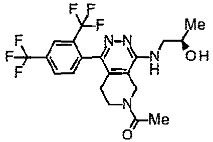 | 89 | 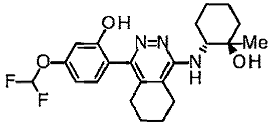 |
| 80 | 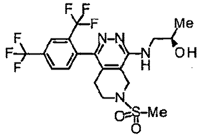 | 90 | 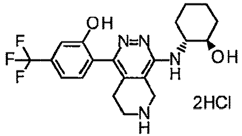 |

**[Table 4-6]**

| Ex | Str | Ex | Str |
|---|---|---|---|
| 91 | 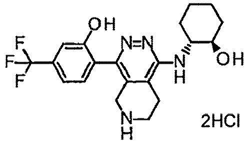 | 96 | 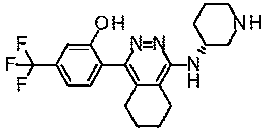 |
| 92 | 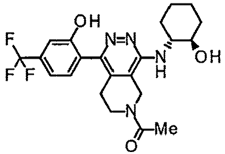 | 97 | 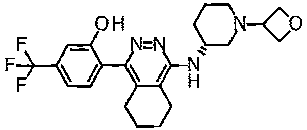 |
| 93 | 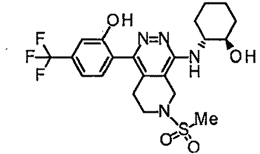 | 98 | 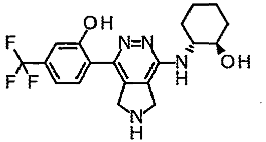 |
| 94 | 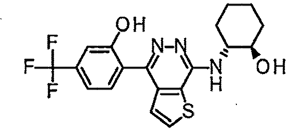 | 99 | 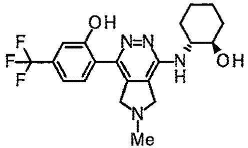 |
| 95 | 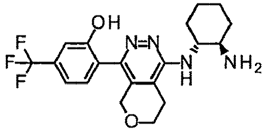 | | |

**[Table 4-7]**

| Ex | Str | Ex | Str |
|---|---|---|---|
| 100 | 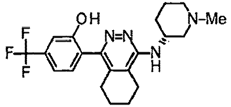 | 108 (2) | 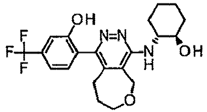 |
| 101 (1) | 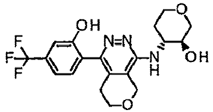 | 109 | 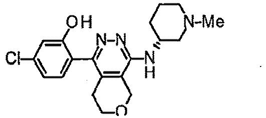 |
| 101 (2) | 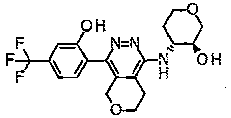 | 110 | 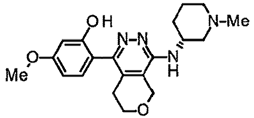 |
| 102 | 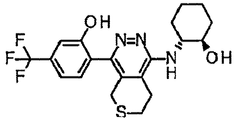 | 111 | 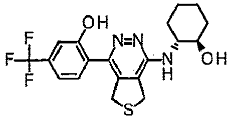 |
| 103 | 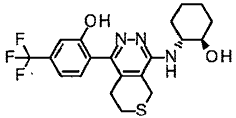 | 112 | 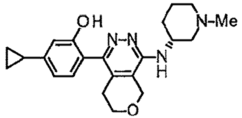 |
| 104 | 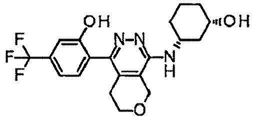 | 113 | 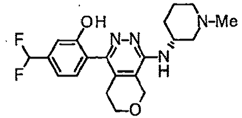 |
| 105 | 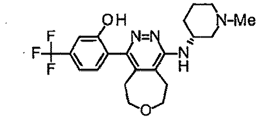 | 114 | 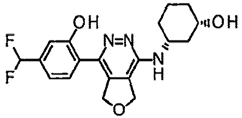 |
| 106 | 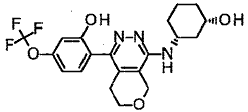 | 115 | 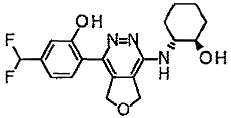 |
| 107 | 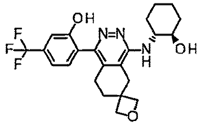 | 116 | 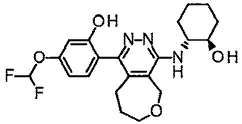 |
| 108 (1) | 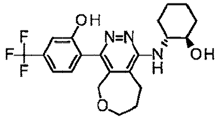 | 117 | 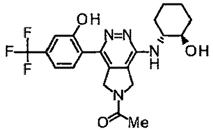 |

**[Table 4-8]**

| Ex | Str | Ex | Str |
|---|---|---|---|
| 118 | 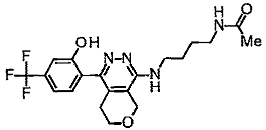 | 128 | 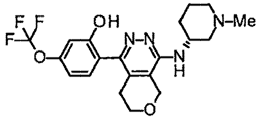 |
| 119 | 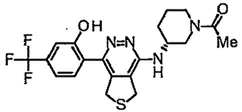 | 129 | 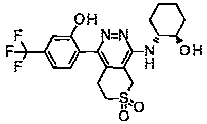 |
| 120 | 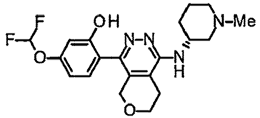 | 130 | 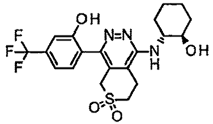 |
| 121 | 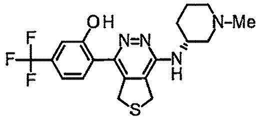 | 131 (1) | 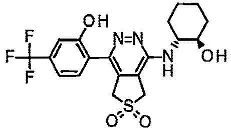 |
| 122 | 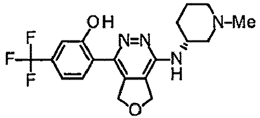 | 131 (2) | 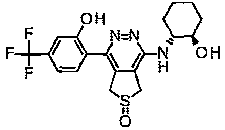 |
| 123 | 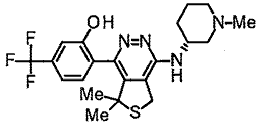 | 132 | 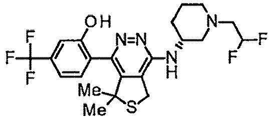 |
| 124 | 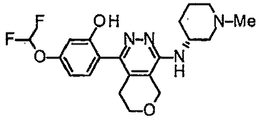 | 133 | 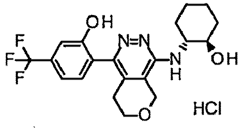 |
| 125 | 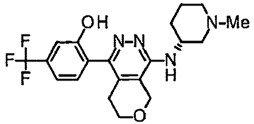 | 134 | 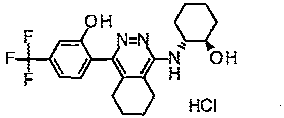 |
| 126 | 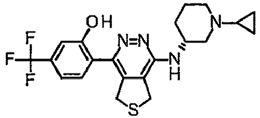 | 135 | 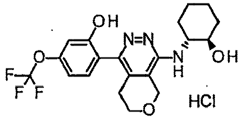 |
| 127 | 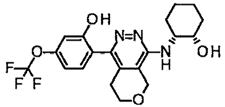 | 136 | 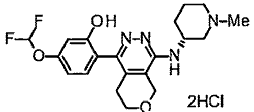 |

**[Table 5-1]**

| PEx | PSyn | DAT |
|---|---|---|
| 1 | - | ESI+; 282.2, 284.2 |
| 2 | - | ESI+; 284.2, 286.1 |
| 3 (1) | - | ESI+; 373.2, 375.2 |
| 3 (2) | - | ESI+; 373.2, 375.2 |
| 4 | - | ESI+; 284.1, 286.1 |
| 5 | - | ESI+; 298.1, 300.2 |
| 6 | 1 | FAB; 242.2 |
| 7 | 1 | ESI+; .228. 1, 230.1 |
| 8 | 1 | ESI+; 333.2, 335.2 |
| 9 | 1 | ESI+; 284.3, 286.3 |
| 10 | 1 | ESI+; 264.3, 266.3 |
| 11 | 1 | ESI+; 264.2, 266.2 |
| 12 | 1 | ESI+; 264.3, 266.3 |
| 13 | 1 | ESI+; 278.3, 280.3 |
| 14 | 1 | ESI+; 239.1, 241.1 |
| 15 | 1 | ESI+; 242.1, 244.0 |
| 16 | 1 | ESI+; 228.1, 230.1 |
| 17 | 1 | ESI+; 268.2, 270.2 |
| 18 | 1 | ESI+; 242.2, 244.2 |
| 19 | 1 | FAB; 377.1 |
| 20 | 1 | FAB; 319.0 |
| 21 | 1 | FAB; 268.0 |
| 22 | 1 | ESI+; 268.1, 270.1 |
| 23 | 1 | ESI+; 296.1, 298.2 |
| 24 | 1 | ESI+; 284.2, 286.1 |
| 25 (1) | 1 | ESI+; 284.1, 286.1 |
| 25 (2) | 1 | ESI+; 284.1, 286.1 |
| 26 | 1 | ESI+; 298.1, 300.1 |
| 27 | 1 | ESI+; 282.2, 284.2 |
| 28 | 1 | ESI+; 267.1, 269.1 |
| 29 | 1 | ESI+; 270.1, 272.1 |
| 30 | 1 | FAB; 254.0 |
| 31 | 1 | FAB; 270.0 |
| 32 | 1 | ESI+; 383.2, 385.2 |
| 33 | 1 | FAB; 305.0 |

**[Table 5-2]**

| PEx | PSyn | DAT |
|---|---|---|
| 34 | 1 | FAB; 277.0 |
| 35 | 1 | ESI+; 284.1, 286.1 |
| 36 | 1 | ESI+; 284.2, 286.2 |
| 37 | 1 | ESI+; 321.3, 323.3 |
| 38 | 1 | ESI+; 283.2, 285.2 |
| 39 | 1 | ESI+; 293.0, 295.0 |
| 40 | 1 | ESI+; 367.2, 369.2 |
| 41 | 1 | ESI+; 369.3, 371.4 |
| 42 | - | ESI+; 168.3 |
| 43 | - | ESI+; 258.3 |
| 44 | - | ESI+; 294.3, 296.3, 298.3 |
| 45 | - | ESI+; 313.2 |
| 46 | 45 | ESI+; 352.4 |
| 47 | - | ESI+; 450.3 |
| 48 | - | ESI+; 499.3 |
| 49 | 47 | ESI+; 478.4 |
| 50 | 47 | FAB; 503.6 |
| 51 | 47 | ESI+; 434.3 |
| 52 | 47 | ESI+; 470.3 |
| 53 | 47 | ESI+; 499.2 |
| 54 (1) | 47 | ESI+; 509.2 |
| 54 (2) | 47 | ESI+; 509.2 |
| 55 | 47 | ESI+; 515.2 |
| 56 | 47 | ESI+; 445.3 |
| 57 | 47 | ESI+; 472.3 |
| 58 | 47 | ESI+; 436.3 |
| 59 | 47 | ESI+; 424.4 |
| 60 | 47 | ESI+; 523.3 |
| 61 | 47 | ESI+; 493.5 |
| 62 | 47 | ESI+; 495.5 |
| 63 | - | ESI+; 520.4 |
| 64 | - | ESI+; 378.2 |
| 65 | - | ESI+; 462.3 |
| 66 | 67 | FAB; 236.9 |
| 67 | - | ESI+; 223.1, 225.1 |

**[Table 5-3]**

| PEx | PSyn | DAT |
|---|---|---|
| 68 | 69 | FAB; 241.0 |
| 69 | - | ESI+; 227.1, 229.1 |
| 70 | - | ESI+; 281.0 |
| 71 | 70 | ESI+; 295.0 |
| 72 | - | ESI+; 280.1, 282.1 |
| 73 | - | ESI+; 205.0, 207.0 |
| 74 | - | ESI+; 219.0, 221.0 |
| 75 | 73 | ESI+; 304.1 |
| 76 | - | ESI-; 259.0 |
| 77 | - | ESI+; 353.2 [M+Na]+ |
| 78 | 77 | EI; 314.2 |
| 79 | 77 | EI; 350.1 |
| 80 | 77 | ESI+; 283.2 [M+Na]+ |
| 81 | - | ESI+; 245.0 |
| 82 | 81 | EI; 302.0 |
| 83 | - | EI; 266.0 |
| 84 | - | CI; 282.9 |
| 85 | - | ESI+; 296.2, 298.2 |
| 86 | - | ESI+; 271.2 |
| 87 | - | ESI+; 182.1 |
| 88 | - | ESI+; 242.1 |
| 89 | - | ESI+; 153.0 |
| 90 | - | ESI+; 340.2 |
| 91 | - | ESI+; 251. 2 |
| 92 | - | ESI+; 355.2 |
| 93 | - | ESI+; 487.2 |
| 94 | - | FAB; 364.1 |
| 95 | 94 | FAB; 278.0 |
| 96 | - | FAB; 264.0 |
| 97 | - | FAB; 306.0 |
| 98 | - | CI; 229.1 |
| 99 | - | ESI+; 265.2 |
| 100 | - | ESI+; 284.1, 286.0 |
| 101 | - | ESI+; 409.3 |
| 102 | - | ESI+; 255.2 |

**[Table 5-4]**

| PEx | PSyn | DAT |
|---|---|---|
| 103 | - | ESI+; 155.2 |
| 104 | - | ESI+; 383.3, 385.2 |
| 105 | - | ¹H-NMR (400 MHz, DMSO-*d*₆) *δ* ppm 4.75 (4 H, s) 10.51 (2 H, br s) |
| 106 | - | ¹H-NMR (400 MHz, CDCl₃) *δ* ppm 1.53 (9 H, s) 4.77 - 4.82 (4 H, m) |

**[Table 5-5]**

| PEx | PSyn | DAT |
|---|---|---|
| 107 | 72 | ESI+; 281.1 |
| 108 | 1 | ESI+; 286.1 |
| 109 (1) | 1 | ESI+; 300.1, 302.1 |
| 109 (2) | 1 | ESI+; 300.1, 302.1 |
| 110 | 1 | ESI+; 324.2 |
| 111 | 1 | ESI+; 357.0 |
| 112 | 1 | ESI+; 369.2, 371.2 |
| 113 | 1 | ESI+; 298.2, 300.2 |
| 114 | - | ESI+; 369.3 |
| 115 | 1 | ESI+; 369.0 |
| 116 | - | ESI+; 286.1, 288.0 |
| 117 | 1 | ESI+; 371.2, 373.3 |
| 118 | 1 | ESI+; 377.3, 379.3 [M+Na]⁺ |
| 119 | 1 | ESI+; 270.1, 272.1 |
| 120 | - | ESI+; 270.1, 272.0 |
| 121 | 1 | ESI+; 421.3, 423.3 [M+Na]⁺ |
| 122 | - | ¹H NMR(400 MHz, CDCl₃) δ ppm 4.40 (4 H, s) |
| 123 | 44 | ¹H NMR(400 MHz, CDCl₃) δ ppm 1.88 (6 H, s) 4.25 (2 H, s) |
| 124 | 47 | ESI+; 540.5 |
| 125 | 47 | ESI+; 511.4 |
| 126 | 47 | ESI+; 483.3 |
| 127 | 47 | ESI+; 495.3 |
| 128 (1) | - | ESI+; 537.4 |
| 128 (2) | - | ESI+; 537.4 |
| 129 | 47 | ESI+; 425.2 |
| 130 | 47 | ESI+; 435.2 |
| 131 | 47 | ESI+; 497.3 |
| 132 | 47 | ESI+; 481.4 |
| 133 | 47 | ESI+; 422.3 |
| 134 | - | ESI+; 422.3 |
| 135 | 47 | ESI+; 525.1 |
| 136 | 64 | - |
| 137 | 64 | ESI+; 395.2 |
| 138 | 64 | ESI+; 397.2 |
| 139 | 64 | ESI+; 381.3 |

**[Table 5-6]**

| PEx | PSyn | DAT |
|---|---|---|
| 140 | 64 | ESI+; 425.2 |
| 141 | 72 | ESI+; 297.2, 299.2 |
| 142 | 73 | ESI+; 221.1 |
| 143 | 73 | ESI+; 244.8 |
| 144 | - | ESI+; 219.0, 221.0 |
| 145 | 77 | ¹H NMR (400 MHz, CDCl₃) δ ppm 0.68 - 0.74 (2 H, m) 0.95 - 0.98 (2 H, m) 1. 33 (12 H, s) 1.83 - 1.87 (1 H, m) 3.52 (3 H, s) 5.18 (2 H, s) 6.66 - 6.69 (1 H, m) 6.76 (1 H, d, *J*=1.2 Hz) 7. 58 (1 H, d, *J*=7. 6 Hz) |
| 146 | 81 | ¹H NMR (400 MHz, CDCl₃) δ ppm 0.65 - 0.68 (2 H, m) 0.94 - 0.99 (2 H, m) 1.80 - 1.88 (1 H, m) 3.52 (3 H, s) 5.23 (2 H, s) 6.52 - 6.57 (1 H, m) 6.90 (1 H, d, *J*=2.0 Hz) 7.38 (1 H, d, *J*=8.3 Hz) |
| 147 | 90 | ESI+; 365.3 |
| 148 | 90 | ESI+; 251.2 |
| 149 | 90 | ESI+; 336.2 |
| 150 | 92 | ESI+; 380.3 |
| 151 | 92 | ESI+; 351.2 |
| 152 | - | ESI+; 512.3 |
| 153 | 152 | ESI+; 483.3 |
| 154 | - | ESI+; 357.3 [M+Na]⁺ |
| 155 | 96 | ESI+; 269.1 |
| 156 | 98 | - |
| 157 | 99 | ESI+; 265.2 |
| 158 | 100 | ESI+; 284.1, 286.1 |
| 159 | - | ESI+; 230.2 |
| 160 | - | ESI+; 393.3 |
| 161 | 160 | ESI+; 393.5 |
| 162 | - | ESI+; 298.1, 300.1 |
| 163 | - | ESI+; 283.1 |
| 164 | - | ¹H NMR (400 MHz, CDCl₃) δ ppm 0.65 - 0.68 (2 H, m) 0.94 - 0.98 (2 H, m) 1.79 - 1.85 (1 H, m) 5.41 (1 H, s) 6.53 - 6.55 (1 H, m) 6.71 (1 H, d, *J*=2.0 Hz) 7.30 (1 H, d, *J*=8.4 Hz) |
| 165 | - | ¹H NMR (400 MHz, DMSO-*d*₆) δ ppm 4.10 (4 H, s) 11. 26 (1 H, br s) 11.97 (1 H, br s) |
| 166 | - | ¹H NMR (400 MHz, DMSO-*d*₆) δ ppm 4.05 (4 H, s) 6.43 (6 H, br s) |

**[Table 5-7]**

| PEx | PSyn | DAT |
|---|---|---|
| 167 | - | ¹H NMR (400 MHz, CDCl₃) δ ppm 1. 85 (6 H, s) 4.23 (2 H, s) |
| 168 | - | ¹H NMR (400 MHz, CDCl₃) δ ppm 1.29 (3 H, t, *J*=7.1 Hz) 1.49 (9 H, s) 1.70 (6 H, s) 3.97 (2 H, s) 4.22 (2 H, q, *J*=7.1 Hz) 6.54 (1 H, br s) 7.33 (1 H, br d, *J*= 2.3 Hz) |
| 169 | - | ¹H NMR (400 MHz, CDCl₃) δ ppm 1.31 (3 H, t, *J*=7.1 Hz) 1.68 (6 H, s) 4.00 (2 H, s) 4.26 (2 H, q, *J*=7.2 Hz) |

**[Table 6-1]**

| Ex | Syn | DAT |
|---|---|---|
| 1 | - | ESI+; 408.2 |
| | | ¹H NMR (500 MHz, DMSO-*d*₆) δ ppm 1. 14 - 1. 35 (4 H, m) 1. 56 - 1.72 (4 H, m) 1.72 - 1.82 (2 H, m) 1.88 - 1.98 (1 H, m) 2.06 - 2. 16 (1 H, m) 2.29 - 2.48 (4 H, m) 3.46 - 3.54 (1 H, m) 3.85 - 3.93 (1 H, m) 4.79 (1 H, d, *J*=4.4 Hz) 5.55 (1 H, d, *J*=6.9 Hz) 7.17 - 7.23 (2 H, m) 7.34 (1 H, d, *J*=7.7 Hz) 10.39 (1 H, br s) |
| 2(1) | - | ESI+; 410.2 |
| | | ¹H NMR (500 MHz, DMSO-*d*₆) δ ppm 1. 14 - 1. 35 (4 H, m) 1. 58 - 1. 72 (2 H, m) 1.87 - 1.99 (1 H, m) 2.01 - 2.10 (1 H, m) 2.34 - 2.47 (2 H, m) 3.43 - 3.53 (1 H, m) 3.72 - 3.83 (2 H, m) 3.90 - 4.01 (1 H, m) 4.48 (1 H, d, *J*=16.9 Hz) 4.53 (1 H, d, *J*=16.7 Hz) 4.71 (1 H, d, *J*=4.4 Hz) 5.61 (1 H, d, *J*=7.5 Hz) 7.17 - 7.26 (2 H, m) 7.40 (1 H, d, *J*=7.8 Hz) 10.53 (1 H, br s) |
| 2(2) | - | ESI+; 410.2 |
| 3(1) | - | ESI+; 426.2 |
| | | ¹H NMR (500 MHz, DMSO-*d*₆) δ ppm 1. 13 - 1. 35 (4 H, m) 1. 56 - 1.75 (2 H, m) 1.86 - 1.98 (1 H, m) 2.00 - 2. 11 (1 H, m) 2.33 - 2.48 (2 H, m) 3.42 - 3.53 (1 H, m) 3.70 - 3.84 (2 H, m) 3.89 - 4.00 (1 H, m) 4.47 (1 H, d, *J*=16.8 Hz) 4.52 (1 H, d, *J*=16.9 Hz) 4.71 (1 H, d, *J*=4.4 Hz) 5.57 (1 H, d, *J*=7.5 Hz) 6.83 - 6.90 (2 H, m) 7.26 - 7.32 (1 H, m) 10.50 (1 H, br s) |
| 3 (2) | - | ESI+; 426.2 |
| 4(1) | - | ESI+; 410.2 |
| | | ¹H NMR (500 MHz, DMSO-*d*₆) δ ppm 1.21 - 1.41 (2 H, m) 1.44 - 1.84 (6 H, m) 2.37 - 2.48 (2 H, m) 3.70 - 3.85 (2 H, m) 3.92 - 4.02 (1 H, m) 4.06 - 4.16 (1 H, m) 4.48 - 4.63 (2 H, m) 4.74 (1 H, br s) 5.69 (1 H, br s) 7.19 - 7.29 (2 H, m) 7.42 (1 H, d, *J*=7.8 Hz) 10.61 (1 H, br s) |
| 4(2) | - | ESI+; 410.3 |
| 5 | - | ESI+; 424.3 |
| | | ¹H NMR (500 MHz, CDCl₃) δ ppm 1.17 - 1.52 (4 H, m) 1.72 - 1.84 (2 H, m) 2.09 - 2.24 (2 H, m) 2.84 - 2.94 (2 H, m) 3.04 - 3.14 (2 H, m) 3.44 - 3.51 (1 H, m) 3.64 (1 H, s) 3.75 - 3.97 (4 H, m) 3.97 - 4.09 (1 H, m) 4.48 (1 H, d, *J*=6.6 Hz) 7.12 - 7.21 (2 H, m) 7.29 (1 H, s) 10.59 (1 H, br s) |

**[Table 6-2]**

| Ex | Syn | DAT |
|---|---|---|
| 6 | 1 | ESI+; 298.1 |
| 7 | 1 | FAB; 314.3 |
| 8 | 1 | ESI+; 406.3 |
| 9 | 1 | ESI+; 511.2 |
| 10 | 1 | ESI+; 439.3 |
| 11 | 1 | ESI+; 410.3 |
| 12 | 1 | ESI+; 390.3 |
| 13 | 1 | ESI+; 390.4 |
| 14 | 1 | ESI+; 390.3 |
| 15 | 1 | ESI+; 404.2 |
| 16 (1) | 1 | FAB; 365.1 |
| 16 (2) | 1 | FAB; 365.0 |
| 17 | 1 | ESI+; 368.1 |
| 18 | 1 | ESI+; 354.2 |
| 19 | 1 | ESI+; 394.3 |
| 20 | 1 | FAB; 367.2 |
| 21 | 1 | ESI+; 368.2 |
| 22 | 1 | ESI+; 353.2 |
| 23 | 1 | FAB; 445.2 |
| 24 | 1 | FAB; 394.2 |
| 25 | 1 | ESI+; 374.2, 376.1 |
| 26 | 1 | ESI+; 354.2 |
| 27 | 1 | ESI+; 424.2 |
| 28 | 1 | ESI+; 370.3 |
| 29 | 1 | ESI+; 394.2 |
| 30 | 1 | ESI+; 360.2, 362.1 |
| 31 | 1 | ESI+; 340.2 |
| 32 | 1 | ESI+; 410.2 |
| 33 | 1 | ESI+; 356.2 |
| 34 | 1 | ESI+; 422.3 |
| 35 | 1 | ESI+; 410.2 |
| 36 | 1 | ESI+; 356.2 |

**[Table 6-3]**

| Ex | Syn | DAT |
|---|---|---|
| 37 | 1 | ESI+; 426.2 |
| 38 | 1 | ESI+; 372.2 |
| 39 | 1 | ESI+; 376.2, 378.2 |
| 40 | 1 | ESI+; 406.3 |
| 41 | 1 | ESI+; 410.3 |
| 42 | 1 | ESI+; 410.2 |
| 43 | 1 | ESI+; 438.3 |
| 44 | 1 | ESI+; 424.2 |
| 45 | 1 | ESI+; 440.2 |
| 46 | 1 | ESI+; 370.3 |
| 47 | 1 | ESI+; 384.3 |
| 48 | 1 | ESI+; 408.2 |
| 49 | 1 | ESI+; 386.3 |
| 50 | 1 | ESI+; 393.2 |
| 51 | 1 | ESI+; 380.3 |
| 52 (1) | 1 | ESI+; 382.3 |
| 52 (2) | 1 | ESI+; 382.3 |
| 53 | 1 | ESI+; 422.3 |
| 54 | 1 | ESI+; 438.2 |
| 55 | 1 | ESI+; 368.3 |
| 56 (1) | 1 | ESI+; 396.2 |
| 56 (2) | 1 | ESI+; 396.2 |
| 57 (1) | 1 | ESI+; 412.2 |
| 57 (2) | 1 | ESI+; 412.2 |
| 58 | 1 | ESI+; 384.3 |
| 59 | 1 | FAB; 380.4 |
| 60 | 1 | ESI+; 395.8 |
| 61 | 1 | ESI+; 342.2 |

**[Table 6-4]**

| Ex | Syn | DAT |
|---|---|---|
| 62 (1) | 1 | ESI+; 374.2 |
| 62 (2) | 1 | ESI+; 374.2 |
| 63 | 1 | ESI+; 430.9 |
| 64 | 1 | ESI+; 402.9 |
| 65 | 1 | FAB; 432.2 |
| 66 (1) | 1 | ESI+; 410.2 |
| 66 (2) | 1 | ESI+; 410.3 |
| 67 | 1 | FAB; 404.2 |
| 68 | 1 | ESI+; 440.3 |
| 69 | 1 | ESI+; 372.3 |
| 70 | - | ESI+; 376.2, 378.2 |
| | | ¹H NMR (500 MHz, DMSO-*d*₆) δ ppm 1. 14 - 1.34 (4 H, m) 1.60 - 1.72 (2 H, m) 1.89 - 1.96 (1 H, m) 2.02 - 2.09 (1 H, m) 2.37 - 2.47 (2 H, m) 3.42 - 3.52 (1 H, m) 3.72 - 3.81 (2 H, m) 3.89 - 3.99 (1 H, m) 4.47 (1 H, d, *J*=16.8 Hz) 4.52 (1 H, d, *J*=16.9 Hz) 4.71 (1 H, d, *J*=4.4 Hz) 5. 56 (1 H, d, *J*=7.3 Hz) 6.91 - 6.96 (2 H, m) 7.19 (1 H, d, *J*=8.3 Hz) 10.38 (1 H, br s) |
| 71 | 1 | ESI+; 447.5 |
| 72 (1) | 1 | ESI+; 419.3 |
| 72 (2) | 1 | ESI+; 419.3 |
| 73 | - | ESI+; 423.3 |
| | | ¹H NMR (500 MHz, DMSO-*d*₆) δ ppm 1.11 - 1.41 (4 H, m) 1.59 - 1.74 (2 H, m) 1.89 - 1.98 (1 H, m) 2.04 - 2. 13 (1 H, m) 2.39 (3 H, s) 2.42 - 2.58 (2 H, m) 3.20 - 3.40 (4 H, m) 3.44 - 3.54 (1 H, m) 3.89 - 3.99 (1 H, m) 4.72 (1 H, d, *J*=4. 7 Hz) 5.62 (1 H, d, *J*=7.3 Hz) 7.18 - 7.23 (2 H, m) 7. 37 (1 H, d, *J*=7.8 Hz) 10.47 (1 H, br s) |
| 74 | 73 | ESI+; 435.4 |
| 75 | 73 | ESI+; 423.2 |
| 76 | 73 | ESI+; 439.3 |
| 77 | 73 | ESI+; 369.3 |
| 78 | 73 | ESI+; 437.3 |
| 79 | - | ESI+; 463.4 |

**[Table 6-5]**

| Ex | Syn | DAT |
|---|---|---|
| 80 | - | ESI+; 499.3 |
| 81 | - | ESI+; 420.3 |
| 82 | - | FAB; 403.1 |
| 83 | - | ESI+; 406.3 |
| | | ¹H NMR (500 MHz, DMSO-*d*₆) δ ppm 1.13 - 1.38 (4 H, m) 1.52 - 1.83 (6 H, m) 1.86 - 2.00 (1 H, m) 2. 05 - 2. 17 (1 H, m) 2. 28 - 2.47 (4 H, m) 3.43 - 3.55 (1 H, m) 3.80 - 3.93 (1 H, m) 4.81 (1 H, d, *J*=4.6 Hz) 5.48 (1 H, d, *J*=6.7 Hz) 6.61 - 6.73 (2 H, m) 7.13 - 7.18 (1 H, m) 7.23 (1 H, t, *J*=74.2 Hz) 10.20 (1 H, br s) |
| 84 | 83 | ESI+; 390.3 |
| 85 | 83 | ESI+; 426.2 |
| 86 | 83 | ESI+; 428.3 |
| 87 | 83 | ESI+; 392.2 |
| 88 | - | ESI+; 408.2 |
| | | ¹H NMR (500 MHz, DMSO-*d*₆) δ ppm 1. 13 - 1.35 (4 H, m) 1.59 - 1.73 (2 H, m) 1.87 - 1.98 (1 H, m) 2.01 - 2.11 (1 H, m) 2.38 - 2.47 (2 H, m) 3.41 - 3. 52 (1 H, m) 3. 71 - 3.82 (2 H, m) 3.89 - 3.99 (1 H, m) 4.47 (1 H, d, *J*=16.7 Hz) 4.52 (1 H, d, *J*=16.6 Hz) 4.72 (1 H, d, *J*=4.4 Hz) 5.55 (1 H, d, *J*=7.3 Hz) 6.66 - 6.73 (2 H, m) 7.20 - 7.25 (1 H, m) 7.24 (1 H, t, *J*=74.1 Hz) 10.36 (1 H, br s) |
| 89 | 88 | ESI+; 420.3 |
| 90 | - | ESI+; 409.2 |
| 91 | 90 | ESI+; 409.2 |
| 92 | - | ESI+; 451.3 |
| 93 | - | ESI+; 487.3 |
| 94 | - | ESI+; 410.2 |
| 95 | - | ESI+; 409.2 |
| 96 | - | ESI+; 393.3 |
| 97 | - | ESI+; 449.4 |
| 98 | - | ESI+; 395.3 |
| 99 | - | ESI+; 409.3 |

**[Table 6-6]**

| Ex | Syn | DAT |
|---|---|---|
| 100 | 1 | ESI+; 407.2 |
| 101 (1) | 1 | ESI+; 412.4 |
| 101 (2) | 1 | ESI+; 412.1 |
| 102 | 1 | ESI+; 426.3 |
| 103 | 1 | ESI+; 426.3 |
| 104 | 1 | ESI+; 410.3 |
| 105 | 1 | ESI+; 423.3 |
| 106 | 1 | ESI+; 426.3 |
| 107 | 1 | ESI+; 450.2 |
| 108 (1) | 1 | ESI+; 424.3 |
| 108 (2) | 1 | ESI+; 424.3 |
| 109 | 1 | ESI+; 375.1, 377.0 |
| 110 | 1 | ESI+; 371.1 |
| 111 | - | ESI+; 412.1 |
| | | ¹H NMR (400 MHz, CDCl₃) δ ppm 1.27 - 1.50 (4 H, m) 1.78 - 1.84 (2 H, m) 2. 15 - 2.28 (2 H, m) 3.52 (1 H, dt, *J*=4.1, 10.1 Hz) 4.06 - 4.15 (3 H, m) 4.35 (1 H, d, *J*=7.3 Hz) 4.60 (2 H, t, *J*=3.2 Hz) 7. 14 (1 H, d, *J*=8.7 Hz) 7.35 (1 H, s) 7.57 (1 H, d, *J*=8.3 Hz) |
| 112 | - | ESI+; 381.3 |
| 113 | 112 | ESI+; 391.2 |
| 114 | - | ESI+; 378.1 |
| | | ¹H NMR (400 MHz, CDCl₃) δ ppm 1.48 - 1. 53 (2 H, m) 1.60 - 1.68 (3 H, m) 1.85 - 1.95 (3 H, m) 2.21 - 2.24 (1 H, m) 4. 07 - 4.10 (1 H, m) 4.41 - 4.49 (1 H, m) 5.01 -5.08 (3 H, m) 5.47 - 5.48 (2 H, m) 6.62 (1 H, t, *J*=56.5 Hz) 7.05 (1 H, d, *J*=7.8 Hz) 7.15 (1 H, d, *J*=8.3 Hz) 7.22 (1 H, s) |
| 115 | 114 | ESI+; 378.2 |
| 116 | 88 | ESI+; 422.5 |
| 117 | - | ESI+; 437.4 |

**[Table 6-7]**

| Ex | Syn | DAT |
|---|---|---|
| 118 | 117 | ESI+; 447.0 [M+Na]⁺ |
| 119 | 117 | ESI+; 439.2 |
| 120 | 124 | ESI+; 407.3 |
| 121 | 97 | ESI+; 411.3 |
| 122 | 97 | ESI+; 395.3 |
| 123 | 97 | ESI+; 439.3 |
| 124 | - | ESI+; 407.3 |
| | | ¹H NMR (500 MHz, DMSO-*d*₆) δ ppm 1.21 - 1. 41 (1 H, m) 1.49 - 1. 61 (1 H, m) 1. 65 - 1.74 (1 H, m) 1.79 - 1.94 (3 H, m) 2. 18 (3 H, s) 2.40 - 2.46 (2 H, m) 2.60 - 2.69 (1 H, m) 2.90 - 2.99 (1 H, m) 3.76 (2 H, t, *J*=5.6 Hz) 4. 19 - 4.28 (1 H, m) 4.46 (2 H, s) 5.57 (1 H, d, *J*=7.6 Hz) 6.66 - 6. 73 (2 H, m) 7.08 - 7.40 (2 H, m) 10.31 (1 H, br s) |
| 125 | 97 | ESI+; 409.3 |
| 126 | - | ESI+; 437.2 |
| 127 | - | ESI+; 426.3 |
| 128 | - | ESI+; 425.3 |
| 129 | - | ESI+; 458.1 |
| 130 | 129 | ESI+; 458.1 |
| 131 (1) | 129 | ESI+; 444.2 |
| 131 (2) | 129 | ESI+; 428.2 |
| 132 | - | ESI+; 489.4 |

**[Table 6-8]**

| Ex | Syn | DAT |
|---|---|---|
| 133 | - | ESI+; 410.5 |
| | | ¹H NMR (400 MHz, DMSO-*d*₆) δ ppm 1. 13 - 1. 39 (3 H, m) 1. 40 - 1. 55 (1 H, m) 1.62 - 1.77 (2 H, m) 1.84 - 2.00 (2 H, m) 2.40 - 2.63 (2 H, m) 3.46 - 3.60 (1 H, m) 3.77 - 3. 91 (3 H, m) 4.63 (1 H, d, *J*=17.7 Hz) 4. 74 (1 H, d, *J*=17.7 Hz) 5. 14 (1 H, br s) 7. 29 - 7.37 (2 H, m) 7.50 (1 H, d, *J*=7.3 Hz) 7.92 (1 H, br s) 11. 06 (1 H, br s) 14. 74 (1 H, br s) 2 *θ* (° )=6.0, 7.0, 10.1, 10. 5, 13. 5, 15.2, 17.4, 21.1, 22.2, 27.2 DSC¹; 184°C |
| 134 | - | ESI+; 408.3 |
| | | ¹H NMR (500 MHz, DMSO-*d*₆) δ ppm 1. 14 - 1.39 (3 H, m) 1.43 - 1. 59 (1 H, m) 1.61 - 1.75 (4 H, m) 1.75 - 1.85 (2 H, m) 1.85 --- 2.00 (2 H, m) 2.34 - 2.48 (2 H, m) 2.48 - 2.70 (2 H, m) 3.51 - 3.62 (1 H, m) 3.77 - 3.88 (1 H, m) 5.08 (1 H, br s) 7.27 - 7.36 (2 H, m) 7.46 (1 H, d, *J*=7.3 Hz) 7.92 (1 H, br s) 10.92 (1 H, br s) 14.44 (1 H, br s) DSC²;161°C |
| 135 | - | ESI+; 426.3 |
| | | ¹H NMR (500 MHz, DMSO-*d*₆) δ ppm 1. 17 - 1.37 (3 H, m) 1.39 - 1. 52 (1 H, m) 1.64 - 1.75 (2 H, m) 1.86 - 2.00 (2 H, m) 2.45 - 2.62 (2 H, m) 3.46 - 3. 59 (1 H, m) 3. 74 - 3.94 (3 H, m) 4. 62 (1 H, d, *J*=17.6 Hz) 4.72 (1 H, d, *J*=17.6 Hz) 5. 10 (1 H, br s) 6.94 - 6.99 (1 H, m) 6.99 (1 H, s) 7.40 (1 H, d, *J*=8.4 Hz) 7.84 (1 H, br s) 10.97 (1 H, br s) 14.70 (1H, br s) 2 *θ* (° ) =5. 7, 6.5, 7.4, 9.7, 10. 3, 11. 5, 12. 7, 16.0, 16.9, 17.6 DSC¹;165°C |
| 136 | - | ESI+; 407.3 |
| | | ¹H NMR (500 MHz, METHANOL-*d*₄) δ ppm 1.69 - 1.81 (1 H, m) 1.87 - 2.00 (1 H, m) 2.09 - 2.18 (1 H, m) 2. 18 - 2.31 (1 H, m) 2.69 - 2.77 (2 H, m) 2.83 - 3.05 (5 H, m) 3.49 - 3.59 (1 H, m) 3.74 - 3.82 (1 H, m) 3.90 - 4.00 (2 H, m) 4.43 - 4.56 (1 H, m) 4.73 (2 H, s) 6.81 (1 H, d, *J*=2.3 Hz) 6.86 (1 H, dd, *J*=8.5, 2.2 Hz) 6.94 (1 H, t, *J*=73.3 Hz) 7.38 - 7.44 (1 H, m) DSC²; 121°C |

### INDUSTRIAL APPLICABILITY

A compound of formula (I) or a salt thereof has an inhibitory effect on NLRP3 inflammasome activation, which is expected to be used as a prophylactic and/or therapeutic agent for inflammatory diseases and/or neurodegenerative diseases.

## Claims

1. A compound of formula (I): 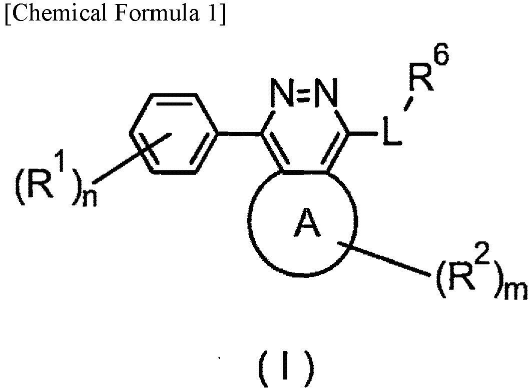
or a salt thereof, wherein
ring A is C₅₋₈ cycloalkenyl, 5- to 11-membered partially unsaturated heterocyclyl, aryl, or heteroaryl;
R¹, which are identical or different from each other, are OH, C₁₋₆ alkyl, halogeno C₁₋₆ alkyl, C₃₋₈ cycloalkyl, halogen, cyano, -O-C₁₋₆ alkyl, -O-halogeno C₁₋₆ alkyl, or -O-C₃₋₈ cycloalkyl; R², which are identical or different from each other, are C₁₋₆ alkyl, -C₁₋₆ alkylene-aryl, C₃₋₈ cycloalkyl, halogen, -O-C₁₋₆ alkyl, -O-halogeno C₁₋₆ alkyl, oxo, -C(O)-C₁₋₆ alkyl, or -S(O)₂-C₁₋₆ alkyl;
L is -NR³-, -O-, or -CR⁴R⁵-;
R³ is H or C₁₋₆ alkyl;
R⁴ and R⁵, which are identical or different from each other, are H or C₁₋₆ alkyl;
R⁶ is C₁₋₆ alkyl substituted with one to four identical or different R⁷,
-C₁₋₆ alkylene-(C₃₋₈ cycloalkyl optionally substituted with one to four identical or different R⁸), -C₁₋₆ alkylene-(4- to 7-membered saturated heterocyclyl optionally substituted with one to four identical or different R⁹), C₃₋₈ cycloalkyl optionally substituted with one to four identical or different R¹⁰, or 4- to 7-membered saturated heterocyclyl optionally substituted with one to four identical or different R¹¹;
R⁷ is -OR¹², -NR¹³R¹⁴, halogen, or cyano;
R⁸ and R¹⁰ are C₁₋₆ alkyl, halogeno C₁₋₆ alkyl, C₃₋₈ cycloalkyl, -OR¹², -NR¹³R¹⁴,
-C₁₋₆ alkylene-OR¹², -C₁₋₆ alkylene-NR¹³R¹⁴, halogen, or cyano;
R⁹ and R¹¹ are C₁₋₆ alkyl, halogeno C₁₋₆ alkyl, C₃₋₈ cycloalkyl, 4- to 7-membered saturated heterocyclyl, -OR¹², -NR¹³R¹⁴, -C₁₋₆ alkylene-OR¹², -C₁₋₆ alkylene-NR¹³R¹⁴, halogen, cyano, oxo, -C(O)-C₁₋₆ alkyl, or -S(O)₂-C₁₋₆ alkyl;
R¹² is H or C₁₋₆ alkyl;
R¹³ and R¹⁴, which are identical or different from each other, are H, C₁₋₆ alkyl, or
-C(O)-C₁₋₆ alkyl;
n is an integer of 1 to 4 and represents the number of substituents R¹; and
m is an integer of 0 to 3 and represents the number of substituents R²,
provided that when ring A is aryl or heteroaryl, formula (I) is formula (Ia): 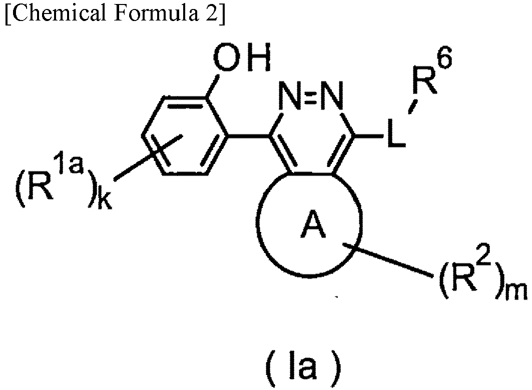
R^{1a}, which are identical or different from each other, are C₁₋₆ alkyl, halogeno C₁₋₆ alkyl, C₃₋₈ cycloalkyl, halogen, cyano, -O-C₁₋₆ alkyl, -O-halogeno C₁₋₆ alkyl, or -O-C₃₋₈ cycloalkyl; and
k is an integer of 0 to 3 and represents the number of substituents R^{1a}.

2. The compound or a salt thereof according to claim 1, wherein
ring A is C₅₋₈ cycloalkenyl, 5- to 8-membered partially unsaturated heterocyclyl, aryl, or heteroaryl; and
R², which are identical or different from each other, are C₁₋₆ alkyl, -C₁₋₆ alkylene-aryl, C₃₋₈ cycloalkyl, halogen, -O-C₁₋₆ alkyl, -O-halogeno C₁₋₆ alkyl, -C(O)-C₁₋₆ alkyl, or -S(O)₂-C₁₋₆ alkyl.

3. The compound or a salt thereof according to claim 2, wherein formula (I) is formula (Ia).

4. The compound or a salt thereof according to claim 3, wherein ring A is C₅₋₈ cycloalkenyl or 5- to 8-membered partially unsaturated heterocyclyl.

5. The compound or a salt thereof according to claim 1, wherein
formula (I) is formula (Ia);
R^{1a}, which are identical or different from each other, are C₁₋₆ alkyl, halogeno C₁₋₆ alkyl, C₃₋₈ cycloalkyl, halogen, -O-C₁₋₆ alkyl, or -O-halogeno C₁₋₆ alkyl;
R², which are identical or different from each other, are C₁₋₆ alkyl, oxo, -C(O)-C₁₋₆ alkyl, or -S(O)₂-C₁₋₆ alkyl;
L is -NR³-, -O-, or -CR⁴R⁵-;
R³ is H;
R⁴ and R⁵ are H;
R⁶ is C₁₋₆ alkyl substituted with one R⁷, -C₁₋₆ alkylene-(C₃₋₈ cycloalkyl optionally substituted with one R⁸), -C₁₋₆ alkylene-(4- to 7-membered saturated heterocyclyl), C₃₋₈ cycloalkyl optionally substituted with one or two identical or different R¹⁰, or 4- to 7-membered saturated heterocyclyl optionally substituted with one or two identical or different R¹¹;
R⁷ is -OR¹² or -NR¹³R¹⁴;
R⁸ and R¹⁰ are C₁₋₆ alkyl, -OR¹², -NR¹³R¹⁴, -C₁₋₆ alkylene-OR¹², halogen, or cyano;
R⁹ and R¹¹ are C₁₋₆ alkyl, halogeno C₁₋₆ alkyl, C₃₋₈ cycloalkyl, 4- to 7-membered saturated heterocyclyl, -OR¹², oxo, or -C(O)-C₁₋₆ alkyl;
R¹² is H;
R¹³ and R¹⁴ are each H, or R¹³ is -C(O)-C₁₋₆ alkyl, and R¹⁴ is H;
k is an integer of 0 to 2 and represents the number of substituents R^{1a}; and
m is an integer of 0 to 2 and represents the number of substituents R².

6. The compound or a salt thereof according to claim 5, wherein
ring A is C₅₋₈ cycloalkenyl or 5- to 11-membered partially unsaturated heterocyclyl;
L is -NR³-;
R⁶ is C₁₋₆ alkyl substituted with one R⁷, C₃₋₈ cycloalkyl optionally substituted with one or two identical or different R¹⁰, or 4- to 7-membered saturated heterocyclyl optionally substituted with one or two identical or different R¹¹;
R¹⁰ is C₁₋₆ alkyl, -OR¹², -NR¹³R¹⁴, -C₁₋₆ alkylene-OR¹², halogen, or cyano; and
R¹¹ is C₁₋₆ alkyl, halogeno C₁₋₆ alkyl, C₃₋₈ cycloalkyl, 4- to 7-membered saturated heterocyclyl, -OR¹², oxo, or -C(O)-C₁₋₆ alkyl.

7. The compound or a salt thereof according to claim 6, wherein
ring A is C₅₋₈ cycloalkenyl or 5- to 8-membered partially unsaturated heterocyclyl;
R^{1a}, which are identical or different from each other, are halogeno C₁₋₆ alkyl, halogen, or -O-halogeno C₁₋₆ alkyl;
R² is C₁₋₆ alkyl;
R⁶ is C₃₋₈ cycloalkyl optionally substituted with one or two identical or different R¹⁰ or 4- to 7-membered saturated heterocyclyl optionally substituted with one or two identical or different R¹¹;
R¹⁰ is C₁₋₆ alkyl or -OR¹²;
R¹¹ is C₁₋₆ alkyl or -OR¹²;
k is 1 or 2 and represents the number of substituents R^{1a}; and
m is 0 or 1 and represents the number of substituents R².

8. The compound or a salt thereof according to claim 1, wherein
formula (I) is formula (Ia);
ring A is 5- to 11-membered partially unsaturated heterocyclyl;
R^{1a}, which are identical or different from each other, are C₁₋₆ alkyl, halogeno C₁₋₆ alkyl, C₃₋₈ cycloalkyl, halogen, -O-C₁₋₆ alkyl, or -O-halogeno C₁₋₆ alkyl;
R², which are identical or different from each other, are C₁₋₆ alkyl, oxo, -C(O)-C₁₋₆ alkyl, or -S(O)₂-C₁₋₆ alkyl;
L is -NR³-;
R³ is H;
R⁶ is C₁₋₆ alkyl substituted with one R⁷, C₃₋₈ cycloalkyl optionally substituted with one or two identical or different R¹⁰, or 4- to 7-membered saturated heterocyclyl optionally substituted with one or two identical or different R¹¹;
R⁷ is -OR¹² or -NR¹³R¹⁴;
R¹⁰ is C₁₋₆ alkyl, -OR¹², -NR¹³R¹⁴, -C₁₋₆ alkylene-OR¹², halogen, or cyano; and
R¹¹ is C₁₋₆ alkyl, halogeno C₁₋₆ alkyl, C₃₋₈ cycloalkyl, 4- to 7-membered saturated heterocyclyl, -OR¹², oxo, or -C(O)-C₁₋₆ alkyl;
R¹² is H;
R¹³ and R¹⁴ are each H, or R¹³ is -C(O)-C₁₋₆ alkyl, and R¹⁴ is H;
k is an integer of 0 to 2 and represents the number of substituents R^{1a}; and
m is an integer of 0 to 2 and represents the number of substituents R².

9. The compound or a salt thereof according to claim 8, wherein
formula (I) is formula (If), (Ig), (Ih), (Ii), (Ij), (Iu), (Iv), (Iw), or (Iy): 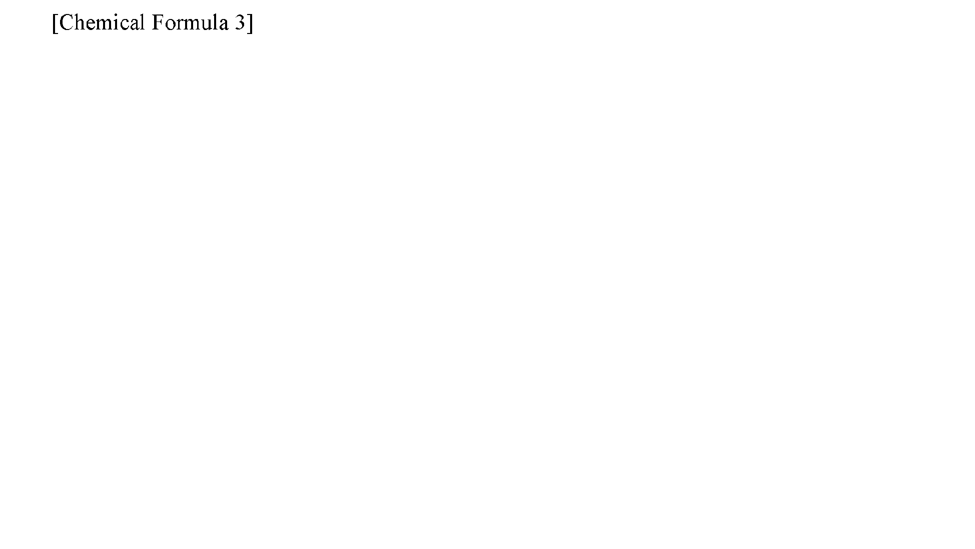 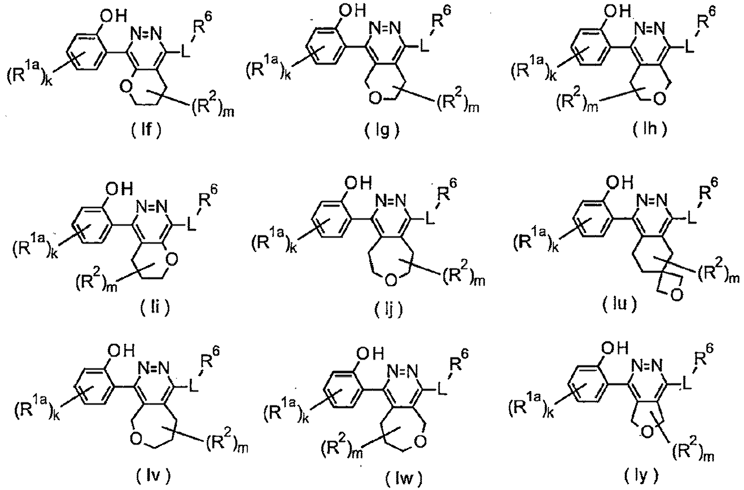
R¹¹ is C₁₋₆ alkyl or -OR¹²; and
m is 0.

10. The compound or a salt thereof according to claim 9, wherein
formula (I) is formula (Ih);
R¹⁰ is C₁₋₆ alkyl, -OR¹², or cyano;
R¹³ is -C(O)-C₁₋₆ alkyl, and R¹⁴ is H.

11. The compound or a salt thereof according to claim 1, wherein the compound is selected from the group consisting of:
2-(4-{[(1R,2R)-2-hydroxycyclohexyl]amino}-5,6,7,8-tetrahydrophthalazin-1-yl)-5-(trifluoro methyl)phenol;
2-(4-{[(1R,2R)-2-hydroxycyclohexyl]amino}-7,8-dihydro-5H-pyrano[3,4-d]pyridazin-1-yl)-5 -(trifluoromethyl)phenol;
2-(4-{[(1R,2R)-2-hydroxycyclohexyl]amino}-7,8-dihydro-5H-pyrano[3,4-d]pyridazin-1-yl)-5 -(trifluoromethoxy)phenol;
2-(4-{[(1R,2S)-2-hydroxycyclohexyl]amino}-7,8-dihydro-5H-pyrano[3,4-d]pyridazin-1-yl)-5 -(trifluoromethyl)phenol;
2-(4-{[(1R,2R)-2-hydroxycyclohexyl]amino}-5,6,8,9-tetrahydrooxepino[4,5-d]pyridazin-1-yl )-5-(trifluoromethyl)phenol;
5-chloro-2-(4-{[(1R,2R)-2-hydroxycyclohexyl]amino}-7,8-dihydro-5H-pyrano[3,4-d]pyridaz in-1-yl)phenol;
2-(4-{[(1R,2R)-2-hydroxycyclohexyl]amino}-6-methyl-5,6,7,8-tetrahydropyrido[3,4-d]pyrid azin-1-yl)-5-(trifluoromethyl)phenol;
5-(difluoromethoxy)-2-(4-{[(1R,2R)-2-hydroxycyclohexyl]amino}-5,6,7,8-tetrahydrophthala zin-1-yl)phenol;
5-(difluoromethoxy)-2-(4-{[(1R,2R)-2-hydroxycyclohexyl]amino}-7,8-dihydro-5H-pyrano[3 ,4-d]pyridazin-1-yl)phenol;
5-(difluoromethoxy)-2-(4-{ [(3 R)-1-methylpiperidin-3-yl] amino}-7, 8-dihydro-SH-pyrano[3,4 -d]pyridazin-1-yl)phenol;
2-(4-{[(1R,2R)-2-hydroxycyclohexyl]amino}-5,7-dihydrothieno[3,4-d]pyridazin-1-yl)-5-(trif luoromethyl)phenol; and
5-(difluoromethyl)-2-(4-{[(1R,3S)-3-hydroxycyclohexyl]amino}-5,7-dihydrofuro[3,4-d]pyrid azin-1-yl)phenol.

12. The compound or a salt thereof according to claim 1, wherein the compound is selected from the group consisting of:
2-(4-{[(1R,2R)-2-hydroxycyclohexyl]amino}-7,8-dihydro-5H-pyrano[3,4-d]pyridazin-1-yl)-5 -(trifluoromethyl)phenol;
2-(4-{[(1R,2R)-2-hydroxycyclohexyl]amino}-7,8-dihydro-5H-pyrano[3,4-d]pyridazin-1-yl)-5 -(trifluoromethoxy)phenol;
2-(4-{[(1R,2S)-2-hydroxycyclohexyl]amino}-7,8-dihydro-5H-pyrano[3,4-d]pyridazin-1-yl)-5 -(trifluoromethyl)phenol;
2-(4-{[(1R,2R)-2-hydroxycyclohexyl]amino}-5,6,8,9-tetrahydrooxepino[4,5-d]pyridazin-1-yl )-5-(trifluoromethyl)phenol;
5-chloro-2-(4-{[(1R,2R)-2-hydroxycyclohexyl]amino}-7,8-dihydro-5H-pyrano[3,4-d]pyridaz in-1-yl)phenol;
2-(4-{[(1R,2R)-2-hydroxycyclohexyl]amino}-6-methyl-5,6,7,8-tetrahydropyrido[3,4-d]pyrid azin-1-yl)-5-(trifluoromethyl)phenol;
5-(difluoromethoxy)-2-(4-{[(1R,2R)-2-hydroxycyclohexyl]amino}-7,8-dihydro-5H-pyrano[3 ,4-d]pyridazin-1-yl)phenol;
5-(difluoromethoxy)-2-(4-{ [(3 R)-1-methylpiperidin-3-yl] amino}-7, 8-dihydro-SH-pyrano[3,4 -d]pyridazin-1-yl)phenol;
2-(4-{[(1R,2R)-2-hydroxycyclohexyl]amino}-5,7-dihydrothieno[3,4-d]pyridazin-1-yl)-5-(trif luoromethyl)phenol; and
5-(difluoromethyl)-2-(4-{[(1R,3S)-3-hydroxycyclohexyl]amino}-5,7-dihydrofuro[3,4-d]pyrid azin-1-yl)phenol.

13. A pharmaceutical composition comprising the compound or a salt thereof according to claim 1 and one or more pharmaceutically acceptable excipients.

14. The pharmaceutical composition according to claim 13, which is an inhibitor of NLRP3 inflammasome activation.

15. The pharmaceutical composition according to claim 13, which is a pharmaceutical composition for preventing and/or treating inflammatory diseases and/or neurodegenerative diseases.

16. Use of the compound or a salt thereof according to claim 1 for production of a pharmaceutical composition for preventing and/or treating inflammatory diseases and/or neurodegenerative diseases.

17. Use of the compound or a salt thereof according to claim 1 for prevention and/or treatment of inflammatory diseases and/or neurodegenerative diseases.

18. The compound or a salt thereof according to claim 1 for use in prevention and/or treatment of inflammatory diseases and/or neurodegenerative diseases.

19. A method for preventing and/or treating inflammatory diseases and/or neurodegenerative diseases, comprising administering an effective amount of the compound or a salt thereof according to claim 1 to a subject.
